# EUROPEAN PATENT APPLICATION

(11) **EP 4 248 743 A1**
(43) Date of publication of application: **27.09.2023**
(21) Application number: 21878043.5
(22) Date of filing: 08.10.2021
(51) Int. Cl.: A01K 67/027, C07K 16/46

(54) **METHOD FOR PREPARING TRANSGENIC NON-HUMAN ANIMAL HAVING GENOME INCLUDING HUMANIZED IMMUNOGLOBULIN GENE LOCUS**

(30) Priority: 08.10.2020 KR 20200130328
(71) Applicant: HUMAB CO. LTD., Seoul 07793 (KR)
(72) Inventor: OH, Chang Kyu, Seoul 03012 (KR); PARK, Soon Ik, Seoul 04385 (KR); CHOI, Ae Jin, Namyangju-si, Gyeonggi-do 12181 (KR); BANG, Eui Sock, Incheon 21548 (KR)
(74) Representative: dompatent von Kreisler Selting Werner - Partnerschaft von Patent- und Rechtsanwälten mbB
(86) International application number: PCT/KR2021/013849
(87) International publication number: WO 2022/075793

(57) **Abstract**

The present invention relates to a method for producing a transgenic non-human animal having a genome including a humanized immunoglobulin locus. With the use of the technique disclosed in the present specification, it is possible to provide a method for producing a transgenic non-human animal cell or a transgenic non-human animal, each having a genome including a humanized immunoglobulin locus, using transgenesis that utilize recombination of chromosomes. In addition, the transgenic non-human animal prepared by the method can be provided for production of humanized or human antibodies.

## Description

### Technical Field

The present invention relates to a method for producing a transgenic non-human animal having a genome comprising humanized immunoglobulin gene locus (humanized immunoglobulin locus; or humanized IG locus). More specifically, the present invention relates to a method for producing a transgenic non-human animal cell or a transgenic non-human animal, each having a genome comprising humanized immunoglobulin gene locus, using transgenesis techniques that utilize recombination of chromosomes.

### Background Art

Transgenic animals are animals into which artificial traits are introduced. The transgenic animals are used for various disease studies, mechanism studies, and therapeutic drug development. For the production of transgenic animals, the method for producing transgenic animals involves the process of introducing the desired trait into an animal cell. For this purpose, methods using cloning vectors are currently used. A method using a cloning vector is a method of cloning a trait of interest, i.e., a gene of interest to be expressed in a transgenic animal, and transferring an artificial vector to an animal cell so that the artificial vector can be inserted into the genome. This method uses plasmids, bacterial artificial chromosomes (BACs), or yeast artificial chromosomes (YACs). BAC or YAC is a DNA structure capable of carrying larger fragments (150-350 kbp) than plasmids and is widely used for transgenesis. In particular, it has the advantage that BAC or YAC can carry relatively large fragments compared to plasmids and is used for transformation of large target genes.

However, for larger target genes, multiple BACs or YACs are required. In particular, when producing mice that produce human antibodies, in order to produce such mice, mouse cells in which a human immunoglobulin (Ig) gene is introduced must be produced. To do this, a transformation vector cloning a human immunoglobulin heavy (IGH) gene with a size of 1250 kilobases (kb) must be produced. When the transformation vectors are BACs, at least four to nine BACs are made, each with different DNA fragments. These BACs thus created are sequentially introduced into mouse cells and inserted into the genome. That is, the first BAC is introduced into the mouse cells and inserted into the genome, and the mouse cells into which the first BAC is inserted are selected. A second BAC is introduced into the selected mouse cells and inserted into the genome, and the mouse cells into which the second BAC is inserted are selected. Such a process must be repeated to select mouse cells with genome in which the gene of interest, i.e., the full length (total DNA) of the human IGH gene is inserted. This repetitive process becomes a factor that reduces the yield of mouse cells into which the full-length of the gene of interest is inserted. In addition, there are problems such as time consumption and cost consumption due to repetition of trait introduction and selection processes. In addition, the time and cost of the process of making multiple BACs is considerable.

To solve the problems of the existing systems using transgenic vectors such as BAC and YAC, the present invention provides a method for producing transgenic non-human animal cells or transgenic non-human animals having a genome including a humanized immunoglobulin locus, using the artificial interspecies chromosome segment exchange (AiCE) technology (KR10-2019-0042840), which is a transgenic technique that uses recombination between chromosomes.

### Disclosure

### Technical Problem

One objective of the present invention is to provide a method for producing a transgenic non-human animal cell having a genome comprising humanized immunoglobulin gene locus.

Another objective of the present invention is to provide a transgenic non-human animal cell having a genome comprising humanized immunoglobulin gene locus.

A further objective of the present invention is to provide a method for producing a transgenic non-human animal having a genome comprising humanized immunoglobulin locus.

A yet further objective of the present invention is to provide a transgenic non-human animal having a genome comprising humanized immunoglobulin locus.

### Technical Solution

The present invention provides method for producing transgenic non-human animal cell having genome comprising humanized immunoglobulin gene locus. In addition, the present invention provides a method for producing a transgenic non-human-animal cell produced through a method for producing a transgenic non-human-animal cell having a genome comprising humanized immunoglobulin gene locus. In this case, the humanized immunoglobulin locus may comprise variable region of a human immunoglobulin gene and constant region of an endogenous non-human animal immunoglobulin locus. Alternatively, the humanized immunoglobulin locus may comprise variable and constant regions of human immunoglobulin genes in an endogenous non-human-animal immunoglobulin locus.

As an embodiment, the method,
a) preparing a recipient cell having a recipient chromosome and a donor cell having a donor chromosome,
   wherein the recipient chromosome is an engineered non-human-animal chromosome having two or more RRS and non-human animal immunoglobulin locus; the two or more RRS are a first RRS and a second RRS; wherein the first RRS and the second RRS are not paired; the first RRS is located on the 5' end of the variable region of the non-human animal immunoglobulin locus, the second RRS is located on the 3' end of the variable region of the non-human animal immunoglobulin locus; the recipient cell is a non-human-animal cell;
   wherein the donor chromosome is an engineered human chromosome having two or more RRS and human immunoglobulin locus; the two or more RRS are a third RRS and a fourth RRS; wherein the third RRS and the fourth RRS are not paired; the third RRS is located on the 5' end of the variable region of the human immunoglobulin locus, the fourth RRS is located on the 3' end of the variable region of the human immunoglobulin locus; the donor cell is a human cell;
   wherein the first RRS and the third RRS are RRS for a first interchromosomal exchange, and the second RRS and fourth RRS are RRS for a second interchromosomal exchange;
b) producing a plurality of microcells using the donor cell,
   at least one microcell among the plurality of microcells includes the donor chromosome;
c) producing a fusion non-human animal cell by contacting at least one microcell with the recipient cell;
   the fusion non-human animal cell comprises the recipient chromosome and the donor chromosome; and
d) producing a recombinant non-human-animal cell having a recombinant chromosome by treating the fused non-human-animal cell with a recombinase,
   the recombinase recognizes the RRS for a first interchromosomal exchange and the RRS for a second interchromosomal exchange to induce recombination between the recipient chromosome and the donor chromosome; whereby variable region of the non-human animal immunoglobulin locus existing between the first and second RRS of the recipient chromosome is exchanged for the variable region of the human immunoglobulin locus between the third and fourth RRS of the donor chromosome;

As a result, a recombinant chromosome comprising variable region of a human immunoglobulin locus and constant region of an endogenous non-human animal immunoglobulin locus is generated.

The non-human animal immunoglobulin locus is non-human animal immunoglobulin heavy locus, the human immunoglobulin locus is human immunoglobulin heavy locus. Wherein the recombinant chromosome comprises a human immunoglobulin locus comprising variable region of a human immunoglobulin heavy locus and the constant region of an endogenous non-human animal immunoglobulin heavy locus.

The non-human animal immunoglobulin locus is an non-human-animal immunoglobulin kappa locus, the human immunoglobulin locus is a human immunoglobulin kappa locus. Wherein the recombinant chromosome comprises a human immunoglobulin locus comprising variable region of a human immunoglobulin kappa locus and the constant region of an endogenous non-human animal immunoglobulin kappa locus.

The non-human animal immunoglobulin locus is an non-human-animal immunoglobulin lambda locus, the human immunoglobulin locus is a human immunoglobulin lambda locus. Wherein the recombinant chromosome comprises a human immunoglobulin locus comprising variable region of a human immunoglobulin lambda locus and the constant region of an endogenous non-human animal immunoglobulin lambda locus.

The non-human animal immunoglobulin locus is an non-human-animal immunoglobulin kappa locus, the human immunoglobulin locus is a human immunoglobulin lambda locus. Wherein the recombinant chromosome comprises a human immunoglobulin locus comprising variable region of a human immunoglobulin lambda locus and the constant region of an endogenous non-human animal immunoglobulin kappa locus.

In the a), the recipient cell is prepared by providing a vector for a recipient chromosome to a non-human-animal cell, the donor cell is prepared by providing a vector for a donor chromosome to a human cell.

The vector for a recipient chromosome includes a first vector and a second vector, the first vector includes a first RRS, and the second vector includes a second RRS.

The vector for a donor chromosome includes a third vector and a fourth vector, the third vector includes a third RRS, and the fourth vector includes a fourth RRS.

The first RRS and the third RRS are each one selected from Loxp, FRT, attP, attB, ITR, and variants thereof, the first RRS and the third RRS are paired.

The second RRS and the fourth RRS are each one selected from Loxp, FRT, attP, attB, ITR, and variants thereof, the second RRS and the fourth RRS are paired.

The first vector, the second vector, the third vector and/or the fourth vector may further include one or more selection element. In this case, the selection element may be an antibiotic resistant gene or a fluorescent protein gene.

The recombinase includes a first recombinase capable of recognizing RRS for a first interchromosomal exchange and a second recombinase capable of recognizing RRS for a second interchromosomal exchange. Wherein the first recombinase is Cre recombinase, flippase (FLP), integrase or transposase. Wherein the second recombinase is Cre recombinase, flippase (FLP), integrase or transposase.

The first recombinase and the second recombinase are the same recombinase.

The non-human animal cell is mouse cell. Wherein the non-human animal immunoglobulin locus is mouse immunoglobulin locus.

In another embodiment, the method,
a) preparing a recipient cell having a recipient chromosome and a donor cell having a donor chromosome,
   wherein the recipient chromosome is an engineered non-human-animal chromosome having two or more RRS and non-human animal immunoglobulin locus; the two or more RRS are a first RRS and a second RRS; wherein the first RRS and the second RRS are not paired; the first RRS is located on the 5' end of the variable region of the non-human animal immunoglobulin locus, the second RRS is located on the 3' end of the variable region of the non-human animal immunoglobulin locus; the recipient cell is a non-human-animal cell;
   wherein the donor chromosome is an engineered human chromosome having two or more RRS and human immunoglobulin locus; the two or more RRS are a third RRS and a fourth RRS; wherein the third RRS and the fourth RRS are not paired; the third RRS is located on the 5' end of the variable region of the human immunoglobulin locus, the fourth RRS is located on the 3' end of the variable region of the human immunoglobulin locus; the donor cell is a human cell;
   wherein the first RRS and the third RRS are RRS for a first interchromosomal exchange, and the second RRS and fourth RRS are RRS for a second interchromosomal exchange;
b) producing a plurality of microcells using the donor cell,
   at least one microcell among the plurality of microcells include the donor chromosome;
c) producing a fusion non-human animal cell by contacting at least one microcell with the recipient cell;
   the fusion non-human animal cell comprises the recipient chromosome and the donor chromosome;
d) producing a recombinant non-human-animal cell having a recombinant non-human-animal chromosome by treating the fused non-human-animal cell with a recombinase,
   the recombinase recognizes the RRS for a first interchromosomal exchange and the RRS for a second interchromosomal exchange to induce recombination between the recipient chromosome and the donor chromosome; whereby variable region of the non-human animal immunoglobulin locus existing between the first and second RRS of the recipient chromosome is exchanged for the variable region of the human immunoglobulin locus between the third and fourth RRS of the donor chromosome,

As a result, a recombinant chromosome comprising variable region of a human immunoglobulin locus and constant region of an endogenous non-human animal immunoglobulin locus is generated.

The non-human animal immunoglobulin locus is an non-human-animal immunoglobulin kappa locus, the human immunoglobulin locus is a human immunoglobulin kappa locus. Wherein the recombinant non-human-animal chromosome may include a variable region and a constant region of the human immunoglobulin kappa locus.

The non-human animal immunoglobulin locus is an non-human-animal immunoglobulin lambda locus, the human immunoglobulin locus is a human immunoglobulin lambda locus. Wherein the recombinant non-human-animal chromosome may include a variable region and a constant region of the human immunoglobulin lambda locus.

The non-human animal immunoglobulin locus is an non-human-animal immunoglobulin kappa locus, the human immunoglobulin locus is a human immunoglobulin lambda locus. Wherein the recombinant non-human-animal chromosome may include a variable region and a constant region of the human immunoglobulin lambda locus.

In the a), the recipient cell is prepared by providing a vector for a recipient chromosome to a non-human-animal cell, the donor cell is prepared by providing a vector for a donor chromosome to a human cell.

The vector for a recipient chromosome includes a first vector and a second vector. The first vector includes a first RRS, and the second vector includes a second RRS.

The vector for a donor chromosome includes a third vector and a fourth vector. The third vector includes a third RRS, and the fourth vector includes a fourth RRS.

The first RRS and the third RRS are each one selected from Loxp, FRT, attP, attB, ITR, and variants thereof, the first RRS and the third RRS are paired.

The second RRS and the fourth RRS are each one selected from Loxp, FRT, attP, attB, ITR, and variants thereof, the second RRS and the fourth RRS are paired.

The first vector, the second vector, the third vector and/or the fourth vector may further include one or more selection element. In this case, the selection element may be an antibiotic resistant gene or a fluorescent protein gene.

The recombinase includes a first recombinase capable of recognizing RRS for a first interchromosomal exchange and a second recombinase capable of recognizing RRS for a second interchromosomal exchange. Wherein the first recombinase is Cre recombinase, flippase (FLP), integrase or transposase. Wherein the second recombinase is Cre recombinase, flippase (FLP), integrase or transposase.

The first recombinase and the second recombinase are the same recombinase.

The non-human animal cell is mouse cell. Wherein the non-human animal immunoglobulin locus is mouse immunoglobulin locus.

As another embodiment, the method,
a) preparing a recipient cell having a recipient chromosome and a donor cell having a donor chromosome,
   wherein the recipient chromosome is an engineered non-human-animal chromosome having two or more RRS and non-human animal immunoglobulin locus; the two or more RRS are a first RRS and a second RRS; wherein the first RRS and the second RRS are not paired; the first RRS is located on the 5' end of the variable region of the non-human animal immunoglobulin locus, the second RRS is located on the 3' end of the variable region of the non-human animal immunoglobulin locus; the recipient cell is a non-human-animal cell;
   wherein the donor chromosome is an engineered human chromosome having two or more RRS and human immunoglobulin locus; the two or more RRS are a third RRS and a fourth RRS; wherein the third RRS and the fourth RRS are not paired; the third RRS is located on the 5' end of the variable region of the human immunoglobulin locus, the fourth RRS is located on the 3' end of the variable region of the human immunoglobulin locus; the donor cell is a human cell;
   wherein the first RRS and the third RRS are RRS for a first interchromosomal exchange, and the second RRS and fourth RRS are RRS for a second interchromosomal exchange;
b) producing a plurality of microcells using the donor cell,
   at least one microcell among the plurality of microcells includes the donor chromosome;
c) producing a fusion non-human animal cell by contacting at least one microcell with the recipient cell;
   the fusion non-human animal cell comprises the recipient chromosome and the donor chromosome; and
d) producing a recombinant non-human-animal cell having a recombinant chromosome by treating the fused non-human-animal cell with a recombinase,
   the recombinase recognizes the RRS for a first interchromosomal exchange and the RRS for a second interchromosomal exchange to induce recombination between the recipient chromosome and the donor chromosome; whereby variable region of the non-human animal immunoglobulin locus existing between the first and second RRS of the recipient chromosome is exchanged for the variable region of the human immunoglobulin locus between the third and fourth RRS of the donor chromosome;

As a result, a recombinant chromosome comprising variable region of a human immunoglobulin locus and constant region of an endogenous non-human animal immunoglobulin locus is generated.

The non-human animal immunoglobulin locus is non-human animal immunoglobulin heavy locus, the human immunoglobulin locus is human immunoglobulin heavy locus. Wherein the recombinant chromosome comprises a human immunoglobulin locus comprising variable region of a human immunoglobulin heavy locus and the constant region of an endogenous non-human animal immunoglobulin heavy locus.

The non-human animal immunoglobulin locus is an non-human-animal immunoglobulin kappa locus, the human immunoglobulin locus is a human immunoglobulin kappa locus. Wherein the recombinant chromosome comprises variable region of a human immunoglobulin kappa locus and the constant region of an endogenous non-human animal immunoglobulin kappa locus.

The non-human animal immunoglobulin locus is an non-human-animal immunoglobulin lambda locus, the human immunoglobulin locus is a human immunoglobulin lambda locus. Wherein the recombinant chromosome comprises variable region of a human immunoglobulin lambda locus and the constant region of an endogenous non-human animal immunoglobulin lambda locus.

The non-human animal immunoglobulin locus is an non-human-animal immunoglobulin lambda locus, the human immunoglobulin locus is a human immunoglobulin lambda locus. Wherein the recombinant chromosome comprises variable region of a human immunoglobulin lambda locus and the constant region of an endogenous non-human animal immunoglobulin lambda locus.

In the a), the recipient cell is prepared by providing a vector for a recipient chromosome to a non-human-animal cell, the donor cell is prepared by providing a vector for a donor chromosome to a human cell.

The vector for a recipient chromosome includes a first vector and a second vector, the first vector includes a first RRS, and the second vector includes a second RRS. Wherein the first vector may additionally include one more RRS, and the added RRS may be the fifth RRS. Wherein the second vector may additionally include one more RRS, and the added RRS may be the sixth RRS. The fifth RRS and the sixth RRS may be first RRS for inversion. Wherein the fifth RRS and sixth RRS are each selected from among Loxp, FRT, attP, attB, ITR, and variants thereof, and the fifth RRS and sixth RRS may be paired with each other.

When the vector for the recipient chromosome includes the fifth RRS and the sixth RRS, respectively, in a), the recipient cell further comprises providing the vector for the recipient chromosome to the non-human-animal cell, and then treating the recipient cell with a recombinase. The recombinase may be a recombinase that recognizes the RRS for the first inversion, the recombinase may be Cre recombinase, flippase (FLP), integrase or transposase.

The vector for a donor chromosome includes a third vector and a fourth vector, the third vector includes a third RRS, and the fourth vector includes a fourth RRS. Wherein the third vector may additionally include one more RRS, and the added RRS may be the seventh RRS. Wherein the fourth vector may additionally include one more RRS, and the added RRS may be the eighth RRS. The seventh RRS and the eighth RRS may be second RRS for inversion. Wherein the seventh RRS and eighth RRS are each selected from among Loxp, FRT, attP, attB, ITR, and variants thereof, and the seventh RRS and eighth RRS may be paired with each other.

When the vector for the donor chromosome includes the seventh RRS and the eighth RRS, respectively, in a), the donor cell may further include a step of providing the donor chromosome vector to the human cell and then treating the donor cell with a recombinase. The recombinase may be a recombinase that recognizes the RRS for the second inversion, the recombinase may be Cre recombinase, flippase (FLP), integrase or transposase.

The first RRS and the fourth RRS are each one selected from Loxp, FRT, attP, attB, ITR, and variants thereof, the first RRS and the fourth RRS are paired,

The second RRS and the third RRS are each one selected from Loxp, FRT, attP, attB, ITR, and variants thereof, the second RRS and the third RRS are paired.

The first vector, the second vector, the third vector and/or the fourth vector may further include one or more selection element. In this case, the selection element may be an antibiotic resistant gene or a fluorescent protein gene.

The recombinase includes a first recombinase capable of recognizing RRS for a first interchromosomal exchange and a second recombinase capable of recognizing RRS for a second interchromosomal exchange. Wherein the first recombinase is Cre recombinase, flippase (FLP), integrase or transposase. Wherein the second recombinase is Cre recombinase, flippase (FLP), integrase or transposase.

The first recombinase and the second recombinase are the same recombinase.

The non-human animal cell is mouse cell. Wherein the non-human animal immunoglobulin locus is mouse immunoglobulin locus.

As another embodiment, the method,
a) preparing a recipient cell having a recipient chromosome and a donor cell having a donor chromosome,
   wherein the recipient chromosome is an engineered non-human-animal chromosome having two or more RRS and non-human animal immunoglobulin locus; the two or more RRS are a first RRS and a second RRS; wherein the first RRS and the second RRS are not paired; the first RRS is located on the 5' end of the variable region of the non-human animal immunoglobulin locus, the second RRS is located on the 3' end of the variable region of the non-human animal immunoglobulin locus; the recipient cell is a non-human-animal cell;
   wherein the donor chromosome is an engineered human chromosome having two or more RRS and human immunoglobulin locus; the two or more RRS are a third RRS and a fourth RRS; wherein the third RRS and the fourth RRS are not paired; the third RRS is located on the 5' end of the variable region of the human immunoglobulin locus, the fourth RRS is located on the 3' end of the variable region of the human immunoglobulin locus; the donor cell is a human cell;
   wherein the first RRS and the third RRS are RRS for a first interchromosomal exchange, and the second RRS and fourth RRS are RRS for a second interchromosomal exchange;
b) producing a plurality of microcells using the donor cell,
   at least one microcell among the plurality of microcells includes the donor chromosome;
c) producing a fusion non-human animal cell by contacting at least one microcell with the recipient cell;
   the fusion non-human animal cell comprises the recipient chromosome and the donor chromosome; and
d) producing a recombinant non-human-animal cell having a recombinant chromosome by treating the fused non-human-animal cell with a recombinase,
   the recombinase recognizes the RRS for a first interchromosomal exchange and the RRS for a second interchromosomal exchange to induce recombination between the recipient chromosome and the donor chromosome; whereby variable region of the non-human animal immunoglobulin locus existing between the first and second RRS of the recipient chromosome is exchanged for the variable region of the human immunoglobulin locus between the third and fourth RRS of the donor chromosome;

As a result, a recombinant chromosome comprising variable region of a human immunoglobulin locus and constant region of an endogenous non-human animal immunoglobulin locus is generated.

The non-human animal immunoglobulin locus is non-human animal immunoglobulin heavy locus, the human immunoglobulin locus is human immunoglobulin heavy locus. Wherein the recombinant chromosome comprises a human immunoglobulin locus comprising variable region of a human immunoglobulin heavy locus and the constant region of an endogenous non-human animal immunoglobulin heavy locus.

The non-human animal immunoglobulin locus is an non-human-animal immunoglobulin kappa locus, the human immunoglobulin locus is a human immunoglobulin kappa locus. Wherein the recombinant chromosome comprises a human immunoglobulin locus comprising variable region of a human immunoglobulin kappa locus and the constant region of an endogenous non-human animal immunoglobulin kappa locus.

The non-human animal immunoglobulin locus is an non-human-animal immunoglobulin lambda locus, the human immunoglobulin locus is a human immunoglobulin lambda locus. Wherein the recombinant chromosome comprises variable region of a human immunoglobulin lambda locus and the constant region of an endogenous non-human animal immunoglobulin lambda locus.

The non-human animal immunoglobulin locus is an non-human-animal immunoglobulin kappa locus, the human immunoglobulin locus is a human immunoglobulin lambda locus. Wherein the recombinant chromosome comprises variable region of a human immunoglobulin lambda locus and the constant region of an endogenous non-human animal immunoglobulin kappa locus.

In the a), the recipient cell is prepared by providing a vector for a recipient chromosome to a non-human-animal cell, the donor cell is prepared by providing a vector for a donor chromosome to a human cell.

The vector for a recipient chromosome includes a first RRS.

The vector for a donor chromosome includes a second RRS.

The first RRS and the second RRS are each one selected from Loxp, FRT, attP, attB, ITR, and variants thereof, the first RRS and the second RRS are paired,

The vector for recipient chromosome and the vector for a donor chromosome may further include one or more selection element. In this case, the selection element may be an antibiotic resistant gene or a fluorescent protein gene.

The recombinase is Cre recombinase, flippase (FLP), integrase or transposase.

The non-human animal cell is mouse cell, the non-human animal immunoglobulin locus is mouse immunoglobulin locus.

The present invention provides method for producing transgenic non-human animal cell having genome comprising humanized immunoglobulin gene locus. In addition, the present invention provides a method for producing a transgenic non-human-animal produced through a method for producing a transgenic non-human-animal having a genome comprising humanized immunoglobulin gene locus. In this case, the humanized immunoglobulin locus may comprise variable region of a human immunoglobulin gene and constant region of an endogenous non-human animal immunoglobulin locus. Alternatively, the humanized immunoglobulin locus may comprise variable and constant regions of human immunoglobulin genes in an endogenous non-human-animal immunoglobulin locus.

As an embodiment, the method,
a) preparing a recipient cell having a recipient chromosome and a donor cell having a donor chromosome,
   wherein the recipient chromosome is an engineered non-human-animal chromosome having two or more RRS and non-human animal immunoglobulin locus; the two or more RRS are a first RRS and a second RRS; wherein the first RRS and the second RRS are not paired; the first RRS is located on the 5' end of the variable region of the non-human animal immunoglobulin locus, the second RRS is located on the 3' end of the variable region of the non-human animal immunoglobulin locus; the recipient cell is a non-human-animal cell;
   wherein the donor chromosome is an engineered human chromosome having two or more RRS and human immunoglobulin locus; the two or more RRS are a third RRS and a fourth RRS; wherein the third RRS and the fourth RRS are not paired; the third RRS is located on the 5' end of the variable region of the human immunoglobulin locus, the fourth RRS is located on the 3' end of the variable region of the human immunoglobulin locus; the donor cell is a human cell;
   wherein the first RRS and the third RRS are RRS for a first interchromosomal exchange, and the second RRS and fourth RRS are RRS for a second interchromosomal exchange;
b) producing a plurality of microcells using the donor cell,
   at least one microcell among the plurality of microcells includes the donor chromosome;
c) producing a fusion non-human animal cell by contacting at least one microcell with the recipient cell;
   the fusion non-human animal cell comprises the recipient chromosome and the donor chromosome; and
d) producing a recombinant non-human-animal cell having a recombinant chromosome by treating the fused non-human-animal cell with a recombinase,
   the recombinase recognizes the RRS for a first interchromosomal exchange and the RRS for a second interchromosomal exchange to induce recombination between the recipient chromosome and the donor chromosome; whereby variable region of the non-human animal immunoglobulin locus existing between the first and second RRS of the recipient chromosome is exchanged for the variable region of the human immunoglobulin locus between the third and fourth RRS of the donor chromosome; As a result, a recombinant chromosome comprising variable region of a human immunoglobulin locus and constant region of an endogenous non-human animal immunoglobulin locus is generated.
e) producing offspring using the recombinant non-human-animal cell.

The non-human animal cell is a somatic cell. Wherein the recombinant non-human animal cell is a somatic cell. Wherein in the e), offspring is produced through somatic cell nuclear transfer (SCNT) using recombinant somatic cells.

The non-human animal cell is a non-human animal embryo. Wherein the recombinant non-human animal cell is a non-human animal embryo. Wherein in the e), offspring is produced by implantation of a recombinant non-human animal embryo into the uterus of a surrogate mother.

The non-human animal cell is a non-human animal embryonic stem cell(ES cell). Wherein the recombinant non-human animal cell is a recombinant non-human animal embryonic stem cell. Wherein in the e), recombinant non-human animal embryonic stem cell is transplanted into blastocysts to produce a chimeric blastocyst, offspring is produced by implantation of a chimeric blastocyst into the uterus of a surrogate mother.

The non-human animal cell is a mouse cell. Wherein the transgenic non-human animal is a mouse.

As another embodiment, the method,
a) preparing a recipient cell having a recipient chromosome and a donor cell having a donor chromosome,
   wherein the recipient chromosome is an engineered non-human-animal chromosome having two or more RRS and non-human animal immunoglobulin locus; the two or more RRS are a first RRS and a second RRS; wherein the first RRS and the second RRS are not paired; the first RRS is located on the 5' end of the variable region of the non-human animal immunoglobulin locus, the second RRS is located on the 3' end of the variable region of the non-human animal immunoglobulin locus; the recipient cell is a non-human-animal cell;
   wherein the donor chromosome is an engineered human chromosome having two or more RRS and human immunoglobulin locus; the two or more RRS are a third RRS and a fourth RRS; wherein the third RRS and the fourth RRS are not paired; the third RRS is located on the 5' end of the variable region of the human immunoglobulin locus, the fourth RRS is located on the 3' end of the variable region of the human immunoglobulin locus; the donor cell is a human cell;
   wherein the first RRS and the third RRS are RRS for a first interchromosomal exchange, and the second RRS and fourth RRS are RRS for a second interchromosomal exchange;
b) producing a plurality of microcells using the donor cell,
   at least one microcell among the plurality of microcells includes the donor chromosome;
c) producing a fusion non-human animal cell by contacting at least one microcell with the recipient cell;
   the fusion non-human animal cell comprises the recipient chromosome and the donor chromosome; and
d) producing a recombinant non-human-animal cell having a recombinant chromosome by treating the fused non-human-animal cell with a recombinase,
   the recombinase recognizes the RRS for a first interchromosomal exchange and the RRS for a second interchromosomal exchange to induce recombination between the recipient chromosome and the donor chromosome; whereby variable region of the non-human animal immunoglobulin locus existing between the first and second RRS of the recipient chromosome is exchanged for the variable region of the human immunoglobulin locus between the third and fourth RRS of the donor chromosome; As a result, a recombinant chromosome comprising variable region of a human immunoglobulin locus and constant region of an endogenous non-human animal immunoglobulin locus is generated.
e) producing offspring using the recombinant non-human-animal cell.

The non-human animal cell is a somatic cell. Wherein the recombinant non-human animal cell is a somatic cell. Wherein in the e), offspring is produced through somatic cell nuclear transfer (SCNT) using recombinant somatic cells.

The non-human animal cell is a non-human animal embryo. Wherein the recombinant non-human animal cell is a non-human animal embryo. Wherein in the e), offspring is produced by implantation of a recombinant non-human animal embryo into the uterus of a surrogate mother.

The non-human animal cell is a non-human animal embryonic stem cell(ES cell). Wherein the recombinant non-human animal cell is a recombinant non-human animal embryonic stem cell. Wherein in the e), recombinant non-human animal embryonic stem cell is transplanted into blastocysts to produce a chimeric blastocyst, offspring is produced by implantation of a chimeric blastocyst into the uterus of a surrogate mother.

The non-human animal cell is a mouse cell. Wherein the transgenic non-human animal is a mouse.

As another embodiment, the method,
a) preparing a recipient cell having a recipient chromosome and a donor cell having a donor chromosome,
   wherein the recipient chromosome is an engineered non-human-animal chromosome having two or more RRS and non-human animal immunoglobulin locus; the two or more RRS are a first RRS and a second RRS; wherein the first RRS and the second RRS are not paired; the first RRS is located on the 5' end of the variable region of the non-human animal immunoglobulin locus, the second RRS is located on the 3' end of the variable region of the non-human animal immunoglobulin locus; the recipient cell is a non-human-animal cell;
   wherein the donor chromosome is an engineered human chromosome having two or more RRS and human immunoglobulin locus; the two or more RRS are a third RRS and a fourth RRS; wherein the third RRS and the fourth RRS are not paired; the third RRS is located on the 5' end of the variable region of the human immunoglobulin locus, the fourth RRS is located on the 3' end of the variable region of the human immunoglobulin locus; the donor cell is a human cell;
   wherein the first RRS and the third RRS are RRS for a first interchromosomal exchange, and the second RRS and fourth RRS are RRS for a second interchromosomal exchange;
b) producing a plurality of microcells using the donor cell,
   at least one microcell among the plurality of microcells includes the donor chromosome;
c) producing a fusion non-human animal cell by contacting at least one microcell with the recipient cell;
   the fusion non-human animal cell comprises the recipient chromosome and the donor chromosome; and
d) producing a recombinant non-human-animal cell having a recombinant chromosome by treating the fused non-human-animal cell with a recombinase,
   the recombinase recognizes the RRS for a first interchromosomal exchange and the RRS for a second interchromosomal exchange to induce recombination between the recipient chromosome and the donor chromosome; whereby variable region of the non-human animal immunoglobulin locus existing between the first and second RRS of the recipient chromosome is exchanged for the variable region of the human immunoglobulin locus between the third and fourth RRS of the donor chromosome; As a result, a recombinant chromosome comprising variable region of a human immunoglobulin locus and constant region of an endogenous non-human animal immunoglobulin locus is generated.
e) producing offspring using the recombinant non-human-animal cell.

The non-human animal cell is a somatic cell. Wherein the recombinant non-human animal cell is a somatic cell. Wherein in the e), offspring is produced through somatic cell nuclear transfer (SCNT) using recombinant somatic cells.

The non-human animal cell is a non-human animal embryo. Wherein the recombinant non-human animal cell is a non-human animal embryo. Wherein in the e), offspring is produced by implantation of a recombinant non-human animal embryo into the uterus of a surrogate mother.

The non-human animal cell is a non-human animal embryonic stem cell(ES cell). Wherein the recombinant non-human animal cell is a recombinant non-human animal embryonic stem cell. Wherein in the e), recombinant non-human animal embryonic stem cell is transplanted into blastocysts to produce a chimeric blastocyst, offspring is produced by implantation of a chimeric blastocyst into the uterus of a surrogate mother.

The non-human animal cell is a mouse cell. Wherein the transgenic non-human animal is a mouse.

As another embodiment, the method,
a) preparing a recipient cell having a recipient chromosome and a donor cell having a donor chromosome,
   wherein the recipient chromosome is an engineered non-human-animal chromosome having two or more RRS and non-human animal immunoglobulin locus; the two or more RRS are a first RRS and a second RRS; wherein the first RRS and the second RRS are not paired; the first RRS is located on the 5' end of the variable region of the non-human animal immunoglobulin locus, the second RRS is located on the 3' end of the variable region of the non-human animal immunoglobulin locus; the recipient cell is a non-human-animal cell;
   wherein the donor chromosome is an engineered human chromosome having two or more RRS and human immunoglobulin locus; the two or more RRS are a third RRS and a fourth RRS; wherein the third RRS and the fourth RRS are not paired; the third RRS is located on the 5' end of the variable region of the human immunoglobulin locus, the fourth RRS is located on the 3' end of the variable region of the human immunoglobulin locus; the donor cell is a human cell;
   wherein the first RRS and the third RRS are RRS for a first interchromosomal exchange, and the second RRS and fourth RRS are RRS for a second interchromosomal exchange;
b) producing a plurality of microcells using the donor cell,
   at least one microcell among the plurality of microcells includes the donor chromosome;
c) producing a fusion non-human animal cell by contacting at least one microcell with the recipient cell;
   the fusion non-human animal cell comprises the recipient chromosome and the donor chromosome; and
d) producing a recombinant non-human-animal cell having a recombinant chromosome by treating the fused non-human-animal cell with a recombinase,
   the recombinase recognizes the RRS for a first interchromosomal exchange and the RRS for a second interchromosomal exchange to induce recombination between the recipient chromosome and the donor chromosome; whereby variable region of the non-human animal immunoglobulin locus existing between the first and second RRS of the recipient chromosome is exchanged for the variable region of the human immunoglobulin locus between the third and fourth RRS of the donor chromosome; As a result, a recombinant chromosome comprising variable region of a human immunoglobulin locus and constant region of an endogenous non-human animal immunoglobulin locus is generated.
e) producing offspring using the recombinant non-human-animal cell.

The non-human animal cell is a somatic cell. Wherein the recombinant non-human animal cell is a somatic cell. Wherein in the e), offspring is produced through somatic cell nuclear transfer (SCNT) using recombinant somatic cells.

The non-human animal cell is a non-human animal embryo. Wherein the recombinant non-human animal cell is a non-human animal embryo. Wherein in the e), offspring is produced by implantation of a recombinant non-human animal embryo into the uterus of a surrogate mother.

The non-human animal cell is a non-human animal embryonic stem cell(ES cell). Wherein the recombinant non-human animal cell is a recombinant non-human animal embryonic stem cell. Wherein in the e), recombinant non-human animal embryonic stem cell is transplanted into blastocysts to produce a chimeric blastocyst, offspring is produced by implantation of a chimeric blastocyst into the uterus of a surrogate mother.

The non-human animal cell is a mouse cell. Wherein the transgenic non-human animal is a mouse.

The present invention provides vector set for producing transgenic non-human animal cell having genome comprising humanized immunoglobulin gene locus.

As an embodiment, the vector set may comprise:
a first vector comprising a first RRS, a first promoter and a first selection element;
a second vector comprising a second promoter, a second selection element, and a second RRS;
a third vector comprising a third promoter, a third selection element, and a third RRS; and

A fourth vector comprising a fourth RRS, a fourth promoter and a fourth selection element.

As another embodiment, the vector set may comprise:
a first vector comprising a first promoter, a first RRS, a second promoter and a second RRS;
a second vector comprising the first selection element, the third RRS and the fourth RRS;
a third vector comprising the fifth RRS, the third promoter and the sixth RRS; and

A fourth vector comprising a second selection element, a seventh RRS, a third selection element, and an eighth RRS.

As another embodiment, the vector set may comprise:
a vector for a recipient chromosome comprising a first promoter, a first selection element, and a first RRS; and
a vector for a donor chromosome comprising a second RRS, a second promoter and a second selection element.

The present invention provides an antibody production method using a transgenic non-human-animal having a genome comprising a humanized immunoglobulin locus.

The method may comprise injecting an antigen into a transgenic non-human-animal having a genome comprising a humanized immunoglobulin locus.

### Advantageous Effects

The present invention relates to a method for producing a transgenic non-human animal having a genome comprising humanized immunoglobulin locus. With the use of the technique disclosed in the present specification, it is possible to provide a method for producing a transgenic non-human animal cell or a transgenic non-human animal, each having a genome including a humanized immunoglobulin locus, using transgenesis that utilize recombination of chromosomes. In addition, the transgenic non-human animal prepared by the method can be provided for production of humanized or human antibodies.

### Description of Drawings

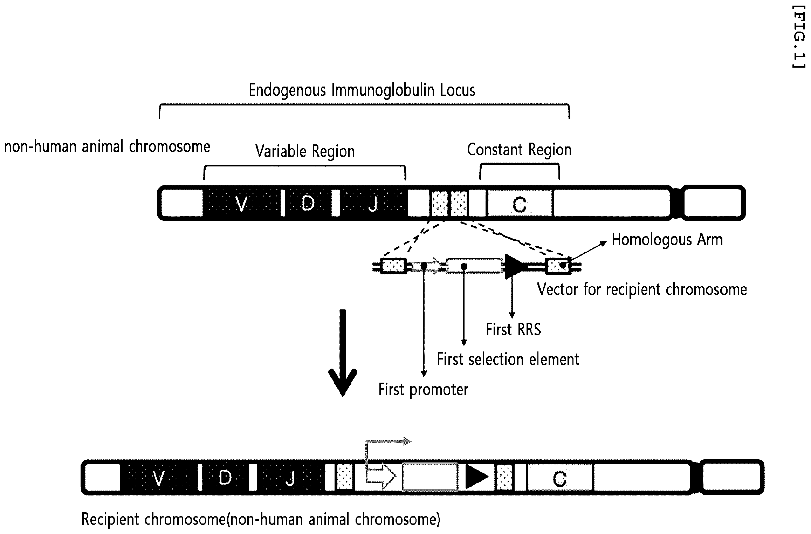

FIGS. 1 to 3 are diagrams related to one embodiment of a method for producing a recombinant cell having a recombinant chromosome using cell fusion and interchromosomal exchange and for producing a transgenic animal using the same recombinant cell. Specifically, these are diagrams related to one embodiment using a set of vectors (first set of vectors) for inserting one RRS.

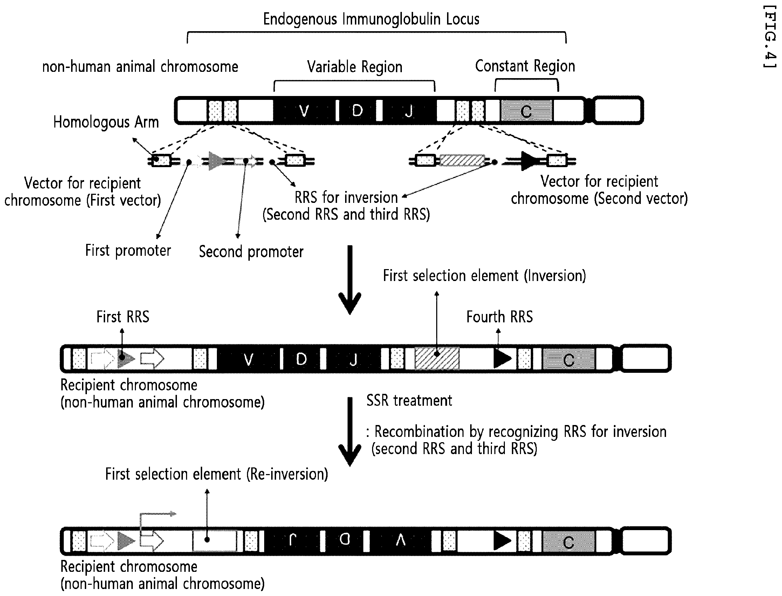

FIGS. 4 to 6 are diagrams related to one embodiment of a method for producing a recombinant cell having a recombinant chromosome using cell fusion and interchromosomal exchange and for producing a transgenic animal using the same recombinant cell. Specifically, these are diagrams related to one embodiment using a set of vectors (second set of vectors) for inserting two or more RRSs.

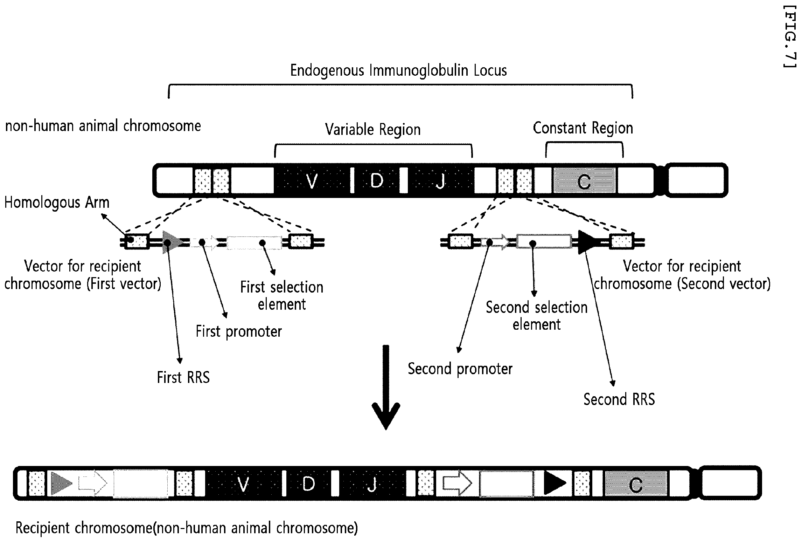

FIGS. 7 to 9 are diagrams related to one embodiment of a method for producing a recombinant cell having a recombinant chromosome using cell fusion and interchromosomal exchange and for producing a transgenic animal using the same recombinant cell. Specifically, these are diagrams related to one embodiment using a set of vectors (third set of vectors) for inserting two or more RRSs.

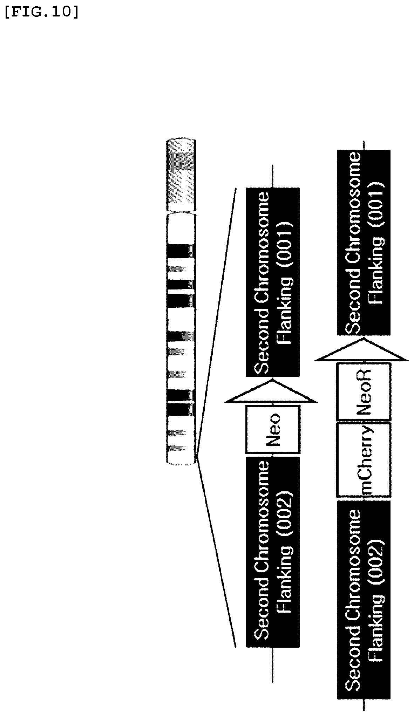

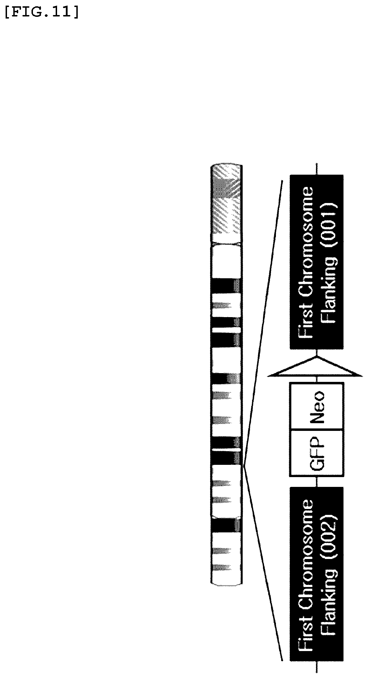

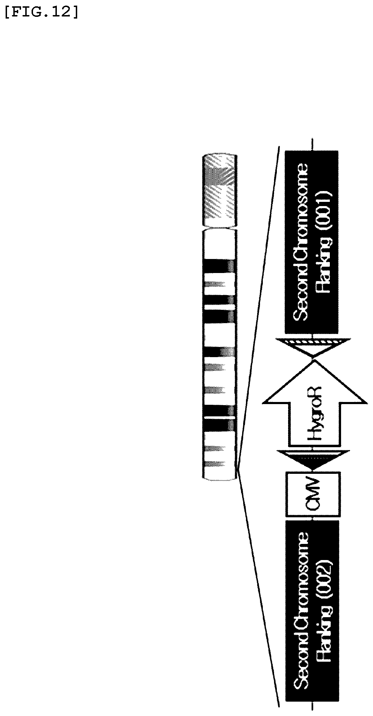

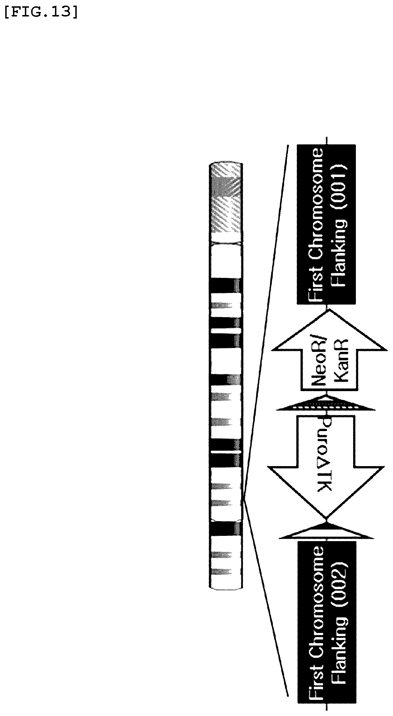

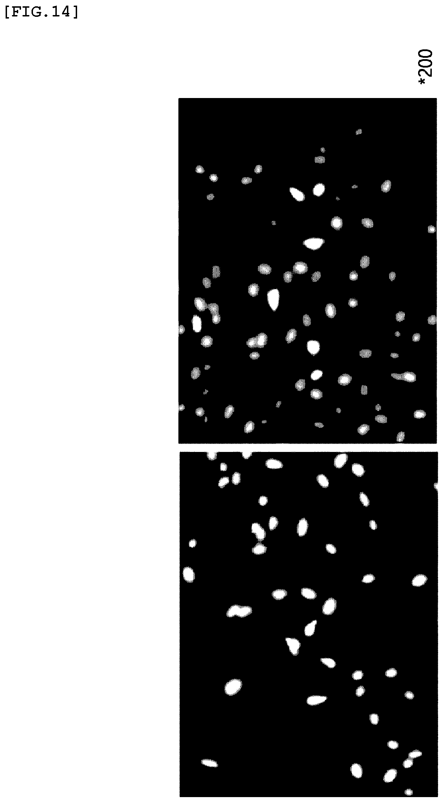

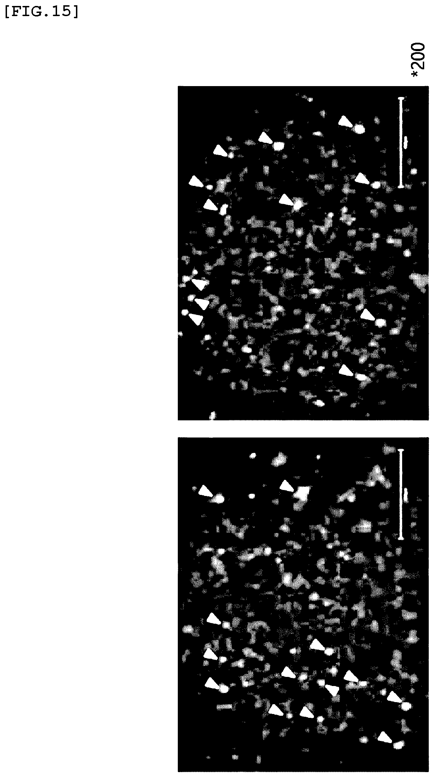

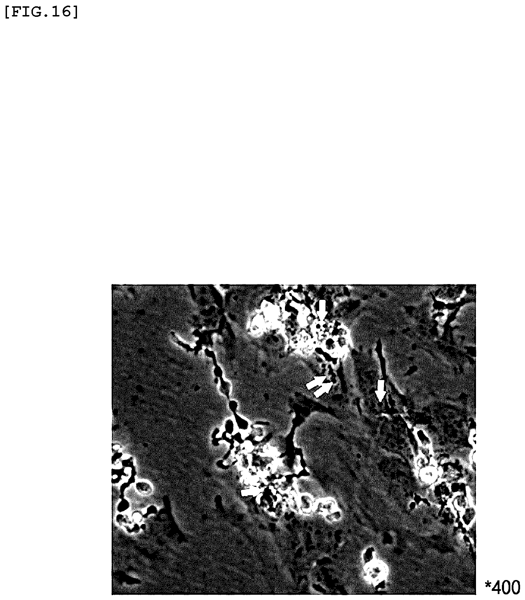

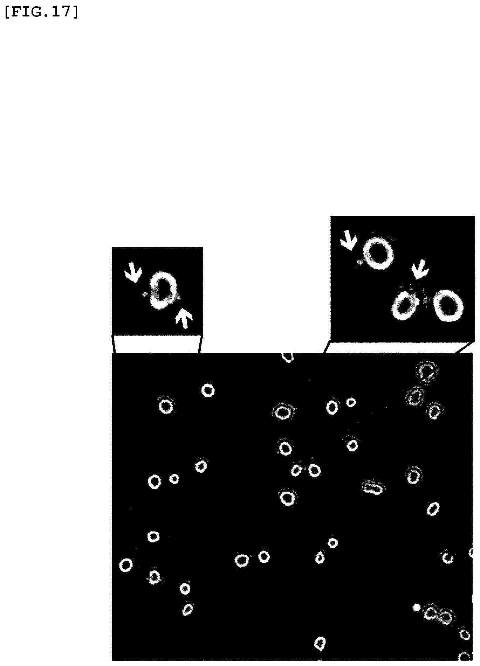

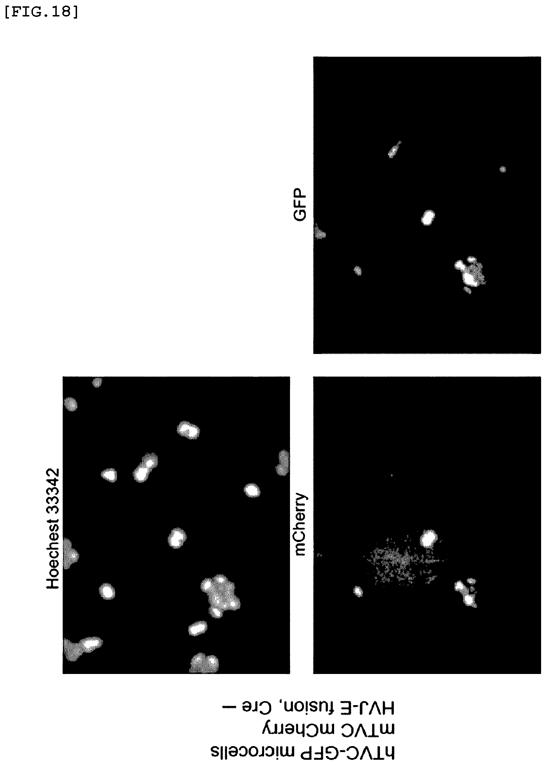

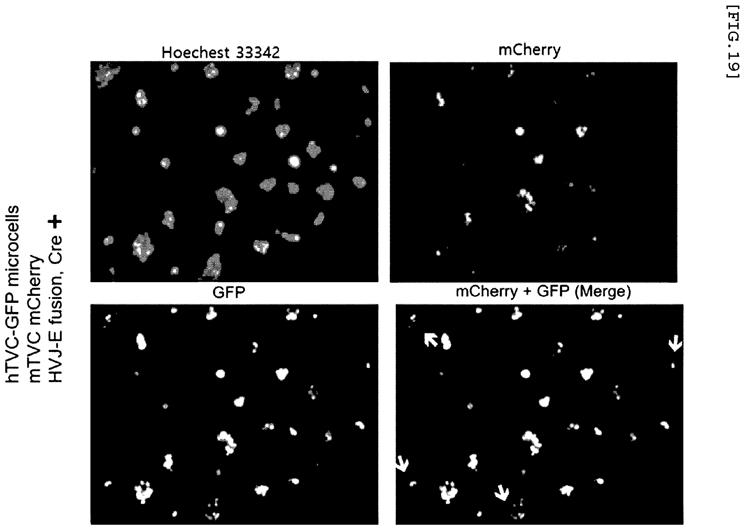

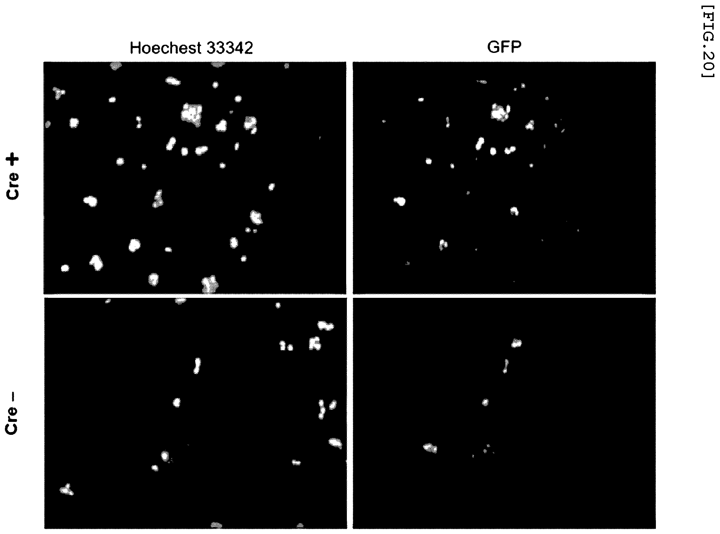

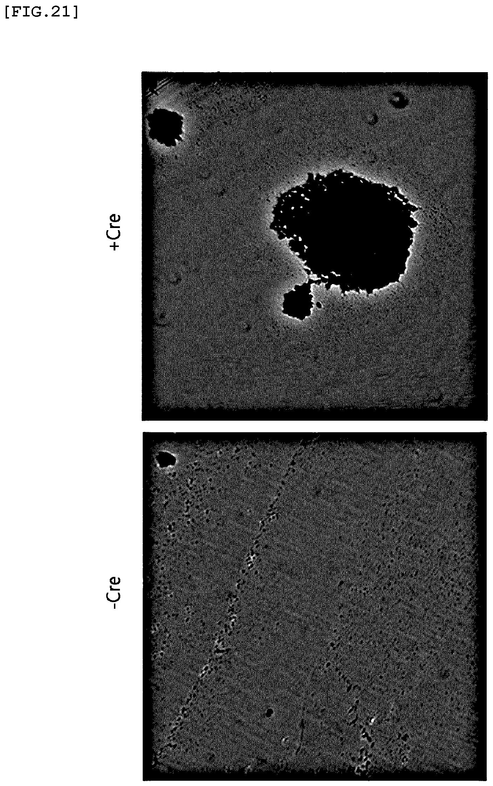

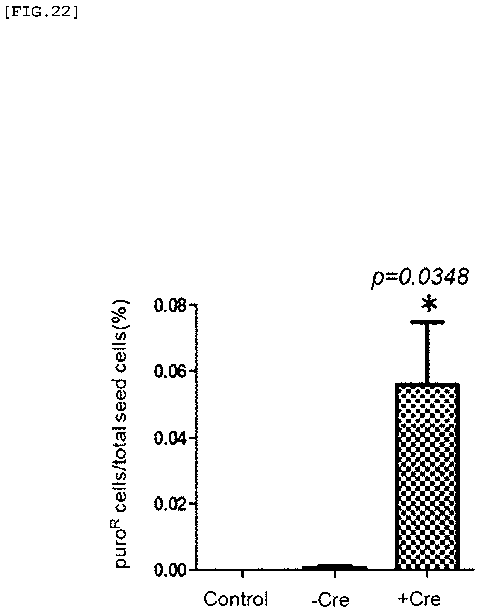

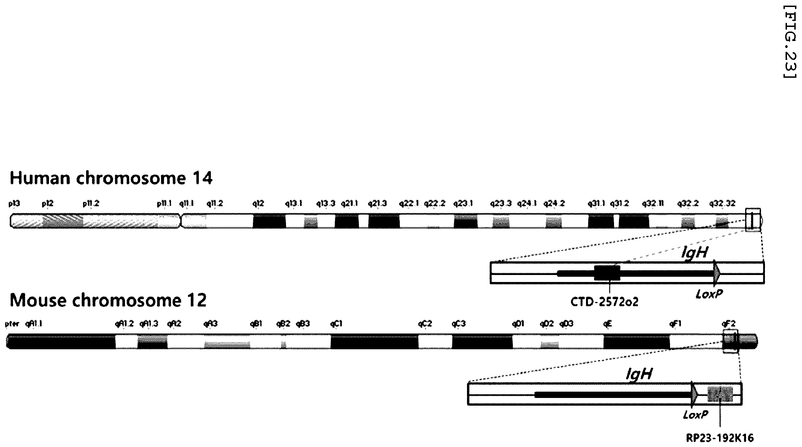

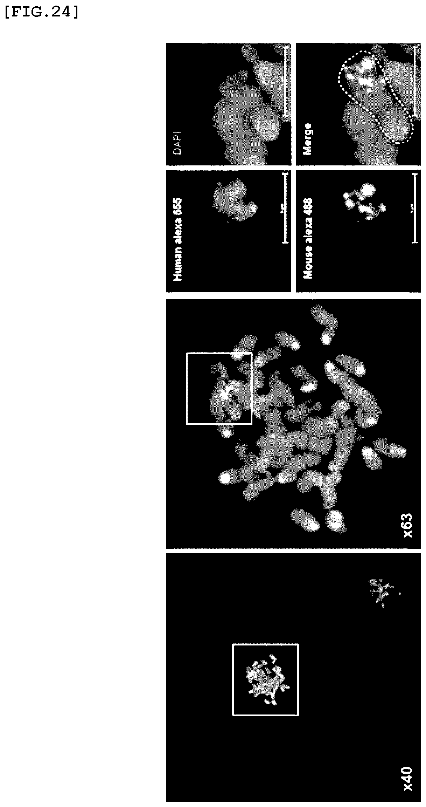

FIGS. 10 to 24 are diagrams related to one embodiment of a method for producing a recombinant cell having a recombinant chromosome using cell fusion and interchromosomal exchange and for producing a transgenic animal using the same recombinant cell. Each of the drawings will be described in detail below.
FIGS. 10 to 13 are schematic diagrams of a DNA structure of a chromosome having one or more RRSs according to one embodiment.
FIGS. 14 to 15 shows results of selection of chromosomes having one or more RRSs according to one embodiment.
FIG. 16 shows a microcell according to one embodiment.
FIG. 17 shows a fusion cell production process according to one embodiment.
FIG. 18 shows a fusion cell according to one embodiment.
FIG. 19 shows a recombinant cell comprising a recombinant chromosome according to one embodiment.
FIGS. 20 to 22 show recombinant cells according to one example and specifically show comparison between a recombinant cell comprising a recombinant chromosome and a recombinant cell not comprising a recombinant chromosome.
FIGS. 23 to 24 show the results of selection of recombinant cells comprising recombinant chromosomes and results of identification of recombinant chromosomes, according to one embodiment.
FIGS. 25 and 26 show the appearance of a chimeric mouse produced using recombinant cells including recombinant chromosomes according to one embodiment.
FIGS. 27 to 34 show the production of recipient cells having recipient chromosomes comprising two or more RRSs and mouse immunoglobulin loci according to one embodiment and show the results of identifying the recipient cells.

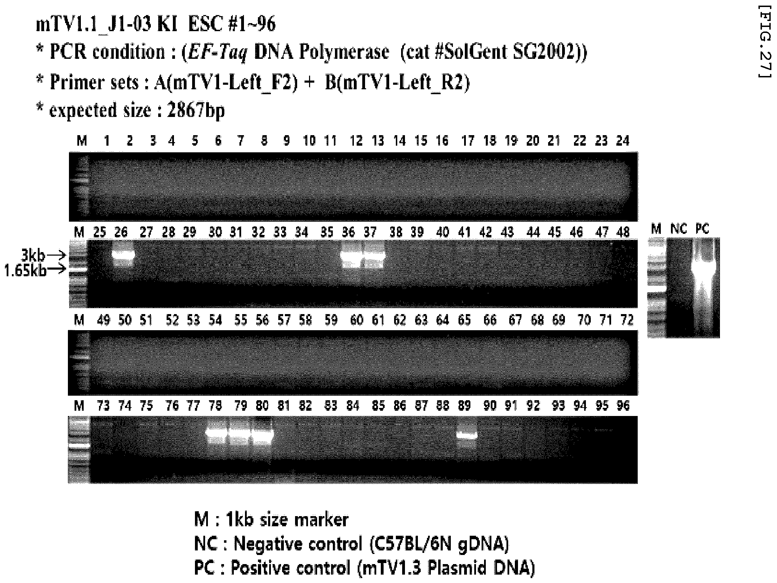

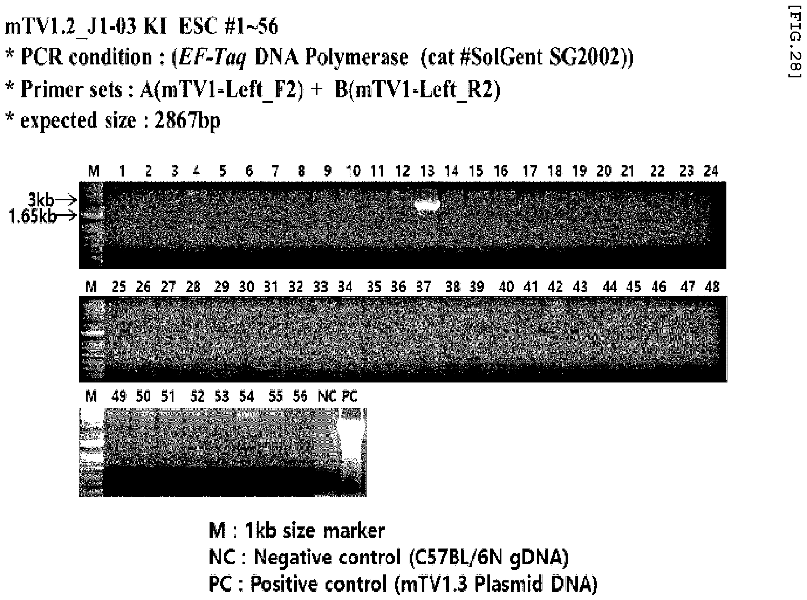

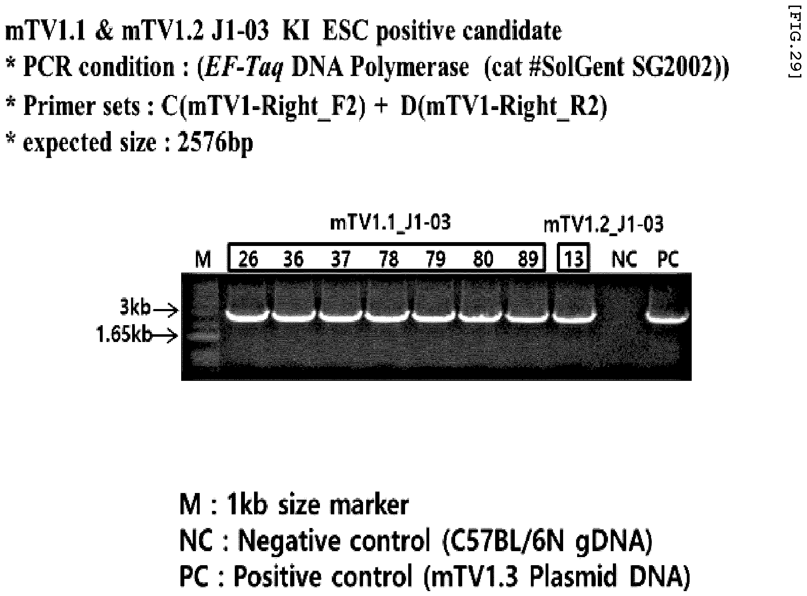

FIGS. 27 to 29 show the production of mouse cells having chromosomes in which one or more RRSs are inserted at the 3' end of a variable region of a mouse immunoglobulin locus and show the results of identifying the mouse cells.

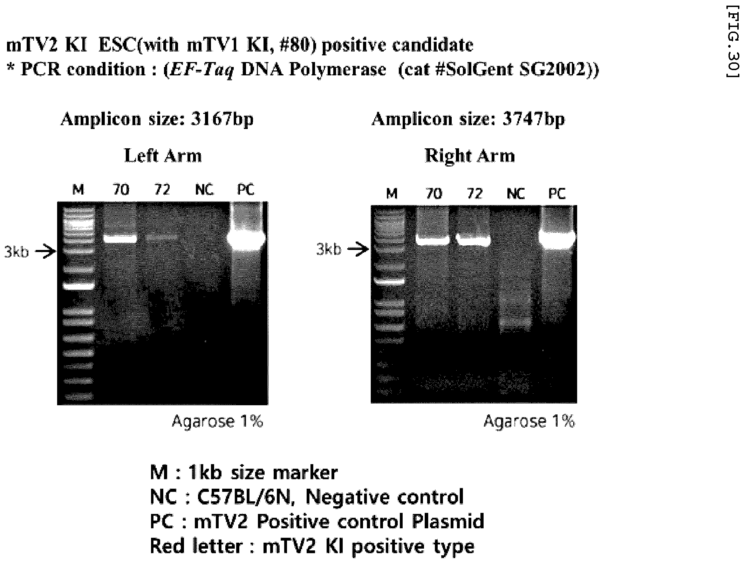

FIG. 30 shows the production of recipient cells having chromosomes in which one or more RRSs are inserted at the 3' end and 5' end of a variable region of a mouse immunoglobulin locus, respectively and show the results of identifying the recipient cells.

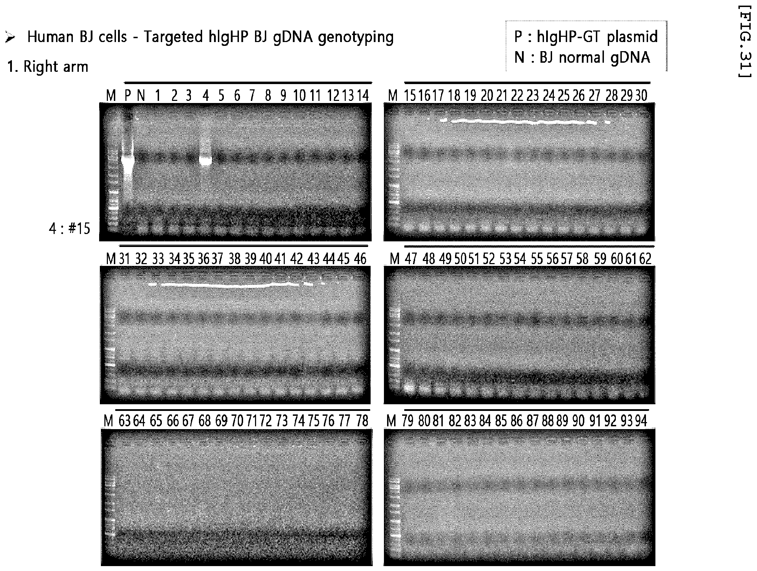

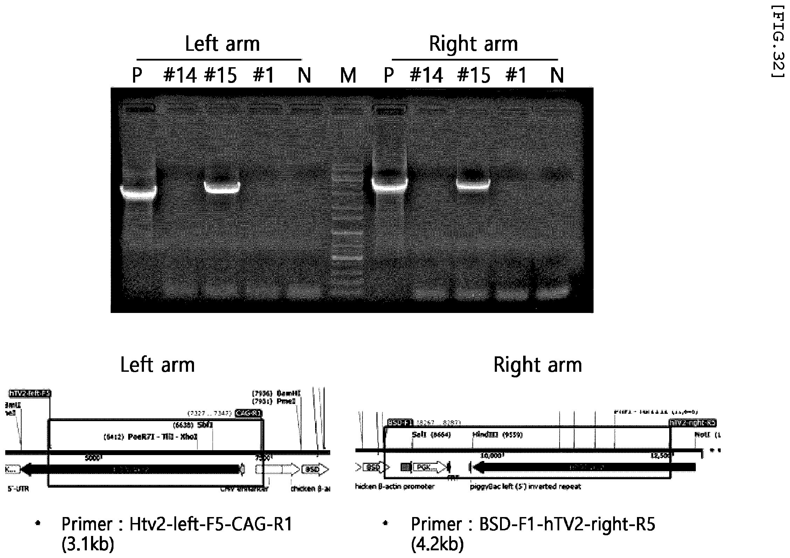

FIGS. 31 and 32 show the production donor cells having donor chromosomes comprising two or more RRSs and a human immunoglobulin gene locus according to one embodiment and show the results of identifying the donor cells. According to the results, the production of human cells with chromosomes in which one or more RRSs are inserted at the 5' end of a variable region of a human immunoglobulin locus was confirmed.

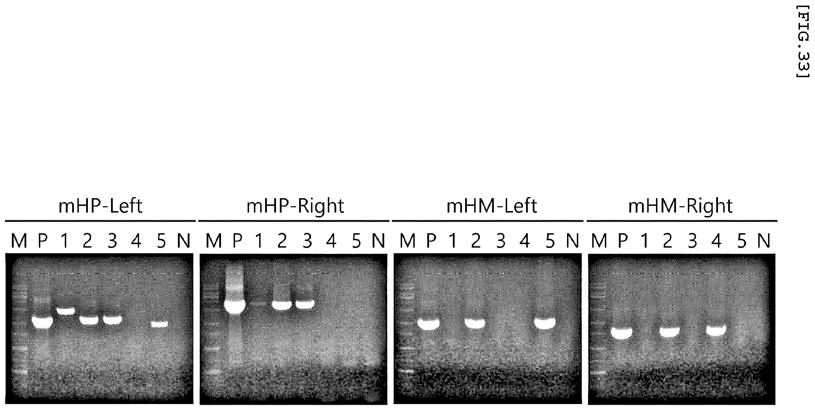

FIG. 33 shows the production of recipient cells having recipient chromosomes in which one or more RRSs are inserted at the 5' end and 3' end of a variable region of a mouse immunoglobulin heavy locus, respectively and show the results of identifying the recipient cells. Sample information is shown below. M is a marker, P is a first or second vector, 1 to 5 are sample numbers, and N is normal J1 ES cell gDNA.

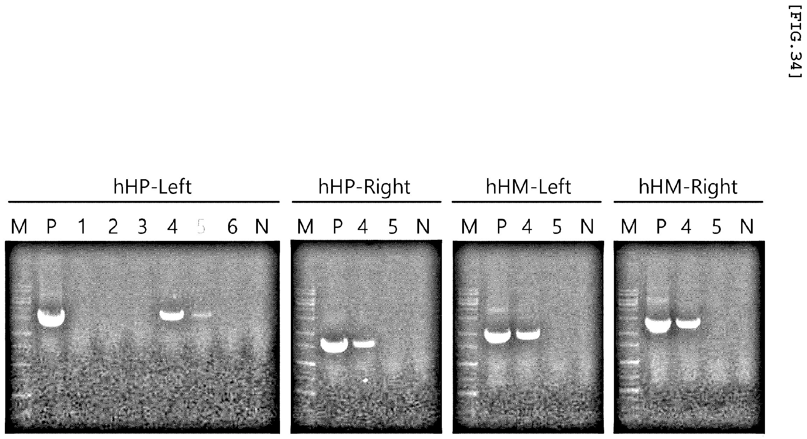

FIG. 34 shows the production of donor cells having donor chromosomes in which one or more RRSs are inserted at the 5' end and 3' end of a variable region of a human immunoglobulin heavy locus, respectively and show the results of identifying the donor cells. Sample information is shown below. M is a marker, P is a third or fourth vector, 1 to 6 are sample numbers, and N is normal BJ cell gDNA.

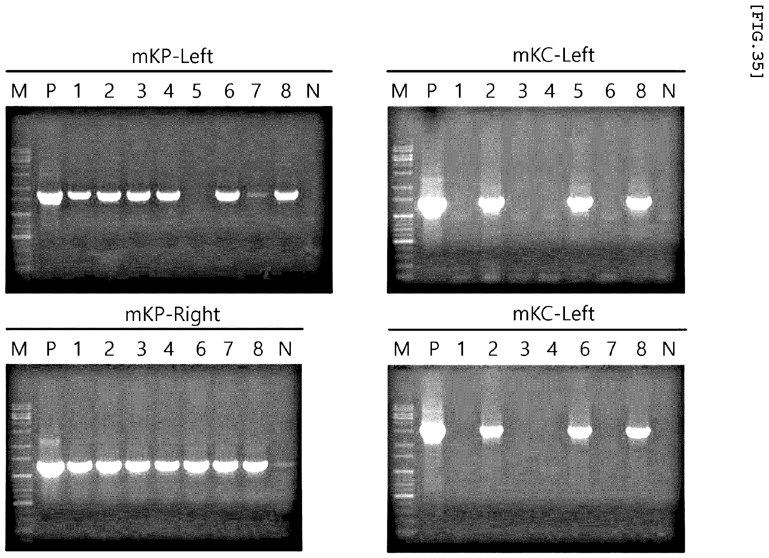

FIG. 35 shows the production of recipient cells having recipient chromosomes comprising one or more RRSs on the 5' end of a variable region of a mouse immunoglobulin kappa locus and on the 3' end of a constant region of a mouse immunoglobulin kappa locus and show the results of identifying the recipient cells. Sample information is shown below. M is a marker, P is a first or second vector, 1 to 8 are sample numbers, and N is normal J1 ES cell gDNA.

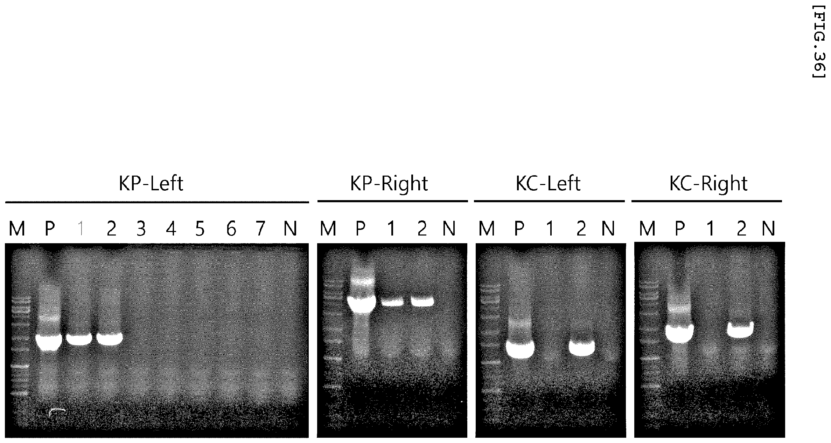

FIG. 36 shows the production of donor cells having donor chromosomes commprising one or more RRSs on the 5' end of a variable region of a human immunoglobulin kappa locus and on the 3' end of a constant region of a human immunoglobulin kappa locus and shows the results of identifying the donor cells. Sample information is shown below. M is a marker, P is a third or fourth vector, 1 to 7 are sample numbers, and N is normal BJ cell gDNA.

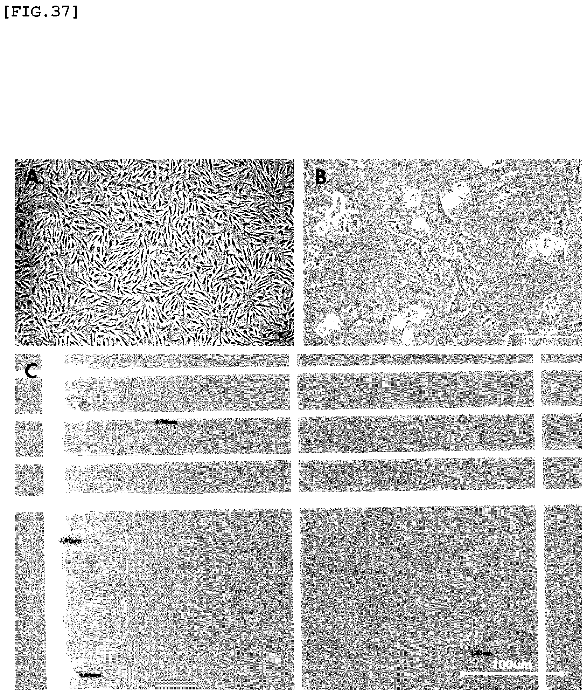

FIG. 37 shows the results of confirming the production of microcells using donor cells (A: targeted BJ cell, B: colcemid-treated cell, and C: microcell).

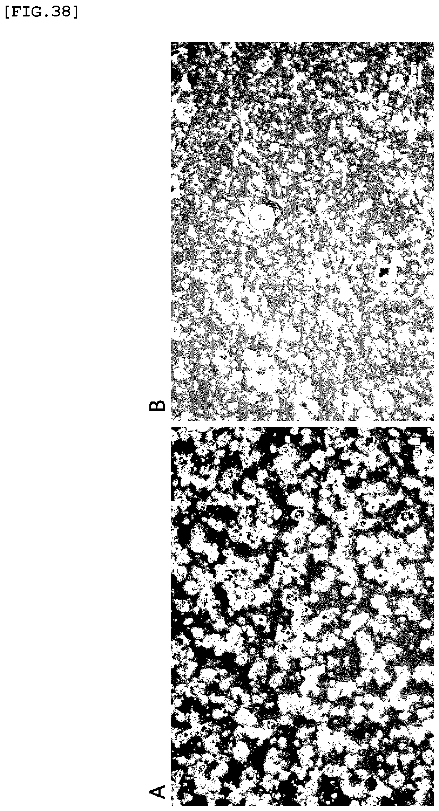

FIG. 38 shows fusion cells obtained by fusing microcells and recipient cells (A: G418 selection after MMCT (Day 3), and B: G418 selection after MMCT (Day 7)).

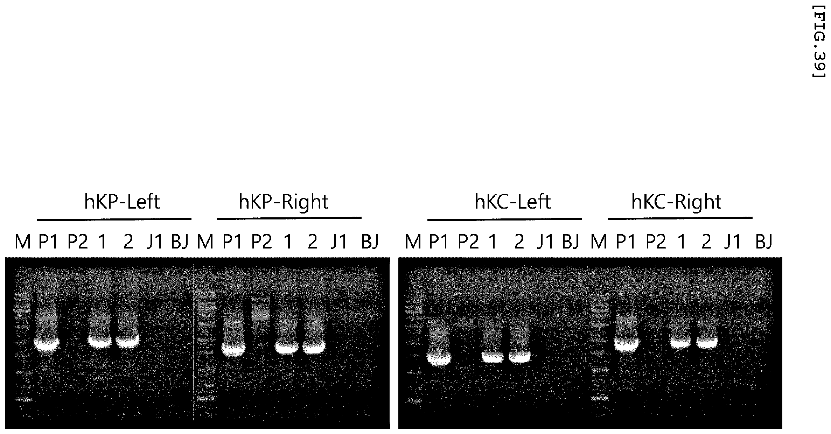

FIG. 39 shows the results of identification of donor chromosomes in which one or more RRSs are inserted at the 5' end of a variable region of a human immunoglobulin kappa locus and the 3' end of a constant region of a human immunoglobulin kappa locus, respectively in the obtained fusion cells. Sample information is shown below. M is a marker, Positive control 1 (P1) is a vector having the same form as the end of a human chromosome (donor chromosome) kappa immunoglobulin variable region including a fourth vector, Positive control 2 (P2) is a vector having the same form as the immunoglobulin kappa gene constant region of a recipient cell after immunoglobulin intrachromosomal exchange via RRS, 1 is a sample with sample number 2 obtained after the first MMCT (G418 selection (Day 8)), 2 is a sample with sampler number 6 obtained after the fifth MMCT (G418 selection (Day 8)), J1 is a normal J1 ES cell gDNA as a negative control, and BJ is a normal BJ cell gDNA as a negative control.

### Best Mode

The details disclosed herein show that artificial recombinant chromosomes can be produced through intrachromosomal recombination, the production being alternative to the existing system using transgenic vectors such as BAC and YAC.

The methods disclosed herein are transfection (transformation) methods using chromosomes rather than using BAC or YAC. The method of transfection using chromosomes is a method of producing transgenic animal cells by inserting a gene of interest into the genome of an animal by introducing one chromosome into an animal cell instead of BAG or YAC conventionally used and for recombination.

The chromosome transfection by the present disclosure comprises largely three processes.

The first process is a process of artificially manipulating chromosomes such that a chromosome including a human immunoglobulin locus and a chromosome including a non-human animal immunoglobulin locus include elements necessary for recombination. The element required for recombination may be RRS. The purpose of the process is to provide a site that can be recognized by recombinases upon intrachromosomal recombination. The process produces a non-human animal cell (recipient cell) with chromosomes having an RRS required for recombination and a non-human animal immunoglobulin locus and a human cell (donor cell) with chromosomes having an RRS required for recombination and a human immunoglobulin locus. In this case, the RRS included in the chromosome of the donor cell is paired with the RRS included in the chromosome of the recipient cell.

The second process is for microcell production and cell fusion. This process directly utilizes donor cells produced in the previous process, in which the microcells produced through this process have chromosomes or fragments having a human immunoglobulin locus and an RRS required for recombination. The fragment includes a human immunoglobulin locus into which the RRS for recombination is inserted. This process may be performed using a known technology, Microcell-Mediated Chromosome Transfer (MMCT). MMCT is a technique commonly used to transfer chromosomes from donor cells to recipient cells (Thorfinn Ege et al., 1974; Thorfinn Ege et al., 1977). The produced microcells fuse with the recipient cells. Through this process, chromosomes having a human immunoglobulin locus and an RRS necessary for recombination of donor cells are introduced (transferred) into the recipient cells through the fusion of microcells.

The third process is a process of producing recombinant non-human animal cells including recombinant chromosomes, using a recombinase. This process is the process of inducing recombination between chromosomes by treating the fusion non-human animal cells produced through cell fusion in the preceding process, with recombinases. When treating with recombinases, recombination between chromosomes having sites that are recognized by recombinases, i.e., RRS required for recombination is induced. As a result, recombinant chromosomes are created by recombination between the two chromosomes and position shift of the gene of interest. A transgenic non-human animals can be produced using recombinant non-human animal cells having the recombinant chromosomes.

The methods disclosed by the present specification have distinctive features described below.
i) Human cells having a chromosome having an endogenous immunoglobulin locus with one or more RRS inserted are used as they are, without cellular alteration.

The prior art used separate cells other than human cells, to insert one or more RRSs into human chromosomes (Yasuhiro Kazuki et al. PNAS, 2019.02.19. vol. 116, no. 8 and US 2019/0254264 A1). However, in the present disclosure, human cells are used as they are to insert one or more RRSs into human chromosomes, and human cells with chromosomes having an endogenous immunoglobulin locus and one or more RRSs are used as donor cells.
ii) The overall chromosomal configuration within cells expressing a humanized immunoglobulin gene is different.

The recombinant chromosomes made herein are incorporated into the configuration of intrinsic chromosomes of non-human animal cells. That is, a system that expresses a foreign gene of interest within the homologous chromosomal configuration with a form of 2n of a transgenic non-human animal is disclosed.

The prior art has been known to use artificial cloning steps, or a method in which separate artificial recombinant chromosomes are generated and added to the intrinsic chromosomal configuration of non-human animal cells. That is, a system expressing a foreign gene of interest within the 2n +1 chromosomal configuration of a transgenic non-human animal is known (Yasuhiro Kazuki et al. PNAS, 2019.02.19. vol. 116, no. 8 and US 2019/0254264 A1).
iii) A recombinant chromosome expressing a humanized immunoglobulin gene has a centromere and/or telomere originating in a recipient cell.

The recombinant chromosomes made herein retain the centromeres and/or telomeres that non-human animal cells have. That is, a system for effectively expressing the humanized immunoglobulin gene due to the same configuration as the centromeres and telomeres of other native chromosomes of a transgenic non-human animal is disclosed.

In the prior art, the configuration of additional recombinant chromosomes is modified so that telomeres are truncated or other chromosomal fragments are deleted. That is, recipient cell-originating centromeres or telomeres were not maintained as they are.
iv) It is effective for the exchange of functionally corresponding immunoglobulin genes between heterologous cells.

The methods disclosed herein are easy to exchange (replace) an immunoglobulin gene positioned in the chromosome of a recipient cell and a corresponding immunoglobulin gene at the corresponding position.

The prior art involves partially exchanging (replacing) an immunoglobulin gene to be exchanged that is located within a chromosome of a recipient cell using a BAG or YAC. In order to completely replace the immunoglobulin gene located in the chromosome of the recipient cell, the partial exchange is repeated according to the capacity limit of BAG or YAC, and this repetition leads to a decrease in yield, high cost, and increase in time.

As such, the methods disclosed by the present specification have the advantage of utilizing the chromosome itself present in the cell without artificial cloning steps. In addition, the method disclosed by the specification significantly reduces the number of sequential introductions over several times using transformation vectors such as BAG and YAC by introducing a large immunoglobulin gene compared to the existing method, thereby solving the problems (efficiency, time, cost, etc.) of the existing method.

### Definition of Terms

Definitions of terms used in this specification are as follows.

### Homologous chromosome

The term "homologous chromosome" means a pair of identical chromosomes present in the nucleus of a cell. Genes relating to the same trait are present at the same location in two chromosomes, and this relationship is called an allele. In this case, the "allele or allelomorphic gene" is a minimal unit for expressing a particular trait present at the same location (the same locus) in the homologous chromosomes. Alleles exist in a pair for each gene in the autosome of a diploid organism with 2n genomes, such as humans, and the alleles in the pair are derived the maternal line and the paternal line, respectively.

### Gene locus (loci)

"Gene locus (loci) or locus (loci)" means a specific position fixed in a chromosome where a particular gene is located. In the case of haploid cells, they have one locus for a particular gene. In the case of diploid cells, they have two loci for a particular gene. In addition, the term "locus or gene locus" means one region that normally transcribes a particular gene. That is, exons, introns, promoters, enhancers, locus control regions (LCRs), etc., are regions within a chromosome that contain all the elements necessary for a particular gene to be expressed normally. For example, the term "immunoglobulin heavy locus (IGH)" refers to the location at which a gene for an immunoglobulin heavy chain is present in a chromosome, and also refers to a region that contains all the necessary elements (promoters, enhancers, variable region genes, constant region genes, etc.) required for the immunoglobulin heavy chain gene to be expressed normally.

### Endogenous immunoglobulin locus

The term "endogenous immunoglobulin locus" refers to an immunoglobulin locus that is naturally present in a cell. Alternatively, the term "endogenous immunoglobulin locus" refers to an immunoglobulin locus that is inherent in the genome of a cell without being externally introduced from the outside of the cell. The endogenous immunoglobulin locus is an endogenous immunoglobulin heavy locus, an endogenous immunoglobulin kappa locus, and/or an endogenous immunoglobulin lambda locus. The endogenous immunoglobulin locus may have an artificial modification that does not affect normal expression of the endogenous immunoglobulin gene. For example, the artificial modification may refer to a form in which an RRS is inserted in an endogenous immunoglobulin locus. Alternatively, the artificial modification may be a form in which a selection factor and a promoter for insertion is inserted in an endogenous immunoglobulin locus.

### The immunoglobulin heavy locus (IGH locus)

The term "immunoglobulin heavy locus (IGH locus)" refers to a fixed position having an immunoglobulin heavy chain gene on a chromosome, in which the immunoglobulin heavy locus includes a variable region including variable (V), diversity (D), and joining (J) segments and a constant region including constant (C) segments. In addition, "immunoglobulin heavy locus (IGH locus)" refers to a region for normal transcription of an immunoglobulin heavy chain genes, including variable (V), diversity (D), joining (J), and constant (C) segments.

The immunoglobulin heavy chain locus comes to have a recombinant V-D-J region generated by recombination of the segments while B cells develop. As B cells develop, the segments included in the immunoglobulin heavy chain loci in the genome of B cells in each developmental process may be changed with recombination. In contrast, a germline immunoglobulin heavy chain locus includes the entirety of V, D, J and C segments that are not recombined, in which the unrecombined V, D and J segments are referred to as "unrearranged variable regions". The unrearranged variable regions of the immunoglobulin heavy chain loci include multiple V segments, multiple D segments, and multiple J segments. For example, in the case of humans, the unrearranged variable regions of an immunoglobulin heavy chain locus may include 38 to 46 V segments, 23 D segments, and 6 J segments. In the case of mice, the unrearranged variable regions of an immunoglobulin heavy chain locus may include 109 V segments, 19 D segments, and 4 J segments.

### Immunoglobulin heavy locus (IGL locus)

The term "immunoglobulin lambda locus (IGL locus)" refers to a fixed position having an immunoglobulin heavy chain gene in a chromosome, the locus including a variable region including variable (V) and joining (J) segments and a constant region including constant (C) segments. In addition, the term "immunoglobulin lambda locus (IGL locus)" refers to a region for normal transcription of an immunoglobulin lambda gene, the locus including variable (V), diversity (D), joining (J), and constant (C) segments.

The immunoglobulin lambda locus includes a recombinant V-J region generated by recombination during the development of B cells. With the development of B cells, the segments included in the immunoglobulin lambda locus in the genome of B cells in each developmental process may vary depending on the recombination. In contrast, a germline immunoglobulin lambda locus includes the entirety of V, J, and C segments that are not recombined, in which the unrecombined V and J segments are referred to as "unrearranged variable region". The unrearranged variable region of the immunoglobulin lambda locus include multiple V segments and multiple J segments. For example, in the case of humans, the unrearranged variable region of an immunoglobulin lambda locus may include 33 V segments and 5 J segments. In the case of mice, the unrearranged variable region of an immunoglobulin lambda locus may include 3 to 8 V segments and 4 J segments.

### Immunoglobulin kappa locus (IGK locus)

The term "immunoglobulin kappa locus (IGK locus)" refers to a fixed position having an immunoglobulin kappa gene in a chromosome, the locus including a variable region including variable (V) and joining (J) segments and a constant region including constant (C) segments. In addition, the term "immunoglobulin kappa locus (IGK locus)" refers to a region for normal transcription of an immunoglobulin kappa gene, the locus including variable (V), joining (J), and constant (C) segments.

The immunoglobulin kappa locus includes a recombinant V-J region generated by recombination during the development of B cells. With the development of B cells, the segments included in the immunoglobulin kappa locus in the genome of B cells in each developmental process may vary depending on the recombination. In contrast, a germline immunoglobulin kappa locus includes the entirety of V, J, and C segments that are not recombined, in which the unrecombined V and J segments are referred to as "unrearranged variable region". The unrearranged variable region of the immunoglobulin kappa locus include multiple V segments and multiple J segments. For example, in the case of humans, the unrearranged variable region of an immunoglobulin kappa locus may include 34 to 48 V segments and 5 J segments. In the case of mice, the unrearranged variable region of an immunoglobulin kappa locus may include 91 V segments and 4 J segments.

### Humanized or humanization

"Humanized or humanization" is a term meaning that the entirety or a portion of a subject is human or of human origin. When the term is used in combination with a gene, i.e., a humanized gene refers to a state in which the entire or partial nucleic acid sequence of a particular gene of a non-human animal is replaced with a nucleic acid sequence of a human gene or has the same nucleic acid sequence as the human gene. For example, the humanized immunoglobulin gene in a mouse may be a gene that contains a variable region of a human immunoglobulin gene instead of a variable region of a mouse immunoglobulin gene, i.e., an endogenous immunoglobulin gene. Alternatively, the mouse's humanized immunoglobulin gene may contain a human immunoglobulin gene instead of a mouse immunoglobulin gene, i.e., a human immunoglobulin gene. Alternatively, the mouse's humanized immunoglobulin gene may be a gene including some segments (V segment) of a variable region of a human immunoglobulin gene instead of a mouse immunoglobulin gene, i.e., a portion (for example, V segment) of a variable region of an endogenous immunoglobulin gene.

In addition, when the term is used in conjunction with a locus, i.e., a humanized locus refers to a state having the entire or partial nucleic acid sequence of a human gene within a specific locus of a non-human animal. For example, a rat humanized immunoglobulin locus is a gene locus including a variable region of a human immunoglobulin gene instead of a variable region of an endogenous immunoglobulin gene in an endogenous immunoglobulin locus. Alternatively, the rat humanized immunoglobulin locus may be a form in which a rat immunoglobulin locus, i.e., a rat endogenous immunoglobulin locus has a constant region of a human immunoglobulin gene instead of a constant region of a rat endogenous immunoglobulin gene. Alternatively, the rat humanized immunoglobulin locus may be a form in which a rat immunoglobulin locus, i.e., a rat endogenous immunoglobulin locus has a human immunoglobulin gene instead of a rat endogenous immunoglobulin gene. Alternatively, the rat humanized immunoglobulin gene may be a form in which a rat immunoglobulin locus, i.e., a rat endogenous immunoglobulin locus has a portion (for example, J segment) of a variable region of a human immunoglobulin gene instead of a portion (for example, J segment) of a variable region of a rat endogenous immunoglobulin gene.

### Recombinase recognition site (RRS)

"Recombinase recognition site (RRS)" means a nucleic acid sequence capable of providing a recombinant site for position-specific recombination. This position-specific recombination uses a site-specific recombinase (SSR) that recognizes a pair of RRSs. In this case, a pair of RRSs paired and an SSR acting on them are referred to as a position-specific recombination system. The position-specific recombination systems vary in RRS depending on the SSR type, and combinations of SSRs and RRSs are known in the art. The position-specific recombination system may be Cre recombinase-loxP, ϕC31 integrase -attP/attB, or transposase-transposon ITR. However, the position-specific recombination system is not limited to those described above, several types of recombinases, integrases, resolvases, or transposases are available as SSRs, and the RRSs used can be varied depending on the SSR.

For example, when the position-specific recombination system is a loxp or a variant thereof, the SSR may be a Cre recombinase. Alternatively, when the RRS is FRT or a variant thereof, the SSR may be flippase (FLP) . Alternatively, when the RRS is attP/attB or a variant thereof, the SSR may be an integrase. Alternatively, when the RRS is an ITR or variant thereof, the SSR may be a tansposase.

The RRS can be a loxP, FRT, attP, attB, an inverted terminal repeat (ITR) sequence, or a variant thereof. However, the RRS is not limited to what is described above.

The RRS may be a known sequence.

For example, the RRS may be LoxP or a variant thereof.

For example, the LoxP variant may be any one or more of Lox m2/71, Lox m2/66, Lox71, and Lox66. The DNA sequences of the loxP variants are shown in Table 1 below. Hereinafter, the sequence numbers are referred to as SEQ ID NOs: 1 to 4.

**[Table 1] DNA sequences of LoxP variants**

| No. | Lox variant | DNA sequence | SEQ ID NO: |
|---|---|---|---|
| 1 | Lox m2/71 | 5'-taccgTTCGTATAtggTttcTTATACGAAGTTAT- 3' | 1 |
| 2 | Lox m2/66 | 5'-ATAACTTCGTATAtggTttcTTATACGAAcggta- 3' | 2 |
| 3 | Lox 71 | 5'-taccgTTCGTATAGCATACATTATACGAAGTTAT- 3' | 3 |
| 4 | Lox 66 | 5'-ATAACTTCGTATAGCATACATTATACGAAcggta- 3' | 4 |

As another example, the RRS may be FRT, attP, attB, ITR (inverted terminal repeat) sequence, or a variant thereof. In this case, the ITR may be a transposon ITR, and the transposon ITR may include a transposon terminal repeat (TR) sequence. For example, the transposon ITR sequence may include a piggyBac terminal repeat (PB-TR).

The DNA sequences of RRSs, such as FRT, attP, attB, and ITRs are shown in Table 2 below. Hereinafter, the sequence numbers are referred to as SEQ ID NOs: 5 to 9.

**[Table 2] DNA sequences of RRSs**

| No. | RRS | DNA sequence | SEQ ID NO: |
|---|---|---|---|
| 1 | FRT | 5'- gaagttcctatactttctagagaataggaacttcgga ataggaacttc-3' | 5 |
| 2 | ϕC31-attP | 5'- cccaggtcagaagcggttttcgggagtagtgccccaa ctggggtaacctttgagttctctcagttgggggcgta gggtcgccgacatgacacaaggggtt-3' | 6 |
| 3 | ϕC31-attB | 5'- ctcgaagccgcggtgcgggtgccagggcgtgcccttg ggctccccgggcgcgtactccacctcacccatc-3' | 7 |
| 4 | PiggyBac right (3') ITR | 5'- ccctagaaagataatcatattgtgacgtacgttaaag ataatcatgcgtaaaattgacgcatg-3' | 8 |
| 5 | PiggyBac left (5') ITR | 5'- catgcgtcaattttacgcagactatctttctaggg- 3' | 9 |

For example, when the RRS for interchromosomal exchange is loxp or a variant thereof, the SSR may be a e recombinase. Alternatively, when the RRS for interchromosomal exchange is FRT or a variant thereof, the SSR may be a flippase (FLP). Alternatively, when the RRS for interchromosomal exchange is attP/attB or a variant thereof, the SSR may be an integrase. Alternatively, when the RRS for interchromosomal exchange is an ITR or a variant thereof, the SSR may be a transposase.

### RRS for inversion

RRS for inversion refers to a pair of paired RRSs to be used for inversion of a particular part within a chromosome. The RRS for inversion is located at both ends of a particular portion to be inverted. For example, when RRSs for inversion are present at both ends of a variable region of an immunoglobulin gene of a donor chromosome, the RRSs for inversion are paired with each other, and the SSR recognizes the paired RRSs, thereby inducing the inversion of the variable region. Since the inversion of a particular portion within a chromosome occurs in a chromatid, the inversing a particular portion using the RRS for inversion means that the RRS for inversion is located in a chromatid.

### RRS for interchromosomal exchange

An RRS for interchromosomal exchange (RRS) is a pair of RRSs to be paired to replace (or exchange) respective specific parts of two chromosomes. RRSs for interchromosomal exchange are located at respective specific parts in two chromosomes to be exchanged. For example, when RRSs for interchromosomal exchange are present such that one RRS is present on a donor chromosome and one RRS is present on a recipient chromosome, then the RRS present on the donor chromosome and the RRS present on the recipient chromosome are paired with each other, and this is recognized by the SSR to cause recombination between the two chromosomes.

### Interchromosomal exchange

"Interchromosomal exchange" means that respective portions of two chromosomes are replaced (or exchanged). For example, when using a first chromosome containing a first-first end and a first-second end and a second chromosome containing a second-first end and a second-second end, the interchromosomal exchange may be performed such that the first-first end of the first chromosome is replaced with the second-first end or the second-second end of the second chromosome. Alternatively, the interchromosomal exchange may be performed such that the first-second ends of the first chromosome are replaced with the second-first end or second-second end of the second chromosome. This chromosomal exchange results in production of a recombinant chromosome in which the first-first end of the first chromosome is exchanged with the second-first end or second-second end or a recombinant chromosome in which the first-second end of the first chromosome is exchanged with the second-first end or second-second end. As another example, the interchromosomal exchange may be performed such that a first region present in the first chromosome is exchanged with a second region present in the second chromosome. This chromosomal exchange results in production of a recombinant chromosome in which a first region within the first chromosome is exchanged with a second region.

Interchromosomal exchange can be caused by RRS for interchromosomal exchange and SSR recognizing it, and this interchromosomal exchange is termed "RRS-SSR mediated chromosomal exchange."

One example of an interchromosomal exchange disclosed herein may be a case where a portion (variable region of an immunoglobulin locus) of a recipient chromosome is replaced (exchanged) with a portion of a donor chromosome.

Another example of interchromosomal exchange disclosed herein may be a case where a portion (immunoglobulin gene) of a recipient chromosome is replaced (exchanged) with a portion of a donor chromosome.

### Selection element

"Selection element" is an element that facilitates cell selection and includes an antibiotic resistant gene, a fluorescent protein gene, and the like.

The antibiotic resistant gene may be, but is not limited to, one or more selected from a hygromycin resistant gene, a neomycin resistant gene, a kanamycin resistant gene, a blasticidin resistant gene, a zeocin resistant gene, and a puroΔTK gene.

The fluorescent protein gene may be, but is not limited to, any one or more selected from among a GFP gene, a YFP gene, an RFP gene, and a mCherry gene.

### Positive selection

"Positive selection" means selection of cells containing a particular selection element under a particular condition. For example, when cells containing an antibiotic resistant gene are mixed with cells not containing an antibiotic resistant gene, when the cells are treated with antibiotics, only the cells containing an antibiotic resistant gene are survived. This can be referred to as positive selection under a particular condition (antibiotic treatment).

### Negative selection

"Negative selection" means selection of cells containing a particular selection element under a particular condition. For example, cells containing an antibiotic sensitive gene are mixed with cells not containing an antibiotic sensitive gene, when the cells are treated with an antibiotic, only the cells not containing an antibiotic sensitive gene are survived. This can be referred to as a negative selection under a particular condition (antibiotic treatment).

### Recipient chromosome

Recipient chromosome refers to an engineered non-human animal chromosome containing one or more RRSs and an endogenous immunoglobulin locus. That is, a recipient chromosome refers to a chromosome having a form in which one or more RRSs are inserted in a wild-type non-human animal chromosome having an endogenous immunoglobulin locus. The endogenous immunoglobulin locus is an immunoglobulin heavy chain locus, an immunoglobulin lambda locus, or an immunoglobulin kappa locus. For example, when the non-human animal is a mouse, the wild-type non-human animal chromosome (mouse chromosome 12) having an endogenous immunoglobulin locus may be a chromosome having a mouse immunoglobulin heavy chain locus, a chromosome (mouse chromosome 16) having a mouse immunoglobulin lambda locus, or a chromosome (mouse chromosome 6) having a mouse immunoglobulin kappa locus. The recipient chromosome may be a chromosome (mouse chromosome 12) having a mouse immunoglobulin heavy chain locus with one or more RRS inserted, a chromosome (mouse chromosome 16) having a mouse immunoglobulin lambda locus with one or more RRS inserted, or a chromosome (mouse chromosome 6) having a mouse immunoglobulin kappa locus with one or more RRS inserted.

Herein, a non-human animal cell containing one or more recipient chromosomes is referred to as a recipient cell.

### Donor chromosome

Donor chromosome refers to an engineered human chromosome having one or more RRSs and endogenous immunoglobulin loci. That is, a donor chromosome refers to a chromosome having a form in which one or more RRSs are inserted in a wild-type human chromosome having an endogenous immunoglobulin locus. The endogenous immunoglobulin locus is an immunoglobulin heavy chain locus, an immunoglobulin lambda locus, or an immunoglobulin kappa locus. Here, the wild-type human chromosome having an endogenous immunoglobulin locus is a chromosome (human chromosome 14) having a human immunoglobulin heavy chain locus, a chromosome (human chromosome 22) having a human immunoglobulin lambda locus, or a chromosome (human chromosome 2) having a human immunoglobulin kappa locus. The donor chromosome may be a chromosome (human chromosome 14) having a human immunoglobulin heavy chain locus with one or more RRSs inserted, a chromosome (human chromosome 22) having a human immunoglobulin lambda locus with one or more RRSs inserted, or a chromosome (human chromosome 2) having a human immunoglobulin kappa locus with one or more RRSs inserted.

Herein, a human cell containing one or more donor chromosomes is referred to as a donor cell.

### Microcell

"Microcell" means any one of two or more parts generated by separation from one cell by artificial manipulation, by exposure to a particular reagent, or by exposure to a specific condition. In addition, the microcell refers to a cellular analog that include one or more chromosomes or chromosomal fragments but cannot perform meiosis or mitosis. For example, the microcells may be two or more cells or cellular analogs separated from one animal cell. In another example, the microcells may be 2n or more chromosomes separated from one animal cell. For example, when the animal cell is a human fibroblast, the microcells may be 2n (46) or more chromosomes separated from a human fibroblast. In this case, the human fibroblast can be separated into 46 or more microcells.

### Cell fusion

"Cell fusion" means a fusion between two or more cells, and/or a fusion between one or more cells and one or more cellular analogs. The fusion may result in a single cell formed through binding (or mixing) of two or more cells, and/or a single cell formed through binding (or mixing) of one or more cells and one or more cell analogs. The cellular analog may be a cell or a cell-derived substance that includes a portion of the genome of the cell analog or some of all chromosomes, but which cannot perform normal mitosis or meiosis. For example, the cellular analog may be a microcell.

Herein, the cell fusion may be a fusion of a recipient cell and one or more microcells. In this case, the one or more microcells may be produced from a donor cell, in which the donor cell may be used for direct microcell production without involving a separate process such as fusion with another cell. The one or more microcells may include a donor chromosome or a fragment of a donor chromosome.

### Fusion cell (fusion non-human animal cell)

"Fusion cell" refers to a cell produced such that non-human animal cell has one or more exogenous chromosomes or fragments of exogenous chromosomes, and is referred to as a fusion non-human animal cell. The one or more exogenous chromosomes or fragments of exogenous chromosomes are externally introduced chromosomes or fragments of chromosomes that are not naturally occurring in a non-human animal cell. The one or more exogenous chromosomes or the fragments of exogenous chromosomes may be chromosomes derived from a species other than a non-human animal cell. For example, when the non-human animal cell is a mouse cell, the fusion cell is a fusion mouse cell. Herein, the fusion cell may include a chromosome derived from a species (for example, human or primate) other than a mouse or a fragment of the chromosome.

Herein, the fusion cells may be cells having a donor chromosome or a fragment of a donor chromosome in the recipient cell.

### Recombinant chromosome

"Recombinant chromosome" means a recombined chromosome produced by interregional exchange between two or more chromosomes derived from two or more cells, i.e., between a donor chromosome and a recipient chromosome. In addition, the recombinant chromosome also refers to a chromosome produced by replication of a chromosome recombined by an intrachromosomal exchange (replacement) between two or more chromosomes derived from two or more cells, i.e., a recipient chromosome and a donor chromosome. For example, the recombinant chromosome may be a chromosome recombined by a specific region replacement (or exchange) between a donor chromosome derived from a donor cell and a recipient chromosome derived from a recipient cell.

Herein, a non-human animal cell containing one or more recombinant chromosomes is referred to as a recombinant non-human animal cell.

Herein, the recombinant chromosome may be a recombinant chromosome through replacement (or exchange) of a particular region (i.e., entirety or part of an immunoglobulin locus) of a recipient chromosome and a particular region of a donor chromosome.

### Non-human animal chromosome containing a humanized immunoglobulin locus

One example of a non-human animal chromosome including a humanized immunoglobulin locus is a chromosome recombined by exchange (replacement) between a particular region of a donor chromosome derived from a human cell (donor cell) and a particular region of a recipient chromosome derived from a non-human animal cell (recipient cell). The particular region includes the entirety or part of an immunoglobulin gene.

For example, the non-human animal chromosome including a humanized immunoglobulin locus may be a non-human animal chromosome having an immunoglobulin locus having a variable region of a human immunoglobulin gene. The non-human animal chromosome including a humanized immunoglobulin locus may not include a variable region of an endogenous immunoglobulin gene.

In one embodiment, the non-human animal chromosome having a humanized immunoglobulin locus may be a non-human animal chromosome having a variable region of a human immunoglobulin heavy chain gene and a constant region of a non-human animal immunoglobulin heavy chain gene. In another embodiment, the non-human animal chromosome including a humanized immunoglobulin locus may be a non-human animal chromosome including a variable region of a human immunoglobulin kappa gene and a constant region of a non-human animal immunoglobulin kappa gene. In a further embodiment, the non-human animal chromosome including a humanized immunoglobulin locus may be a non-human animal chromosome including a variable region of a human immunoglobulin lambda gene and a constant region of a non-human animal immunoglobulin lambda gene. In a yet further embodiment, the non-human animal chromosome having a humanized immunoglobulin locus may be a non-human animal chromosome including a variable region of a human immunoglobulin lambda gene and a constant region of a non-human animal immunoglobulin kappa gene. In a yet further embodiment, the non-human animal chromosome including a humanized immunoglobulin locus may be a non-human animal chromosome including a variable region of a human immunoglobulin kappa gene and a constant region of a non-human animal immunoglobulin lambda gene. The embodiments described are merely examples, and various combinations between immunoglobulin genes (heavy chain, kappa, lambda) are possible, and thus various non-human animal chromosomes including a humanized immunoglobulin locus can be manufactured.

For example, the non-human animal chromosome including a humanized immunoglobulin locus may be a non-human animal chromosome having the entirety (i.e., variable region and constant region) of a human immunoglobulin gene. Herein, the non-human animal chromosome including a humanized immunoglobulin locus may not include a variable region and/or constant region of an endogenous immunoglobulin gene.

In one embodiment, the non-human animal chromosome including a humanized immunoglobulin locus may be a non-human animal chromosome having a variable region of a human immunoglobulin heavy chain gene and a constant region of a non-human animal immunoglobulin heavy chain gene. In another embodiment, the non-human animal chromosome including a humanized immunoglobulin locus may be a non-human animal chromosome including a variable region and a constant region of a human immunoglobulin kappa gene. In a yet further embodiment, the non-human animal chromosome including a humanized immunoglobulin locus may be a non-human animal chromosome including a variable region and a constant region of a human immunoglobulin lambda gene.

Unless otherwise defined, all terms including technical and scientific terms used herein have the same meaning as commonly understood by those who are ordinarily skilled in the art to which the present invention pertains. While methods and materials similar or identical to those described herein may be used for execution or testing of the present invention, suitable methods and materials are described below. All publications, patents, and other references mentioned herein are incorporated by reference in their entirety. In addition, the materials, methods, and examples are only illustrative and are not intended to be limiting.

Hereinafter, the present invention will be described in detail.

### One aspect disclosed herein relate to a method for producing a transgenic cell having a genome including a humanized immunoglobulin locus.

More specifically, the production method relates to a method for producing a recombinant cell having a recombinant chromosome including a humanized immunoglobulin locus, using cell fusion and interchromosomal exchange. The transgenic cell produced by the production method is a non-human animal cell having a humanized immunoglobulin locus. The non-human animal cell is a cell derived from a non-human animal, the cell being capable of producing antibodies or human antibodies.

The production method includes the following:
i) preparing a vector for insertion of RRS;
ii) preparing a non-human animal cell having a recipient chromosome including an immunoglobulin locus with an RRS inserted;
iii) preparing a human cell having a donor chromosome including an immunoglobulin locus with an RRS inserted;
iv) producing a microcell, using the human cell;
v) producing a fusion non-human animal cell by fusing at least one microcell produced in the iv) and the non-human animal cell;
vi) producing a recombinant non-human animal cell having a recombinant chromosome including a humanized immunoglobulin locus by treating the fusion non-human animal cell with a recombinase.

In this case, the step i) may be omitted when using a known material (for example, a vector having a known configuration, a vector available on the market, etc.).

In this case, the steps ii) and iii) may be performed sequentially, in reverse order, or simultaneously.

Hereinafter, each of the steps will be described in detail.

### i) Preparation of vector for insertion of RRS

This step is a step of preparing a vector for producing at least two chromosomes to be used for exchange for production of a recombinant artificial chromosome, through interchromosomal exchange. The at least two chromosomes used for exchange are a donor chromosome and a recipient chromosome. The donor chromosome is a chromosome including an immunoglobulin locus with one or more RRSs inserted for recombination, and the recipient chromosome is a chromosome including an immunoglobulin locus with one or more RRSs inserted for recombination. The donor chromosome originates in a human and the recipient chromosome originates in a non-human animal.

For example, the donor chromosome may be a human chromosome (human chromosome 14) including an immunoglobulin heavy chain locus with one or more RRSs inserted. The recipient chromosome may be a chromosome derived from a non-human animal, for example, mouse. In this case, the recipient chromosome may be a mouse chromosome (mouse chromosome 12) including an immunoglobulin heavy chain locus with one or more RRSs inserted.

For another example, the donor chromosome may be a human chromosome (human chromosome 2) including an immunoglobulin light chain (kappa) locus with one or more RRSs inserted or a human chromosome (human chromosome 22) including an immunoglobulin light chain (lambda) locus. The recipient chromosome may be a chromosome derived from a non-human animal, for example, mouse. In this case, the recipient chromosome may be a mouse chromosome (mouse chromosome 6) including an immunoglobulin light chain (kappa) locus with one or more RRSs inserted or a mouse chromosome (mouse chromosome 16) including an immunoglobulin light chain (lambda) locus.

### i-1) Vector for inserting one RRS

When using a pair of RRSs for interchromosomal exchange, each of the donor chromosome and the recipient chromosome used for interchromosomal exchange includes at least one RRS.

To prepare a donor chromosome with at least one RRSs inserted and a recipient chromosome with at least one RRSs inserted, at least two vectors are designed. In this case, the two vectors each include at least one RRS.

The two vectors consist of a vector for a recipient chromosome for producing a recipient chromosome and a vector for a donor chromosome for producing a donor chromosome.

The two vectors are designed to be organizationally related to each other.

For example, for chromosome exchange between the recipient chromosome and the donor chromosome, the vector for a recipient chromosome and the vector for a donor chromosome need to be designed to include RRSs to be paired with each other. The RRS included in the vector a recipient chromosome is paired with the RRS included in the vector for a donor chromosome. For example, the vector for a recipient chromosome may include an RRS that pairs with an RRS included in the vector for a donor chromosome (see FIGS. 1 to 3).

Hereinafter, the two vectors (that is, the vector for a recipient chromosome and the vector for a donor chromosome) are described below as one vector set.

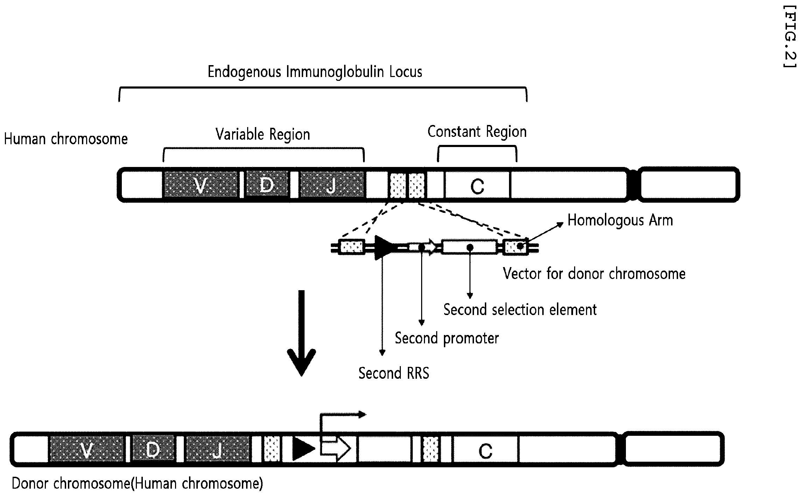

In one embodiment, one vector set (first vector set) may have the following configuration (see FIGS. 1 and 2):
a vector for a recipient chromosome, which includes a first promoter, a first selector, and a first RRS; and
a vector for a donor chromosome, which includes a second RRS, a second promoter, and a second selector.

The vector set is a set of vectors for inserting at least one RRS into each of the recipient chromosome and the donor chromosome, and is hereinafter referred to as a first vector set.

The vector for a recipient chromosome and the vector for a donor chromosome may each include at least one RRS.

The first RRS and the second RRS may be RRSs for interchromosomal exchange. In this case, the first RRS pairs with the second RRS. Each of the first RRS and the second RRS may be one selected from Loxp, FRT, attP, attB, ITR, and variants.

The first selection element may be operably linked to the first promoter. The first selection element may be an antibiotic resistant gene or a fluorescent protein gene. In this case, the first selection element may be a selection element capable of positive and negative selections. For example, the selection element for positive and negative selections may be, but are not limited to, the puroΔTK gene.

The second selection element may be operably linked to the second promoter. The second selection element may be an antibiotic resistant gene or a fluorescent protein gene.

The antibiotic resistant gene may be, but is not limited to, one or more selected from a hygromycin resistant gene, a neomycin resistant gene, a kanamycin resistant gene, a blasticidin resistant gene, a zeocin resistant gene, and a puroΔTK gene.

The fluorescent protein gene may be, but is not limited to, any one or more selected from among a GFP gene, a YFP gene, an RFP gene, and a mCherry gene.

The second selection element included in the vector for a donor chromosome may be different from the first selection element.

The first selection element included in the recipient chromosome may be a selection element capable of positive and negative selections. For example, the selection element for positive and negative selections may be, but are not limited to, the puroΔTK gene.

The vector for a recipient chromosome may further include a homologous arm having homology with a portion of the recipient chromosome to enable insertion into the recipient chromosome.

The vector for a donor chromosome may further include a homologous arm having homology with a portion of the recipient chromosome for insertion into the donor chromosome.

Each of the vector for a recipient chromosome and/or the vector for a donor chromosome may further include at least one RRS.

### i-2) Vector for inserting two or more RRSs

When using two pairs of RRSs for interchromosomal exchange, each of the donor chromosome and the recipient chromosome used for interchromosomal exchange includes two or more RRSs.

To prepare a donor chromosome with two or more RRSs inserted and a recipient chromosome with two or more RRSs inserted, at least four vectors are designed. In this case, the four vectors each include at least one RRS.

The four vectors consist of two vectors for the production of a recipient chromosome and two vectors for the production of a donor chromosome. In this case, the two vectors for the production of a recipient chromosome are vectors for a recipient chromosome and consist of a first vector and a second vector. In this case, the two vectors for the production of a donor chromosome are vectors for a donor chromosome and consist of a third vector and a fourth vector.

The four vectors (that is, first, second, third, and fourth vectors) are designed to have a mutually organizational relationship to each other.

For example, the first and second vectors, which are vectors for a recipient chromosome, need to be inserted into one chromosome. The third and fourth vectors, which are vectors for a donor chromosome, need to be inserted into one chromosome. Eukaryotic cell has a pair of homologous chromosomes. That is, there is a pair of chromosomes that include the same immunoglobulin locus. Thus, the chromosomes into which the first and second vectors (or the third and fourth vectors) can be inserted are also a pair of chromosomes (two chromosomes). For this reason, to produce one chromosome into which both the first and second vectors (or both the third and fourth vectors) are inserted, the first and second vectors (or the third or fourth vectors) may include components closely related to each other. For example, when the first and second vectors (or the third and fourth vectors) are inserted into one chromosome, the selection element included in the second vector (or the fourth vector) may be expressed by the promoter included in the first vector (or the third vector). In this case, the first vector (or third vector) includes a first RRS (inversion), and the second vector (or fourth vector) includes a second RRS (inversion). When both the first and second vectors (or the third and fourth vectors) are inserted into one chromosome, the inverted selection element included in the second vector (or the fourth vector) is re-inverted by the inversion RRSs (first RRS and second RRS) and operably linked with the promoter included in the first vector (or the third vector) such that the selection element can be expressed (FUGS, 4 to 6).

For another example, for chromosome exchange between the recipient chromosome and the donor chromosome, the first, second, third, and fourth vectors need to be designed to include RRSs to be paired with each other. The RRSs included in the first and second vectors that are vectors for a recipient chromosome are paired with the RRSs included in the third and fourth vectors that are vectors for a donor chromosome. For example, the first vector that is a vector for a recipient chromosome may include an RRS that pairs with an RRS that is included in one of the third and fourth vectors that are vectors for a donor chromosome, and the second vector may include an RRS that pairs with an RRS that is included in the remaining one of the third and fourth vectors (see FIGS. 7 to 9) .

Accordingly, the four vectors (that is, the first vector, the second vector, the third vector, and the fourth vector) are designed to be relevant each other, taking into account insertion, recombination, and the like into one chromosome.

Hereinafter, the four vectors (that is, first, second, third, and fourth vectors) are referred to as one vector set.

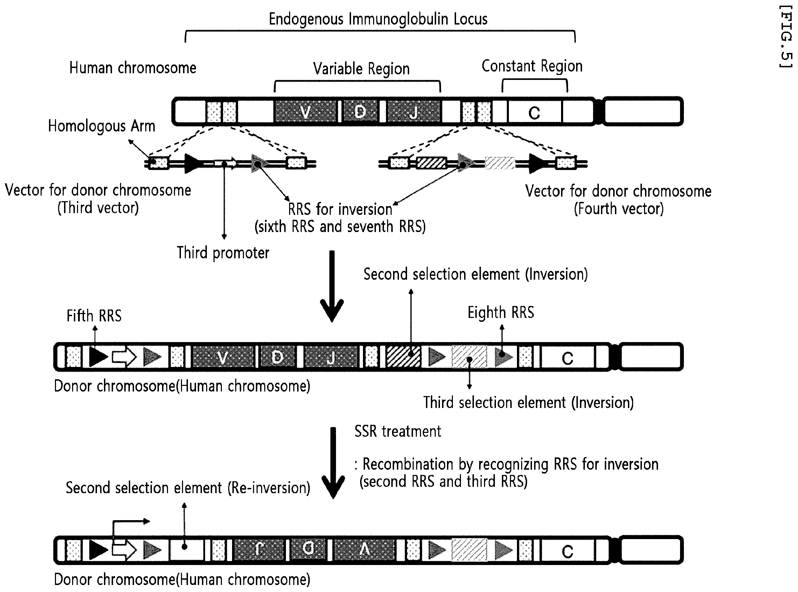

In one embodiment, one vector set (second vector set) may have the following configuration (see FIGS. 4 and 5):
a first vector including a first promoter, a first RRS, a second promoter, and a second RRS;
a second vector including a first selection element, a third RRS, and a fourth RRS; and
a third vector including a fifth RRS, a third promoter, and a sixth RRS; and
a fourth vector including a second selection element, a seventh RRS, a third selection element, and an eighth RRS.

The vector set is a set of vectors for inserting at least two RRSs into each of the recipient chromosome and the donor chromosome, and is hereinafter referred to as a second vector set.

The first and second vectors may be vectors for a recipient chromosome, and the third and fourth vectors may be vectors for a donor chromosome.

The first vector, the second vector, the third vector, and the fourth vector may each include at least two RRSs different from each other. In this case, the RRSs contained in one vector may not be paired with each other.

The first RRS and the eighth RRS may be RRSs for interchromosomal exchange. In this case, the first RRS pairs with the eighth RRS. In this case, each of the first RRS and the eighth RRS may be one selected from Loxp, FRT, attP, attB, ITR, and variants.

The fourth RRS and the fifth RRS may be RRSs for interchromosomal exchange. In this case, the fourth RRS pairs with the fifth RRS. In this case, each of the fourth RRS and the fifth RRS may be any one selected from Loxp, FRT, attP, attB, ITR, and variants.

The second RRS and the third RRS may be RRSs for inversion. In this case, the second RRS pairs with the third RRS. In this case, each of the second RRS and the third RRS may be any one selected from Loxp, FRT, attP, attB, ITR, and variants.

The sixth RRS and the seventh RRS may be RRSs for inversion. In this case, the second RRS pairs with the seventh RRS. In this case, each of the sixth RRS and the seventh RRS may be any one selected from Loxp, FRT, attP, attB, ITR, and variants.

The first selection element may not be operably linked to the promoter. In this case, the first selection element may be in the form of an inversion. The first selection element may be an antibiotic resistant gene or a fluorescent protein gene.

The second selection element may not be operably linked to the promoter. In this case, the second selection element may be in the form of an inversion. The second selection element may be an antibiotic resistant gene or a fluorescent protein gene.

The third selection element may not be operably linked to the promoter. In this case, the third selection element may be in the form of an inversion. The third selection element may be an antibiotic resistant gene or a fluorescent protein gene.

The antibiotic resistant gene may be, but is not limited to, one or more selected from a hygromycin resistant gene, a neomycin resistant gene, a kanamycin resistant gene, a blasticidin resistant gene, a zeocin resistant gene, and a puroΔTK gene.

The fluorescent protein gene may be, but is not limited to, any one or more selected from among a GFP gene, a YFP gene, an RFP gene, and a mCherry gene.

The first promoter may be a promoter for the third selection element included in the fourth vector. When the first RRS and the eighth RRS are recombined in the subsequent process, the third selection element may be operably linked to the first promoter.

The second promoter may be a promoter for the first selection element included in the second vector. When inversion is performed by the second RRS and the third RRS in the subsequent process, the first selection element may be operably linked to the second promoter.

The third promoter may be a promoter for the second selection element included in the fourth vector. When inversion is performed by the sixth RRS and the seventh RRS in the subsequent process, the second selection element may be operably linked to the third promoter.

Each of the first vector and the second vector may further include a homologous arm having homology with a portion of the recipient chromosome to be inserted into the recipient chromosome.

Each of the third vector and the fourth vector may further include a homologous arm having homology with a portion of the recipient chromosome to be inserted into the donor chromosome.

Each of the first vector, the second vector, the third vector, and the fourth vector may optionally include a selection element.

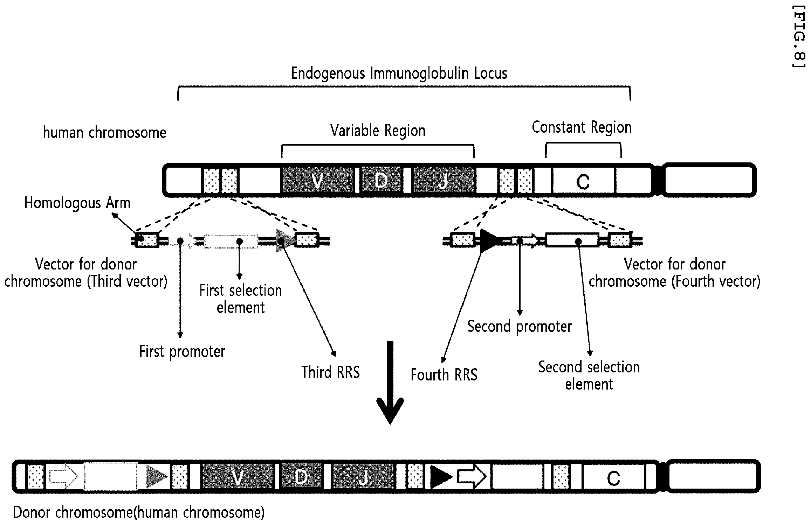

In another embodiment, one vector set (third vector set) may have the following configuration (see FIGS. 7 and 8):
a first vector including a first RRS, a first promoter, and a first selection element;
a second vector including a second selection element, a second selection element, and a second RRS; and
a third vector including a third promoter, and a third selection element, and a third RRS; and
a fourth vector including a fourth RRS, a fourth promoter, and a fourth selection element.

The vector set is a set of vectors for inserting at least two RRSs into each of the recipient chromosome and the donor chromosome, and is hereinafter referred to as a third vector set.

The first and second vectors may be vectors for a recipient chromosome, and the third and fourth vectors may be vectors for a donor chromosome.

Each of the first vector, the second vector, the third vector, and the fourth vector may include one RRS.

The first RRS and the third RRS may be RRSs for interchromosomal exchange. In this case, the first RRS pairs with the third RRS. In this case, each of the first RRS and the third RRS may be any one selected from Loxp, FRT, attP, attB, ITR, and variants.

The second RRS and the fourth RRS may be RRSs for interchromosomal exchange. In this case, the second RRS pairs with the fourth RRS. In this case, each of the second RRS and the fourth RRS may be any one selected from Loxp, FRT, attP, attB, ITR, and variants.

The first selection element may be operably linked to the first promoter. The first selection element may be an antibiotic resistant gene or a fluorescent protein gene.

The second selection element may be operably linked to the second promoter. The second selection element may be an antibiotic resistant gene or a fluorescent protein gene.

The third selection element may be operably linked to the third promoter. The third selection element may be an antibiotic resistant gene or a fluorescent protein gene.

The fourth selection element may be operably linked to the fourth promoter. The fourth selection element may be an antibiotic resistant gene or a fluorescent protein gene.

The antibiotic resistant gene may be, but is not limited to, one or more selected from a hygromycin resistant gene, a neomycin resistant gene, a kanamycin resistant gene, a blasticidin resistant gene, a zeocin resistant gene, and a puroΔTK gene.

The fluorescent protein gene may be, but is not limited to, any one or more selected from among a GFP gene, a YFP gene, an RFP gene, and a mCherry gene.

At least one of the first and second selection elements included in the vectors for a recipient chromosome may be a selection element capable of positive and negative selections. For example, the selection element for positive and negative selections may be, but are not limited to, the puroΔTK gene.

At least one of the third and fourth selection elements included in the vectors for a donor chromosome may be a selection element different from the first and second selection elements.

Each of the first vector and the second vector may further include a homologous arm having homology with a portion of the recipient chromosome to be inserted into the recipient chromosome.

Each of the third vector and the fourth vector may further include a homologous arm having homology with a portion of the recipient chromosome to be inserted into the donor chromosome.

Each of the first vector, the second vector, the third vector, and the fourth vector may optionally include a selection element.

The embodiments described are merely illustrative, and each component (RRS, selector, promoter, etc.) can be modified or altered depending on purposes.

### ii) Preparation of a non-human animal cell containing a recipient chromosome with an RRS inserted

This step is a step of producing a recipient chromosome which is one of two chromosomes used for exchange for production of a recombinant artificial chromosome, through interchromosomal exchange. The recipient chromosome originates in a non-human animal and is a chromosome including an immunoglobulin locus with one or more RRSs for recombination. Through this process, a non-human animal cell having a recipient chromosome including an immunoglobulin locus with one or more RRS inserted. The produced non-human animal cell containing a recipient chromosome will be referred to as "recipient cell". Hereinafter, the non-human animal cell containing a recipient chromosome will be referred to as a recipient cell to be distinguished them from non-human animal cells that do not include a recipient chromosome, i.e., non-human animal cells without RRSs inserted.

To produce a recipient chromosome and a recipient cell containing the same, the step utilizes a vector for a recipient chromosome including or more RRSs. The vector for a recipient chromosome is as described in the section "i) preparing a vector for insertion of RRS" described above.

This step produces a recipient cell by providing a vector for a recipient chromosome to a non-human animal cell.

Such non-human animal cells may be cells derived from mouse cells, rat cells, rodent cells, goral cells, rabbit cells, goat cells, pig cells, camel cells, cattle cells, ungulate cells, chimpanzee cells, monkey cells, non-human primates cells, chicken cells, quail cells, or avian cells, but are not limited thereto.

The non-human animal cell may be derived from somatic cells. For example, the somatic cell may be, but is not limited to, a fibroblast cell, for example.

The non-human animal cell may originate in immune cells. For example, the immune cell may be, but is not limited to, a B-cell, a T-cell, an NK cell, a macrophage, a neutrophil, a basophil, or an eosinophil.

The non-human animal cell may be derived from germ cells. For example, the non-human animal cells may be sperm, spermatocytes, garden stem cells, eggs, oocytes, ovary stem cells, or fertilized eggs but may not be limited thereto.

The non-human animal cells may be derived from stem cells. For example, the stem cells may be derived from, but are not limited to, embryonic stem cells (ES cells), adult stem cells, cord blood stem cells, garden stem cells, or ovarian stem cells.

Vectors for such recipient chromosomes may be provided to non-human animal cells using known transfection methods.

For example, the transfection method may be a viral transfection method, a reagent transfection method, or a physical transfection method. The viral transfection method may be performed using, for example, a lentivirus. The reagent transfection method may be performed using, for example, calcium phosphate, cation lipid, DEAE-dextran, and polyethylenimine (PEI). The physical transfection method may be performed, for example, using electroporation. In addition, the transfection may be performed using a liposome, but is not limited thereto.

The vector for a recipient chromosome may be inserted into a chromosome having an endogenous immunoglobulin locus present in a non-human animal cell. The chromosome having an endogenous immunoglobulin locus present in a non-human animal cell may be a chromosome having an endogenous immunoglobulin heavy chain locus, a chromosome having an endogenous immunoglobulin kappa locus, or a chromosome having an endogenous immunoglobulin lambda locus. In this case, the vector for a recipient chromosome may be inserted into an endogenous immunoglobulin locus, thereby producing a recipient chromosome. The produced recipient chromosome may include one or more RRSs at the endogenous immunoglobulin locus.

Through this step, it is possible to obtain recipient cells having a recipient chromosome including one or more RRSs at the endogenous immunoglobulin locus thereof.

### iii) Preparation of a human cell containing a donor chromosome with an RRS inserted

This step is a step of producing a donor chromosome which is one of two chromosomes used for exchange for production of a recombinant artificial chromosome, through interchromosomal exchange. The donor chromosome originates in a human and is a chromosome including an immunoglobulin locus with one or more RRSs inserted for recombination. Through this process, a human cell having a donor chromosome having an immunoglobulin locus with one or more RRS inserted. The produced human cell containing a donor chromosome will be referred to as a "donor cell". Hereinafter, the human cell containing a donor chromosome will be referred to as a donor cell to be distinguished them from human cells that do not include a donor chromosome, i.e., human cells without RRSs inserted.

To produce a donor chromosome and a donor cell containing the same, the step utilizes a vector for a donor chromosome, which includes one or more RRSs. The vector for a donor chromosome is as described in the section "i) preparing a vector for insertion of RRS" described above.

This step produces a donor cell by providing a vector for a donor chromosome to a human cell.

The human cells may be derived from somatic cells. For example, the somatic cell may be, but is not limited to, a fibroblast cell, for example.

The human cell may be derived from immune cells. For example, the immune cell may be, but is not limited to, a B-cell, a T-cell, an NK cell, a macrophage, a neutrophil, a basophil, or an eosinophil.

The human cells may be derived from germ cells. For example, the non-human animal cells may be sperm, spermatocytes, garden stem cells, eggs, oocytes, ovary stem cells, or fertilized eggs but may not be limited thereto.

The human cells may be derived from stem cells. For example, the stem cells may be derived from, but are not limited to, embryonic stem cells (ES cells), adult stem cells, cord blood stem cells, garden stem cells, or ovarian stem cells.

Vectors for such donor chromosomes may be provided to human cells using known transfection methods.

For example, the transfection method may be a viral transfection method, a reagent transfection method, or a physical transfection method. The viral transfection method may be performed using, for example, a lentivirus. The reagent transfection method may be performed using, for example, calcium phosphate, cation lipid, DEAE-dextran, and polyethylenimine (PEI). The physical transfection method may be performed, for example, using electroporation. In addition, the transfection may be performed using a liposome, but is not limited thereto.

The vector for a donor chromosome may be inserted into a chromosome having an endogenous immunoglobulin locus present in a human cell. The chromosome having an endogenous immunoglobulin locus present in a human cell may be a chromosome having an endogenous immunoglobulin heavy chain locus, a chromosome having an endogenous immunoglobulin kappa locus, or a chromosome having an endogenous immunoglobulin lambda locus. In this case, the vector for a donor chromosome may be inserted into an endogenous immunoglobulin locus, thereby producing a donor chromosome. The produced donor chromosome may include one or more RRSs at the endogenous immunoglobulin locus.

Through this step, it is possible to obtain donor cells having a donor chromosome including one or more RRSs at the endogenous immunoglobulin locus thereof.

Hereinafter, various embodiments for the steps ii) and iii) will be described. The embodiments are related to the production of recipient cells and donor cells, using the vectors for insertion of the RRSs described above. The description will be made specifically for vector sets.

### ii-1, iii-1) Production of a recipient cell containing a recipient chromosome having one RRS inserted and a donor cell containing a donor chromosome having one RRS inserted (using a first set of vectors)

In one embodiment, vectors (first vector set) for insertion of one RRS into a non-human animal cell and into a human cell are provided to produce a recipient cell containing a recipient chromosome having one RRS inserted and a donor cell containing a donor chromosome having one RRS inserted (FIGS. 1 and 2) .

The vectors for inserting one RRS into respective cells may be a first set of vectors (hereinafter, referred to as first vector set).

The first vector set includes: a vector for a recipient chromosome, which includes a first promoter, a first selection element, and a first RRS; and a vector for a donor chromosome, which includes a second RRS, a second promoter, and a second selection element. The details thereof are the same as described in the section ""Vector for Inserting one RRS" described above.

More specifically, to prepare a recipient chromosome having one RRS inserted and a recipient cell containing the same recipient chromosome, a vector for a recipient chromosome is provided to a non-human animal cell to produce a recipient cell. In addition, to prepare a donor chromosome having one RRS inserted and a donor cell containing the same donor chromosome, a vector for a donor chromosome may be provided to a human cell to produce a donor cell.

Vectors for such receptive chromosomes may be provided to non-human animal cells using known transfection methods. Vectors for such donor chromosomes may be provided to human cells using known transfection methods. The transfection method, the non-human animal cell, and the human cell are the same as described in the section "ii) preparing a non-human animal cell containing a recipient chromosome with RRS inserted" and the section "iii) preparing a human cell containing a donor chromosome with RRS inserted".

The vector for a recipient chromosome may be inserted into an endogenous immunoglobulin locus present in a non-human animal cell. In this case, the endogenous immunoglobulin locus present in a non-human animal cell may be an endogenous immunoglobulin heavy chain locus, an endogenous immunoglobulin kappa locus, or an endogenous immunoglobulin lambda locus. In this case, the vector for a recipient chromosome may be inserted into the J segment 3' end or downstream of the variable region of the endogenous immunoglobulin locus. The recipient chromosome thus produced may include one RRS (first RRS) at the J segment 3' end or downstream of the variable region of the endogenous immunoglobulin locus.

The vector for a donor chromosome may be inserted into an endogenous immunoglobulin locus present in a human cell. In this case, the endogenous immunoglobulin locus present in a human cell may be an endogenous immunoglobulin heavy chain locus, an endogenous immunoglobulin kappa locus, or an endogenous immunoglobulin lambda locus. In this case, the vector for a donor chromosome may be inserted into the J segment 3' end or downstream of the variable region of the endogenous immunoglobulin locus. The donor chromosome thus produced may include one RRS (second RRS) at the J segment 3' end or downstream of the variable region of the endogenous immunoglobulin locus.

In this case, the first RRS inserted into the recipient chromosome and the second RRS inserted into the donor chromosome are RRSs for intrachromosomal exchange and may be paired with each other.

Through this step, recipient cells with one RRS (first RRS) at the J segment 3' end or downstream of the variable region of the endogenous (non-human) immunoglobulin locus can be obtained. The recipient cells can be obtained through the expression of a first selection element included in the vector for a recipient chromosome.

Through this step, donor cells with one RRS (second RRS) at the J segment 3' end or downstream of the variable region of the endogenous (human) immunoglobulin locus can be obtained. In this case, the donor cells can be obtained through the expression of a first selection element included in the vector for a donor chromosome.

### ii-2, iii-2) Production of a recipient cell containing a recipient chromosome having two or more RRSs inserted and a donor cell containing a donor chromosome having two or more RRSs inserted (using a second set of vectors)

In one embodiment, vectors (second vector set) for insertion of two or more RRSs into each of a non-human animal cell and a human cell are provided to produce a recipient cell containing a recipient chromosome having one RRS inserted and a donor cell containing a donor chromosome having one RRS inserted (FIGS. 4 and 5).

The vectors for inserting two or more RRSs may be a second set of vectors (hereinafter, referred to as second vector set).

The second vector set includes the following, and this point is described in the section "Vectors for inserting two or more RRSs":
a first vector including a first promoter, a first RRS, a second promoter, and a second RRS;
a second vector including a first selection element, a third RRS, and a fourth RRS; and
a third vector including a fifth RRS, a third promoter, and a sixth RRS; and
a fourth vector including a second selection element, a seventh RRS, a third selection element, and an eighth RRS.

In this case, the first and second vectors may be vectors for a recipient chromosome, and the third and fourth vectors may be vectors for a donor chromosome.

More specifically, to prepare a recipient chromosome having two or more RRSs inserted and a recipient cell containing the same recipient chromosome, vectors for a recipient chromosome are provided to a non-human animal cell to produce a recipient cell. In addition, to prepare a donor chromosome having two or more RRSs inserted and a donor cell containing the same donor chromosome, vectors for a donor chromosome may be provided to a human cell to produce a donor cell.

Vectors for such receptive chromosomes may be provided to non-human animal cells using known transfection methods. The first and second vectors, which are vectors for a recipient chromosome, may be provided to a non-human animal cell simultaneously or sequentially. Vectors for such donor chromosomes may be provided to human cells using known transfection methods. In this case, the third and fourth vectors, which are vectors for a donor chromosome, may be provided to human cells simultaneously or sequentially. The transfection method, the non-human animal cell, and the human cell are the same as described in the section "ii) preparing a non-human animal cell containing a recipient chromosome with RRS inserted" and the section "iii) preparing a human cell containing a donor chromosome with RRS inserted".

The first and second vectors, which are vectors for a recipient chromosome, may be inserted into an endogenous immunoglobulin locus present in a non-human animal cell. In this case, the endogenous immunoglobulin locus present in a non-human animal cell may be an endogenous immunoglobulin heavy chain locus, an endogenous immunoglobulin kappa locus, or an endogenous immunoglobulin lambda locus. In one embodiment, the first vector may be inserted into the V segment 5' end or downstream of the variable region of the endogenous immunoglobulin locus. In this case, the second vector may be inserted into the J segment 3' end or downstream of the variable region of the endogenous immunoglobulin locus. The recipient chromosome thus produced may include the first promoter, the first RRS, the second promoter, and the second RRS at the V segment 5' end or upstream of the variable region of the endogenous immunoglobulin locus, and may include the first selection element, the third RRS, and the fourth RRS at the J segment 3' end or downstream. That is, the recipient chromosome includes the first promoter, the first RRS, the second promoter, and the second RRS at the 5' end of the variable region of the endogenous immunoglobulin locus, and includes the first selector, the third RRS, and the fourth RRS at the 3' end. In another embodiment, the second vector may be inserted into the V segment 5' end or upstream of the variable region of the endogenous immunoglobulin locus. In this case, the first vector may be inserted into the J segment 3' end or downstream of the variable region of the endogenous immunoglobulin locus. The recipient chromosome thus produced may include the first selection element, the third RRS, and the fourth RRS at the V segment 5' end or upstream of the variable region of the endogenous immunoglobulin locus, and may include the first promoter, the first RRS, the second promoter, and the second RRSs at the J segment 3' end or downstream. That is, the recipient chromosome includes the first selection element, the third RRS, and the fourth RRS at the 5' end of the variable region of the endogenous immunoglobulin locus, and includes the first promoter, the first RRS, the second promoter, and the second RRS at the 3' end.

Alternatively, the first and second vectors, which are vectors for a recipient chromosome, may be inserted into an endogenous immunoglobulin locus present in a non-human animal cell. In this case, the endogenous immunoglobulin locus present in a non-human animal cell may be an endogenous immunoglobulin heavy chain locus, an endogenous immunoglobulin kappa locus, or an endogenous immunoglobulin lambda locus. In one embodiment, the first vector may be inserted into the V segment 5' end or downstream of the variable region of the endogenous immunoglobulin locus. In this case, the second vector may be inserted into the 3' end or downstream of the constant region of the endogenous immunoglobulin locus. The recipient chromosome thus produced may include the first promoter, the first RRS, the second promoter, and the second RRS at the V segment 5' end or upstream of the variable region of the endogenous immunoglobulin locus, and may include the first selection element, the third RRS, and the fourth RRS at the 3' end or downstream of the constant region. That is, the recipient chromosome includes the first promoter, the first RRS, the second promoter, and the second RRS at the 5' end of the variable region of the endogenous immunoglobulin locus, and includes the first selection element, the third RRS, and the fourth RRS at the 3' end of the constant region. In another embodiment, the second vector may be inserted into the V segment 5' end or upstream of the variable region of the endogenous immunoglobulin locus. In this case, the first vector may be inserted into the 3' end or downstream of the constant region of the endogenous immunoglobulin locus. The recipient chromosome thus produced may include the first selection element, the third RRS, and the fourth RRS at the V segment 5' end or upstream of the variable region of the endogenous immunoglobulin locus, and may include the first promoter, the first RRS, the second promoter, and the second RRSs at the 3' end or downstream of the constant region. That is, the recipient chromosome includes the first selection element, the third RRS, and the fourth RRS at the 5' end of the variable region of the endogenous immunoglobulin locus, and includes the first promoter, the first RRS, the second promoter, and the second RRS at the 3' end of the constant region.

The third and fourth vectors, which are vectors for a donor chromosome, may be inserted into an endogenous immunoglobulin locus present in a human cell. In this case, the endogenous immunoglobulin locus present in a human cell may be an endogenous immunoglobulin heavy chain locus, an endogenous immunoglobulin kappa locus, or an endogenous immunoglobulin lambda locus. In one embodiment, the third vector may be inserted into the V segment 5' end or upstream of the variable region of the endogenous immunoglobulin locus. In this case, the fourth vector may be inserted into the J segment 3' end or downstream of the variable region of the endogenous immunoglobulin locus. The donor chromosome thus produced may include the fifth RRS, the third promoter, and the sixth RRS at the V segment 5' end or upstream of the variable region of the endogenous immunoglobulin locus, and may include the seventh RRS, the second selection element, the third selection element, and the eighth RRS at the J segment 3' end or downstream. That is, the recipient chromosome includes the fifth RRS, the third promoter, and the sixth RRS at the 5' end of the variable region of the endogenous immunoglobulin locus, and includes the second selection element, the seventh RRS, the third selection element, and the eighth RRS at the 3' end. In another embodiment, the fourth vector may be inserted into the V segment 5' end or upstream of the variable region of the endogenous immunoglobulin locus. In this case, the third vector may be inserted into the J segment 3' end or downstream of the variable region of the endogenous immunoglobulin locus. Therefore, the donor chromosome thus produced includes the seventh RRS, the second selection element, the third selection element, and the eighth RRS at the V segment 5' end or upstream of the variable region of the endogenous immunoglobulin locus, and may include the fifth RRS, the third promoter, and the sixth RRS at the J segment 3' end or downstream. That is, the donor chromosome includes the second selection element, the seventh RRS, the third selection element, and the eighth RRS at the 5' end of the variable region of the endogenous immunoglobulin locus, and includes the fifth RRS, the third promoter, and the sixth RRS at the 3' end.

Alternatively, the third and fourth vectors, which are vectors for a donor chromosome, may be inserted into an endogenous immunoglobulin locus present in a human cell. In this case, the endogenous immunoglobulin locus present in a human cell may be an endogenous immunoglobulin heavy chain locus, an endogenous immunoglobulin kappa locus, or an endogenous immunoglobulin lambda locus. In one embodiment, the third vector may be inserted into the V segment 5' end or upstream of the variable region of the endogenous immunoglobulin locus. In this case, the fourth vector may be inserted into the 3' end or downstream of the constant region of the endogenous immunoglobulin locus. The donor chromosome thus produced may include the fifth RRS, the third promoter, and the sixth RRS at the V segment 5' end or upstream of the variable region of the endogenous immunoglobulin locus, and may include the seventh RRS, the second selection element, the third selection element, and the eighth RRS at the 3' end or downstream of the constant region. That is, the recipient chromosome includes the fifth RRS, the third promoter, and the sixth RRS at the 5' end of the variable region of the endogenous immunoglobulin locus, and includes the second selection element, the seventh RRS, the third selection element, and the eighth RRS at the 3' end of the constant region. In a further embodiment, the fourth vector may be inserted into the V segment 5' end or upstream of the variable region of the endogenous immunoglobulin locus. In this case, the third vector may be inserted into the 3' end or downstream of the constant region of the endogenous immunoglobulin locus. Therefore, the donor chromosome thus produced includes the seventh RRS, the second selection element, the third selection element, and the eighth RRS at the V segment 5' end or upstream of the variable region of the endogenous immunoglobulin locus, and may include the fifth RRS, the third promoter, and the sixth RRS at the 3' end or downstream of the constant region. That is, the donor chromosome includes the second selection element, the seventh RRS, the third selection element, and the eighth RRS at the 5' end of the variable region of the endogenous immunoglobulin locus, and includes the fifth RRS, the third promoter, and the sixth RRS at the 3' end of the constant region.

In this case, the first RRS inserted into the recipient chromosome and the eighth RRS inserted into the donor chromosome are RRSs for intrachromosomal exchange and may be paired with each other.

In this case, the fourth RRS inserted into the recipient chromosome and the fifth RRS inserted into the donor chromosome are RRSs for intrachromosomal exchange and may be paired with each other.

In this case, the second RRS inserted into the recipient chromosome and the third RRS inserted into the donor chromosome are RRSs for inversion and may be paired with each other. In this case, the RRS for inversion may be used to determine whether the first vector and the second vector are inserted into the same chromatid. Since the non-human animal cells have chromosomes (homologous chromosomes) with a pair of immunoglobulin loci, it is necessary to confirm that the first and second vectors are inserted into one chromosome. To this end, it can be confirmed that the first and second vectors are inserted into one chromosome by identifying expression of the first selection element because the first selection element becomes operably linked to the second promoter when the SSR for recognition of the RRS for inversion is treated so that the variable region of the endogenous immunoglobulin locus is inverted or the variable region and constant region of the endogenous immunoglobulin locus are inverted.

In this case, the sixth and seventh RRSs inserted into the donor chromosome are RRSs for inversion and may be paired with each other. In this case, the RRS for inversion may be used to determine whether the third and fourth vectors are inserted into the same chromatid. Since human cells have chromosomes (homologous chromosomes) with a pair of immunoglobulin loci, it is necessary to confirm that the third and fourth vectors are inserted into one chromosome. To this end, it can be confirmed that the third and fourth vectors are inserted into one chromosome by identifying expression of the second selection element because the second selection element becomes operably linked to the third promoter when the SSR for recognition of the RRS for inversion is treated so that the variable region of the endogenous immunoglobulin locus is inverted or the variable region and constant region of the endogenous immunoglobulin locus are inverted.

Through this step, recipient cells including a recipient chromosome having the first RRS at the 5' end of the variable region of the endogenous (non-human) immunoglobulin locus and the fourth RRS at the 3' end can be obtained. Alternatively, recipient cells including a recipient chromosome having the fourth RRS at the 5' end of the variable region of the endogenous (non-human) immunoglobulin locus and having the first RRS at the 3' end. Alternatively, recipient cells including a recipient chromosome having the first RRS at the 5 'end of the variable region of the endogenous (non-human) immunoglobulin locus and the fourth RRS at the 3' end of the constant region can be obtained Alternatively, recipient cells including a recipient chromosome having the fourth RRS at the 5' end of the variable region of the endogenous (non-human) immunoglobulin locus and the first RRS at the 3' end of the constant region can be obtained. The recipient cells can be obtained through the expression of the first selection element included in the vector for a recipient chromosome.

In addition, through this step, donor cells including a donor chromosome having the fifth RRS at the 5' end of the variable region of the endogenous (human) immunoglobulin locus and the eighth RRS at the 3' end can be obtained. Alternatively, donor cells including a donor chromosome having the eighth RRS at the 5' end of the variable region of the endogenous (human) immunoglobulin locus and having the fifth RRS at the 3' end can be obtained. Alternatively, donor cells including a donor chromosome having the fifth RRS at the 5' end of the variable region of the endogenous (human) immunoglobulin locus and the eighth RRS at the 3' end of the constant region can be obtained. Alternatively, donor cells including a donor chromosome having the eighth RRS at the 5' end of the variable region of the endogenous (human) immunoglobulin locus and the fifth RRS at the 3' end of the constant region can be obtained. In this case, the donor cells can be obtained through the expression of the second selection element included in the vector for a donor chromosome.

### ii-3, iii-3) Production of a recipient cell containing a recipient chromosome having two or more RRSs inserted and a donor cell containing a donor chromosome having two or more RRSs inserted (using a third set of vectors)

In a further embodiment, vectors (third vector set) for insertion of two or more RRSs into each of a non-human animal cell and a human cell are provided to produce a recipient cell containing a recipient chromosome having two or more RRSs inserted and a donor cell containing a donor chromosome having two or more RRS inserted (FIGS. 7 and 8).

The vectors for inserting two or more RRSs may be a third set of vectors (hereinafter, referred to as third vector set).

The third vector set includes the following, and this point is the same as described in the section "Vectors for inserting two or more RRSs":
a first vector including a first RRS, a first promoter, and a first selection element;
a second vector including a second selection element, a second selection element, and a second RRS; and
a third vector including a third promoter, and a third selection element, and a third RRS; and
a fourth vector including a fourth RRS, a fourth promoter, and a fourth selection element.

In this case, the first and second vectors may be vectors for a recipient chromosome, and the third and fourth vectors may be vectors for a donor chromosome.

More specifically, to prepare a recipient chromosome having two or more RRSs inserted and a recipient cell containing the same recipient chromosome, vectors for a recipient chromosome are provided to a non-human animal cell to produce a recipient cell. In addition, to prepare a donor chromosome having two or more RRSs inserted and a donor cell containing the same donor chromosome, vectors for a donor chromosome may be provided to a human cell to produce a donor cell.

Vectors for such receptive chromosomes may be provided to non-human animal cells using known transfection methods. The first and second vectors, which are vectors for a recipient chromosome, may be provided to a non-human animal cell simultaneously or sequentially. Vectors for such donor chromosomes may be provided to human cells using known transfection methods. In this case, the third and fourth vectors, which are vectors for a donor chromosome, may be provided to human cells simultaneously or sequentially. The transfection method, the non-human animal cell, and the human cell are the same as described in the section "ii) preparing a non-human animal cell containing a recipient chromosome with RRS inserted" and the section "iii) preparing a human cell containing a donor chromosome with RRS inserted".

The first and second vectors, which are vectors for a recipient chromosome, may be inserted into an endogenous immunoglobulin locus present in a non-human animal cell. In this case, the endogenous immunoglobulin locus present in a non-human animal cell may be an endogenous immunoglobulin heavy chain locus, an endogenous immunoglobulin kappa locus, or an endogenous immunoglobulin lambda locus. In one embodiment, the first vector may be inserted into the V segment 5' end or downstream of the variable region of the endogenous immunoglobulin locus. In this case, the second vector may be inserted into the J segment 3' end or downstream of the variable region of the endogenous immunoglobulin locus. The recipient chromosome thus produced may include the first RRS, the first promoter, and the first selection element at the V segment 5' end or upstream of the variable region of the endogenous immunoglobulin locus, and may include the second promotor, the second selection element, and the second RRS at the J segment 3' end or downstream. That is, the recipient chromosome includes the fifth RRS, the first promoter, and the first selection element at the 5' end of the variable region of the endogenous immunoglobulin locus, and includes the second promoter, the second selection element, and the second RRS at the 3' end. In a further embodiment, the first vector may be inserted into the V segment 5' end or upstream of the variable region of the endogenous immunoglobulin locus. In this case, the second vector may be inserted into the C segment 3' end or downstream of the constant region of the endogenous immunoglobulin locus. The recipient chromosome thus produced may include the first RRS, the first promoter, and the first selection element at the V segment 5' end or upstream of the variable region of the endogenous immunoglobulin locus, and may include the second promoter, the second selection element, and the second RRS at the C segment 3' end or downstream of the constant region of the endogenous immunoglobulin locus. That is, the recipient chromosome includes the first RRS, the first promoter, and the first selection element at the 5' end of the variable region of the endogenous immunoglobulin locus, and includes the second promoter, the second selection element, and the second RRS at the 3' end of the constant region of the endogenous immunoglobulin locus.

The third and fourth vectors, which are vectors for a donor chromosome, may be inserted into an endogenous immunoglobulin locus present in a human cell. In this case, the endogenous immunoglobulin locus present in a human cell may be an endogenous immunoglobulin heavy chain locus, an endogenous immunoglobulin kappa locus, or an endogenous immunoglobulin lambda locus. In one embodiment, the third vector may be inserted into the V segment 5' end or upstream of the variable region of the endogenous immunoglobulin locus. In this case, the fourth vector may be inserted into the J segment 4' end or downstream of the variable region of the endogenous immunoglobulin locus. Therefore, the donor chromosome thus produced includes the promotor, the third selection element, and the third RRS at the V segment 5' end or upstream of the variable region of the endogenous immunoglobulin locus, and may include the fourth RRS, the fourth promoter, and the fourth selection element at the J segment 3' end or downstream. That is, the recipient chromosome includes the third promoter, the third selection element, and the third RRS at the 5' end of the variable region of the endogenous immunoglobulin locus, and includes the fourth RRS, the fourth promoter, and the fourth selection element at the 3' end. In a further embodiment, the third vector may be inserted into the V segment 5' end or upstream of the variable region of the endogenous immunoglobulin locus. In this case, the fourth vector may be inserted into the C segment 3' end or downstream of the constant region of the endogenous immunoglobulin locus. Therefore, the donor chromosome thus produced includes the third promoter, the third selection element, and the third RRS at the V segment 5' end or upstream of the variable region of the endogenous immunoglobulin locus, and may include the fourth RRS, the fourth promoter, and the fourth selection element at the C segment 3' end or downstream of the con of the endogenous immunoglobulin locus. That is, the recipient chromosome includes the third promoter, the third selection element, and the third RRS at the 5' end of the variable region of the endogenous immunoglobulin locus, and includes the fourth RRS, the fourth promoter, and the fourth selection element at the 3' end of the constant region of the endogenous immunoglobulin locus.

In this case, the first RRS inserted into the recipient chromosome and the third RRS inserted into the donor chromosome are RRSs for intrachromosomal exchange and may be paired with each other.

In this case, the second RRS inserted into the recipient chromosome and the fourth RRS inserted into the donor chromosome are RRSs for intrachromosomal exchange and may be paired with each other.

Through this step, recipient cells including a recipient chromosome having the first RRS at the 5' end of the variable region of the endogenous (non-human) immunoglobulin locus and the second RRS at the 3' end can be obtained. Alternatively, recipient cells including a recipient chromosome having the first RRS at the 5 'end of the variable region of the endogenous (non-human) immunoglobulin locus and the second RRS at the 3' end of the constant region of the endogenous (non-human) immunoglobulin locus can be obtained. The recipient cells can be obtained through the expression of the first and second selection elements included in the vectors for a recipient chromosome.

In addition, through this step, donor cells including a donor chromosome having the third RRS at the 5' end of the variable region of the endogenous (human) immunoglobulin locus and the fourth RRS at the 3' end can be obtained. Alternatively, donor cells including a donor chromosome having the third RRS at the 5 'end of the variable region of the endogenous (human) immunoglobulin locus and the fourth RRS at the 3' end of the constant region of the endogenous (human) immunoglobulin locus can be obtained. The donor cells can be obtained through the expression of the third and fourth selection elements included in the vectors for a donor chromosome.

The embodiments described above are merely illustrative, and the embodiments can be modified or altered depending on the configuration and number of vectors for insertion of RRSs.

### iv) Production of a microcell using a human cell containing a donor chromosome with an RRS inserted

This step is a step of producing a microcell for transferring a donor chromosome to a recipient cell for intrachromosomal exchange. More specifically, for intrachromosomal exchange, i.e., exchange between a recipient chromosome and a donor chromosome, the recipient chromosome and the donor chromosome must be present in one cell. To this end, a step of transferring (or moving) the donor chromosome to a recipient cell is required. The step is a step of microcellularizing the donor cell to prepare a microcell containing the donor chromosome. "Microcellularization" means the production of micro cells using one or more cells. In this case, the microcellularization is the production of a plurality of cells or cell-like from one cell.

For the production of microcells from a donor cell may be performed using known techniques. The techniques are described in Thorfinn Ege et. al., 1974; Thorfinn Ege et. al., 1977.

The step involves the following:
a micronucleation process of treating donor cells with an antimicrotubule agent to produce micronucleated cells; and
an enucleation process of centrifuging the micronucleated cells treated with the antimicrotubule agent to produce microcells.

The antimicrotubule agent may be a substance that inhibits the elongation of microtubules. For example, the antimicrotubule agent may be any one or more of colchicine, nocodazole, and colcemid.

The antimicrofiber agent may be a substance that inhibits the elongation of microfibers. For example, the antimicrofiber agent may be cytochalasin B.

The centrifugation may be performed under a percoll gradient.

By the micronucleation, the nuclear membrane of the donor cell can be separated into two or more vesicles.

By the enucleation, the nuclear membrane of the donor cell can be separated into two or more vesicles.

The step may further involve filtration of the microcells.

The filtration may be performed using a membrane filter. In this case, the pore size of the membrane filter may be 3 to 10 um. Alternatively, the pore size of the membrane filter may be 5 to 8 um. Alternatively, the pore size of the membrane filter may be 8 to 15 um. Alternatively, the pore size of the membrane filter may be 12 to 10 um.

Through this step, the donor cell can be made into multiple microcells. In this case, at least one microcell of the plurality of microcells prepared from the donor cell contains a donor chromosome or a fragment of the donor chromosome.

Hereinafter, various embodiments will be described. The embodiments are embodiments using the donor cell described above.

### iv -1) Production of a microcell using a donor cell containing a donor chromosome with an RRS inserted

In one embodiment, the donor cell may be a donor cell containing a donor chromosome with one RRS inserted. The donor cell containing a donor chromosome with one RRS inserted is the same as described in the section "Production of a recipient cell containing a recipient chromosome with one RRS inserted and a donor cell containing a donor chromosome with one RRS inserted (using a first vector set)". Through this step, the donor cell can be made into multiple microcells. In this case, at least one microcell of the plurality of microcells prepared from the donor cell contains a donor chromosome with one RRS inserted or a fragment of the donor chromosome. That is, at least one microcell of the plurality of microcells containing a donor chromosome having one RRS (second RRS) at the J segment 3' end or downstream of the variable region of the endogenous (human) immunoglobulin locus, or a fragment of the donor chromosome.

### iv -2) Production (1) of a microcell using a donor cell containing a donor chromosome with two or mor RRSs inserted

In another embodiment, the donor cell may be a donor cell containing a donor chromosome with two or more RRSs inserted. The donor cell containing a donor chromosome with two or more RRSs inserted is the same as described in the section "Production of a recipient cell containing a recipient chromosome with one RRS inserted and a donor cell containing a donor chromosome with one RRS inserted (using a second vector set)". Through this step, the donor cell can be made into multiple microcells. In this case, at least one microcell of the plurality of microcells prepared from the donor cell contains a donor chromosome with two or more RRSs inserted or a fragment of the donor chromosome. That is, at least one microcell of the plurality of microcells prepared contains a donor chromosome having the fifth RRS at the 5' end of the variable region of the endogenous immunoglobulin locus and the eighth RRS at the 3' end, or a fragment of the donor chromosome. That is, at least one microcell of the plurality of microcells prepared contains a donor chromosome having the eighth RRS at the segment 5' end of the variable region of the endogenous (human) immunoglobulin locus and the fifth RRS at the 3' end, or a fragment of the donor chromosome.

### iv -3) Production (2) of a microcell using a donor cell containing a donor chromosome with two or mor RRSs inserted

In a further embodiment, the donor cell may be a donor cell containing a donor chromosome with two or more RRSs inserted. The donor cell containing a donor chromosome with two or more RRSs inserted is the same as described in the section "Production of a recipient cell containing a recipient chromosome with one RRS inserted and a donor cell containing a donor chromosome with one RRS inserted (using a third vector set)". Through this step, the donor cell can be made into multiple microcells. In this case, at least one microcell of the plurality of microcells prepared from the donor cell contains a donor chromosome with two or more RRSs inserted, or a fragment of the donor chromosome. That is, at least one microcell of the plurality of microcells prepared contains a donor chromosome having the third RRS at the 5' end of the variable region of the endogenous (human) immunoglobulin locus and the fourth RRS at the 3' end or contains a fragment of the donor chromosome. That is, at least one microcell of the plurality of microcells prepared contains a donor chromosome having the third RRS at the 5' end of the variable region of the endogenous (human) immunoglobulin locus and the fourth RRS at the 3' end of the constant region of the endogenous (human) immunoglobulin locus or contains a fragment of the donor chromosome.

The embodiments described above are merely illustrative, and the embodiments can be modified or altered according to known techniques.

### v) Production of a fusion non-human animal cell by fusing at least one microcell with a non-human animal cell containing a recipient chromosome with RRS inserted

This step is a step of fusing a microcell with a recipient cell to transfer a donor chromosome to the recipient cell for intrachromosomal exchange. More specifically, for intrachromosomal exchange, i.e., exchange between a recipient chromosome and a donor chromosome, the recipient chromosome and the donor chromosome must be present in one cell. To this end, the step provides a method of fusing the microcell prepared as described above and a recipient cell. The microcell is a microcell containing a donor chromosome or a fragment of the donor chromosome. The cell produced through this step is a recipient cell containing a donor chromosome (or a fragment of the donor chromosome), and is referred to as a "fusion non-human animal cell", and hereinafter a recipient cell containing a donor chromosome (or a fragment of the donor chromosome), i.e., a non-human animal cell (recipient cell) containing a donor chromosome and a recipient chromosome is referred to as a fusion non-human animal cell.

The step produces fusion non-human animal cells by bringing a recipient cell into contact with a microcell. This step will be performed using known techniques. The techniques are described in [Fournier RE et al 1977; McNeill CA et al 1980]. See, for example, Tomizuka et al., Nature Genetics, 16: 133 (1997).

For example, a fusion non-human animal cell production method includes:
(i) brining a recipient cell into contact with one or more microcells; and
(ii) treating the recipient cells and the one or more microcells with a positively charged surfactant,
thereby achieving cell fusion.

The brining the recipient cell into contact with the one or more microcells may be performed such that the recipient cell and the one or more microcells are located in the same medium or in the same buffer.

The positively charged surfactant may be polyethylene glycol (PEG).

In the method, mitogen treatment may be further performed in the step (ii). In this case, the mitogen may be phytohemagglutinin-P (PHA-P).

The fusion non-human animal cell can be produced by cell fusion of a recipient cell and one or more microcells. In this case, the recipient cell contains a recipient chromosome with an RRS inserted, and the details thereof are as described in the section "ii) Preparation of a non-human animal cell containing a recipient chromosome with RRS inserted" described above. The one or more microcells are made from a donor cell containing a donor chromosome with RRS inserted, in which the one or more micro cells contain a donor chromosome with an RRS inserted or a fragment of the donor chromosome, as described in the section "iv) Production of a microcell containing a donor chromosome with RRS inserted".

The fusion non-human animal cell prepared through the step further includes a microcell-derived donor chromosome (or a fragment thereof) with one or more cells inserted, in addition to the chromosome of a recipient cell containing the recipient chromosome with one or more RRSs inserted. In this case, the fusion non-human animal cell may be a cell in which the number of chromosomes is one or more larger than the total number of chromosomes of the recipient cell. For example, when the total number of chromosomes of the recipient cell is 2n (= 40), the fusion non-human animal cell may be a cell having 2n +1 (= 41) chromosomes. In this case, the fusion non-human animal cell may be a cell having all the chromosomes (2n = 40) of the recipient cell plus one chromosome.

Hereinafter, various embodiments will be described. The embodiments are embodiments using the recipient cell and the microcell described above.

### v-1) Production of a fusion non-human animal cell by fusing at least one microcell with a recipient cell containing a recipient chromosome with one RRS inserted

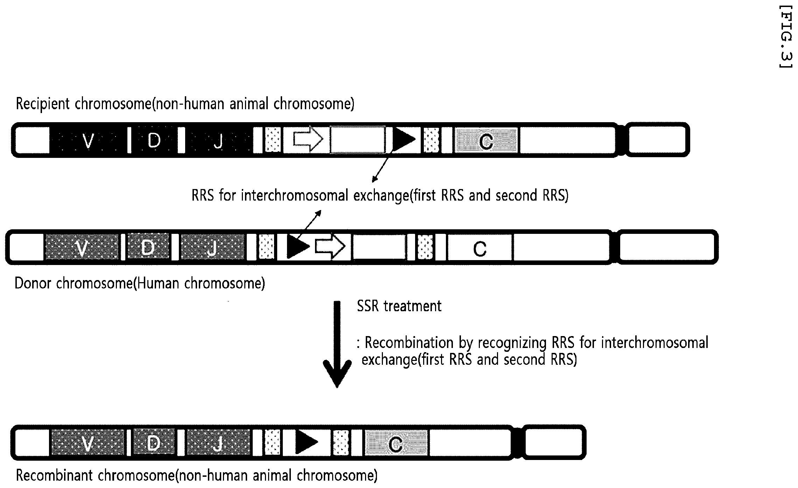

In one embodiment, a fusion non-human animal cell production method may use a recipient cell containing a recipient chromosome with one RRS inserted and at least one microcell. The recipient cell containing a recipient chromosome with one RRS inserted is the same as described in the section "ii-1, iii-1) Production of a donor cell containing a recipient chromosome with one RRS inserted and a donor cell containing a donor chromosome with one RRS inserted (using a first vector set)". The at least one microcell may be one of the microcells prepared from a donor cell containing a donor chromosome with one RRS inserted. The at least one microcell may contain a donor chromosome with one RRS inserted or a fragment of the donor chromosome. The details thereof are as described in the section "iv-1) Production of a microcell, using a donor cell containing a donor chromosome with one RRS inserted". The fusion non-human animal cell prepared through the step contains a recipient chromosome with one RRS inserted and a donor chromosome (or a fragment of the donor chromosome) with one RRS inserted. That is, the prepared fusion non-human animal cell may contain a recipient chromosome having one RRS (first RRS) at the J segment 3' end or downstream of a variable region of a non-human animal immunoglobulin locus and a donor chromosome (or a fragment of the donor chromosome) having one RRS (second RRS) at the J segment 3' end or downstream of a variable region of a human immunoglobulin locus (see FIG. 3).

### v-2) Production (1) of a fusion non-human animal cell by fusing at least one microcell with a recipient cell containing a recipient chromosome with two or more RRS inserted

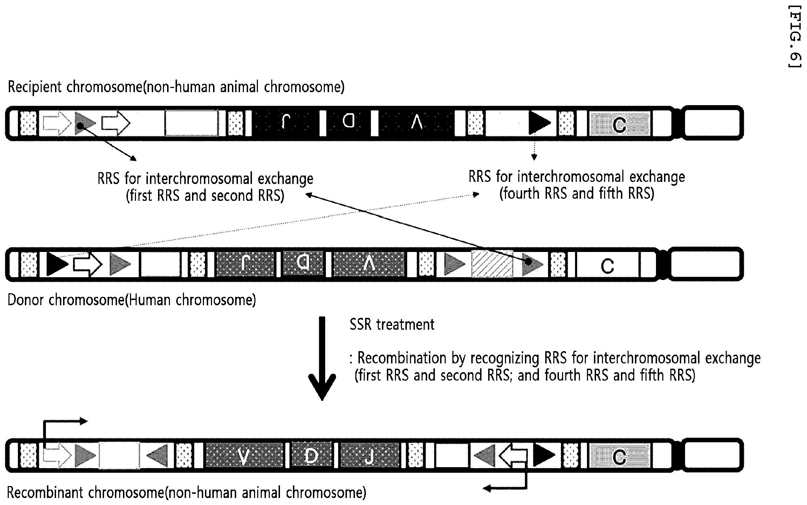

In another embodiment, the fusion non-human animal cell production method may use a recipient cell containing a recipient chromosome with two or more RRSs inserted and at least one microcell. The recipient cell containing a recipient chromosome with two or more RRSs inserted is the same as described in the section "ii-2, iii-2) Production of a recipient cell containing a recipient chromosome with two or more RRSs inserted and a donor cell containing a donor chromosome with two or more RRSs inserted (using a second vector set)". The at least one microcell may be one of the microcells prepared from the donor cell containing a donor chromosome with two or more RRSs inserted. The at least one microcell may contain a donor chromosome with two or more RRSs inserted or a fragment of the donor chromosome. The details thereof are as described in the section "iv-2) Production (1) of a microcell, using a donor cell containing a donor chromosome with two or more RRSs inserted". The fusion non-human animal cell prepared through the step contains a recipient chromosome with two or more RRSs inserted and a donor chromosome (or a fragment of the donor chromosome) with two or more RRSs inserted. That is, the fusion non-human animal cell prepared may contain a recipient chromosome having the first RRS at the 5' end of a variable region of a non-human animal immunoglobulin locus and the fourth RRS at the 3' end and a donor chromosome (or a fragment of the donor chromosome) having the fifth RRS at the 5' end of the variable region of a human immunoglobulin locus and the eighth RRS at the 3' end. Alternatively, the prepared fusion non-human animal cell may contain a recipient chromosome having the fourth RRS at the 5' end of the variable region of the non-human animal immunoglobulin locus and the first RRS at the 3' end and a donor chromosome (or a fragment of the donor chromosome) having the eighth RRS at the 5' end of the variable region of the human immunoglobulin locus and the fifth RRS at the 3' end (see FIG. 6).

### v-3) Production (2) of a fusion non-human animal cell by fusing at least one microcell with a recipient cell containing a recipient chromosome with two or more RRS inserted

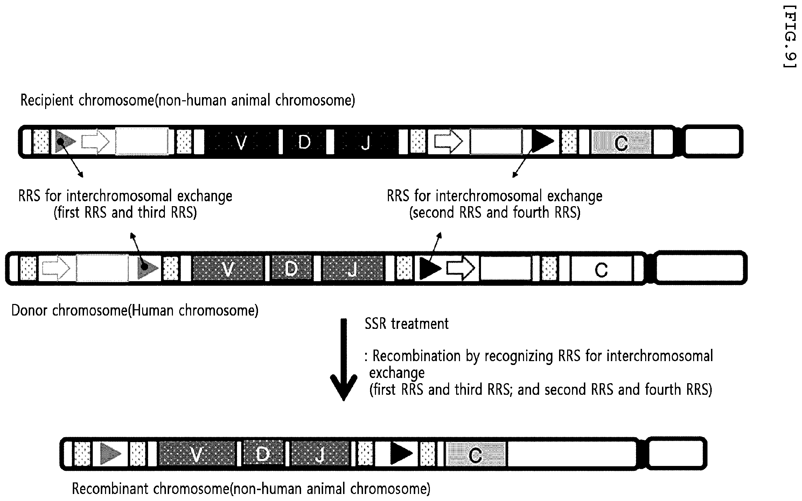

In a further embodiment, the fusion non-human animal cell production method may use a recipient cell containing a recipient chromosome with two or more RRSs inserted and at least one microcell. The recipient cell containing a recipient chromosome with two or more RRSs inserted is the same as described in the section "ii-3, iii-3) Production of a recipient cell containing a recipient chromosome with two or more RRSs inserted and a donor cell containing a donor chromosome with two or more RRSs inserted (using a third vector set)". The at least one microcell may be one of the microcells prepared from the donor cell containing a donor chromosome with two or more RRSs inserted. The at least one microcell may contain a donor chromosome with two or more RRSs inserted or a fragment of the donor chromosome. The details thereof are as described in the section "iv-3) Production (2) of a microcell, using a donor cell containing a donor chromosome with two or more RRSs inserted". The fusion non-human animal cell prepared through the step contains a recipient chromosome with two or more RRSs inserted and a donor chromosome (or a fragment of the donor chromosome) with two or more RRSs inserted. That is, the prepared fusion non-human animal cell may contain a recipient chromosome having the first RRS at the 5' end of the variable region of the non-human animal immunoglobulin locus and the second RRS at the 3' end and a donor chromosome (or a fragment of the donor chromosome) having the third RRS at the 5' end of the variable region of the human immunoglobulin locus and the fourth RRS at the 3' end (see FIG. 9). Alternatively, the fusion non-human animal cell prepared may contain a recipient chromosome having the first RRS at the 5' end of the variable region of the non-human animal immunoglobulin locus and the second RRS at the 3' end of the constant region of the non-human animal immunoglobulin locus and a donor chromosome (or a fragment of the donor chromosome) having the third RRS at the 5' end of the variable region of the human immunoglobulin locus and the fourth RRS at the 3' end of the constant region of the human immunoglobulin locus.

The embodiments described above are merely illustrative, and the embodiments can be modified or altered according to known techniques.

### vi) Production of a recombinant non-human animal cell containing a recombinant chromosome by treating a fusion non-human animal cell with a recombinase

This step is a step of treating a fusion non-human animal cell containing a donor chromosome and a recipient with a recombinase for intrachromosomal exchange, thereby producing a recombinant chromosome. More specifically, for the intrachromosomal exchange between chromosomes (i.e., a recipient chromosome and a donor chromosome), a fusion non-human animal cell containing the recipient chromosome and the donor chromosome is treated with a recombinase, thereby inducing recombination between two chromosomes. To this end, the step provides a method of providing a recombinase to the fusion non-human animal cell prepared as described above. The recombinase is a recombinase for recognizing the RRSs for intrachromosomal exchange, which are included in the recipient chromosome and donor chromosome contained in the fusion non-human animal cell. The recombinase provided to the fusion non-human animal cells may vary depending on the RRS for interchromosomal exchange. When the fusion non-human animal cell is provided with a recombinase in the step, the provided recombinase recognizes the RRSs for interchromosomal exchange, which are included in the recipient chromosome and donor chromosome and induces recombination (exchange or replacement) between the two chromosomes. This results in the production of a recombinant chromosome. The recombinant chromosome is a chromosome in which the variable region of the non-human animal immunoglobulin locus of the recipient chromosome has been replaced (exchanged) with the variable region of the human immunoglobulin locus. That is, the resulting recombinant chromosome is a non-human animal chromosome containing a humanized immunoglobulin locus. The cell generated through the step is a non-human animal cell containing a recombinant chromosome (non-human animal chromosome having a humanized immunoglobulin locus) produced through the recombination of the recipient chromosome and the donor chromosome, and the cell is referred to as a "recombinant non-human animal cell". Hereinafter, a non-human animal cell containing the recombinant chromosome is described as a recombinant non-human animal cell. The non-human animal chromosome having a humanized immunoglobulin locus is as described in the section "Non-human animal chromosome containing a humanized immunoglobulin locus".

This step treats the fusion non-human animal cell with a recombinase to produce the recombinant non-human animal cell.

The recombinant non-human animal cell production method may include a step of treating the fusion non-human animal cell with a site-specific recombinase (SSR).

The fusion non-human animal cell may contain a recipient chromosome having one or more RRSs and a donor chromosome having one or more RRSs. In this case, the one or more RRSs included in the recipient chromosome and the one or more RRSs included in the donor chromosome are RRSs for intrachromosomal exchange.

The SSR may be an SSR that recognizes the RRS for interchromosomal exchange, and the SSR may vary depending on the type of RRS for interchromosomal exchange. For example, when the RRS for interchromosomal exchange is loxp or a variant thereof, the SSR may be a recombinase. Alternatively, when the RRS for interchromosomal exchange is FRT or a variant thereof, the SSR may be a flippase (FLP). Alternatively, when the RRS for interchromosomal exchange is attP/attB or a variant thereof, the SSR may be an integrase. Alternatively, when the RRS for interchromosomal exchange is an ITR or a variant thereof, the SSR may be a transposase.

The SSR may be a Cre recombinase, a flippase (FLP), an integrase, or a transposase.

Here, the amino acid sequence of the Cre recombinase amino acid sequence is disclosed in Table 3 below. However, the Cre recombinase is by way of example and not by way of limitation.

**[Table 3] Amino Acid Sequence of Cre Recombinase**

| Amino acid sequence of Cre recombinase |
|---|
| SNLLTVHQNLPALPVDATSDEVRKNLMDMFRDRQAFSEHTWKMLLSVCRSWAAWCKLNNRKWF PAEPEDVRDYLLYLQARGLAVKTIQQHLGQLNMLHRRSGLPRPSDSNAVSLVMRRIRKENVDA GERAKQALAFERTDFDQVRSLMENSDRCQDIRNLAFLGIAYNTLLRIAEIARIRVKDISRTDG GRMLIHIGRTKTLVSTAGVEKALSLGVTKLVERWISVSGVADDPNNYLFCRVRKNGVAAPSAT SQLSTRALEGIFEATHRLIYGAKDDSGQRYLAWSGHSARVGAARDMARAGVSIPEIMQAGGWT NVNIVMNYIRNLDSETGAMVRLLEDGD(SEQ ID NO: 10) |

The transposase may be a PiggyBac transposase (PB transposase) or a Sleeping Beauty transposase (SB transposase). Here, the amino acid sequence of the PB transposase is disclosed in Table 4 below. However, the PB transposase disclosed in Table 4 below is, by way of example and not by way of limitation.

**[Table 4] Amino Acid Sequence of PiggyBac Transposase**

| Amino acid sequence of PiggyBac transposase |
|---|
| MGSSLDDEHILSALLQSDDELVGEDSDSEISDHVSEDDVQSDTEEAFIDEVHEVQPTSSGSEI LDEQNVIEQPGSSLASNRILTLPQRTIRGKNKHCWSTSKSTRRSRVSALNIVRSQRGPTRMCR NIYDPLLCFKLFFTDEIISEIVKWTNAEISLKRRESMTGATFRDTNEDEIYAFFGILVMTAVR KDNHMSTDDLFDRSLSMVYVSVMSRDRFDFLIRCLRMDDKSIRPTLRENDVFTPVRKIWDLFI HQCIQNYTPGAHLTIDEQLLGFRGRCPFRMYIPNKPSKYGIKILMMCDSGTKYMINGMPYLGR GTQTNGVPLGEYYVKELSKPVHGSCRNITCDNWFTSIPLAKNLLQEPYKLTIVGTVRSNKREI PEVLKNSRSRPVGTSMFCFDGPLTLVSYKPKPAKMVYLLSSCDEDASINESTGKPQMVMYYNQ TKGGVDTLDQMCSVMTCSRKTNRWPMALLYGMINIACINSFIIYSHNVSSKGEKVQSRKKFMR NLYMSLTSSFMRKRLEAPTLKRYLRDNISNILPNEVPGTSDDSTEEPVMKKRTYCTYCPSKIR RKANASCKKCKKVICREHNIDMCQSCF(SEQ ID NO: 11) |

The SSR treatment may involve introducing or delivering an SSR in a protein form. In this case, the introduction or delivery in a protein form may be carried out by electroporation, microinjection, transient cellular compression or squeezing (for example, as disclosed in Lee, et al, (2012) Nano Lett., 12, 6322-6327), lipid-mediated transfection, nanoparticle-, liposome-, or peptide-mediated delivery, or any combination thereof.

The SSR treatment may involve introducing or delivering a vector containing a nucleic acid sequence encoding the SSR. The vector may be a viral vector or a recombinant viral vector. The virus may be, but is not limited to, a retrovirus, a lentivirus, an adenovirus, an adeno-associated virus (AAV), a vaccinia virus, a poxvirus, or a herpes simplex virus. In this case, the introduction or delivery of the vector is performed such that the vector is provided to a non-target protocell using a known transfection method. For example, the transfection method may be a viral transfection method, a reagent transfection method, or a physical transfection method. The viral transfection method may be performed using, for example, a lentivirus. The reagent transfection method may be performed using, for example, calcium phosphate, cation lipid, DEAE-dextran, and polyethylenimine (PEI). The physical transfection method may be performed, for example, using electroporation. In addition, the transfection may be performed using a liposome, but is not limited thereto.

Hereinafter, various embodiments will be described. The embodiments are embodiments using the fusion non-human animal cell described above.

### vi-1) Production of a recombinant non-human animal cell containing a recombinant chromosome by treating a donor chromosome with one RRS inserted and a recipient chromosome with one RRS inserted, with a recombinase

In one embodiment, the recombinant non-human animal cell production method involves treating a fusion non-human animal cell containing a recipient chromosome with one RRS inserted and a donor chromosome with one RRS inserted, with SSR. The fusion non-human animal cell is as described in the section "v-1) Production of a fusion non-human animal cell by fusing at least one microcell with a recipient cell containing a recipient chromosome with one RRS inserted". The fusion non-human animal cell may contain a recipient chromosome having one RRS (first RRS) at the J segment 3' end or downstream of a variable region of a non-human animal immunoglobulin locus and a donor chromosome (or a fragment of the donor chromosome) having one RRS (second RRS) at the J segment 3' end or downstream of a variable region of a human immunoglobulin locus. In this case, the first RRS and the second RRS are RRSs for intrachromosomal exchange. The SSR may be an SSR that recognizes the pairing of the RRSs (first RRS and second RRS) for interchromosomal exchange, and the details thereof are as described in the section "Recombinase recognition site (RRS)" above. When the SSR is provided to the fusion non-human animal cells in the step, the provided SSR recognizes the pairing of RRSs (first RRS and second RRS) for intrachromosomal exchange, which are present in the recipient and donor chromosomes and induces a recombination (exchange or replacement) between two chromosomes. A recombinant chromosome is thus produced. The produced recombinant chromosome has a form in which the variable region of a non-human animal immunoglobulin locus of a recipient chromosome is exchanged (replaced) with the variable region of a human immunoglobulin locus. The produced recombinant chromosome may be a non-human animal chromosome containing the variable region of the recipient chromosome of the human immunoglobulin locus and the variable region of the non-human animal immunoglobulin locus. That is, the produced recombinant chromosome is a non-human animal chromosome having a humanized immunoglobulin locus. The recombinant non-human animal cell generated through the step may include a recombinant chromosome (non-human animal chromosome having a humanized immunoglobulin locus) generated through recombination between the recipient chromosome and the donor chromosome (see FIG. 3).

For example, the non-human animal chromosome having a humanized immunoglobulin locus may be a non-human animal chromosome including a variable region of a human immunoglobulin heavy chain gene and a constant region of a non-human animal immunoglobulin heavy chain gene. Alternatively, the non-human animal chromosome having a humanized immunoglobulin locus may be a non-human animal chromosome including a variable region of a human immunoglobulin kappa gene and a constant region of a non-human animal immunoglobulin kappa gene. Alternatively, the non-human animal chromosome having a humanized immunoglobulin locus may be a non-human animal chromosome having a variable region of a human immunoglobulin lambda gene and a constant region of a non-human animal immunoglobulin lambda gene. Alternatively, the non-human animal chromosome having a humanized immunoglobulin locus may be a non-human animal chromosome having a variable region of a human immunoglobulin lambda gene and a constant region of a non-human animal immunoglobulin kappa gene. Alternatively, the non-human animal chromosome having a humanized immunoglobulin locus may be a non-human animal chromosome having a variable region of a human immunoglobulin kappa gene and a constant region of a non-human animal immunoglobulin lambda gene. The examples are provided only for illustrative purposes, and various combinations between immunoglobulin genes (heavy chain, kappa, lambda) are possible, and thus various non-human animal chromosomes having a humanized immunoglobulin locus can be manufactured.

### vi-2) Production of a recombinant non-human animal cell containing a recombinant chromosome by treating a non-human animal cell containing a donor chromosome with two or more RRSs inserted and a recipient chromosome with two or more RRSs inserted, with a recombinase (1)

In another embodiment, the recombinant non-human animal cell production method involves treating a fusion non-human animal cell containing a recipient chromosome with two or more RRSs inserted and a donor chromosome with two or more RRSs inserted, with SSR. The fusion non-human animal cell is as described in the section "v-2) Production of a fusion non-human animal cell by fusing at least one microcell with a recipient cell containing a recipient chromosome with two or more RRSs inserted". The fusion non-human animal cell prepared may contain a recipient chromosome having the first RRS at the 5' end of a variable region of a non-human animal immunoglobulin locus and the fourth RRS at the 3' end and a donor chromosome (or a fragment of the donor chromosome) having the fifth RRS at the 5' end of a variable region of a human immunoglobulin locus and the eighth RRS at the 3' end. Alternatively, the prepared fusion non-human animal cell may contain a recipient chromosome having the fourth RRS at the 5' end of the variable region of the non-human animal immunoglobulin locus and the first RRS at the 3' end and a donor chromosome (or a fragment of the donor chromosome) having the eighth RRS at the 5' end of the variable region of the human immunoglobulin locus and the fifth RRS at the 3' end. The first RRS and the eighth RRS are RRSs for a first interchromosomal exchange, and the second RRS and the fifth RRS are RRSs for a second interchromosomal exchange. The SSRs may be a first SSR that recognizes the pairing of the RRSs (the first RRS and the eighth RRS) for the first interchromosomal exchange and a second SSR that recognizes the pairing of the RRSs (the fourth RRS and the fifth RRS) for the second interchromosomal exchange, in which the first SSR may be the same as or may be different from the second SSR. The details thereof are as described in the section "Recombinase recognition site (RRS)" above. When the SSRs are provided to the fusion non-human animal cell in the step, the provided SSRs recognize the pairing of the RRSs (the first RRS and the second RRS) for the first intrachromosomal exchange, which are present in the recipient chromosome) and the pairing of the RRSs (the fourth RRS and the fifth RRS) for the second intrachromosomal exchange, which are present in the donor chromosome, and induce a recombination (exchange or replacement) between two chromosomes. Through this process, recombinant chromosomes are produced. The produced recombinant chromosome that is formed by exchange (replacement) of a variable region of a non-human animal immunoglobulin locus, located between the first RRS and fourth RRS of the recipient chromosome, with a variable region of a human immunoglobulin locus, located between the fifth RRS and eighth RRS of the donor chromosome. The recombinant chromosome is a non-human animal chromosome having a variable region of a human immunoglobulin locus and a constant region of a non-human animal immunoglobulin locus. That is, the produced recombinant chromosome may a non-human animal chromosome having a humanized immunoglobulin locus. The recombinant non-human animal cell produced through the step may contain a recombinant chromosome (non-human animal chromosome having a humanized immunoglobulin locus) produced through recombination between the recipient chromosome and the donor chromosome. The recombinant non-human animal cell can be obtained as the first promoter and the third selection element are operably linked to each other by the recombination, and thus the third selection element is expressed (FIG. 6).

For example, the non-human animal chromosome having a humanized immunoglobulin locus may be a non-human animal chromosome including a variable region of a human immunoglobulin heavy chain gene and a constant region of a non-human animal immunoglobulin heavy chain gene. Alternatively, the non-human animal chromosome having a humanized immunoglobulin locus may be a non-human animal chromosome including a variable region of a human immunoglobulin kappa gene and a constant region of a non-human animal immunoglobulin kappa gene. Alternatively, the non-human animal chromosome having a humanized immunoglobulin locus may be a non-human animal chromosome having a variable region of a human immunoglobulin lambda gene and a constant region of a non-human animal immunoglobulin lambda gene. Alternatively, the non-human animal chromosome having a humanized immunoglobulin locus may be a non-human animal chromosome having a variable region of a human immunoglobulin lambda gene and a constant region of a non-human animal immunoglobulin kappa gene. Alternatively, the non-human animal chromosome having a humanized immunoglobulin locus may be a non-human animal chromosome having a variable region of a human immunoglobulin kappa gene and a constant region of a non-human animal immunoglobulin lambda gene. The examples are provided only for illustrative purposes, and various combinations between immunoglobulin genes (heavy chain, kappa, lambda) are possible, and thus various non-human animal chromosomes having a humanized immunoglobulin locus can be manufactured.

### vi-3) (2) Production of a recombinant non-human animal cell containing a recombinant chromosome by treating a non-human animal cell containing a donor chromosome with two or more RRSs inserted and a recipient chromosome with two or more RRSs inserted, with a recombinase (2)

In another embodiment, the recombinant non-human animal cell production method involves treating a fusion non-human animal cell containing a recipient chromosome with two or more RRSs inserted and a donor chromosome with two or more RRSs inserted, with SSRs. The fusion non-human animal cell is as described in the section "v-3) Production (2) of a fusion non-human animal cell by fusing at least one microcell and a recipient cell containing a recipient chromosome with two or more RRSs inserted". The prepared fusion non-human animal cell may contain a recipient chromosome having the first RRS at the 5' end of a variable region of a non-human animal immunoglobulin locus and the second RRS at the 3' end and a donor chromosome (or a fragment of the donor chromosome) having the third RRS at the 5' end of a variable region of a human immunoglobulin locus and the seventh RRS at the 3' end. Alternatively, the prepared fusion non-human animal cell may contain a recipient chromosome having the first RRS at the 5' end of a variable region of a non-human animal immunoglobulin locus and the second RRS at the 3' end of a constant region of the non-human animal immunoglobulin locus and a donor chromosome (or a fragment of the donor chromosome) having the third RRS at the 5' end of a variable region of a human immunoglobulin locus and the fourth RRS at the 3' end of a constant region of the human immunoglobulin locus. The first RRS and the third RRS may be RRSs for a first interchromosomal exchange, and the second RRS and the fourth RRS are RRSs for a second interchromosomal exchange. The SSRs may be a first SSR that recognizes the pairing of the RRSs (the first RRS and the third RRS) for the first interchromosomal exchange and a second SSR that recognizes the pairing of the RRSs (the second RRS and the fourth RRS) for the second interchromosomal exchange, in which the first SSR may be the same as or may be different from the second SSR. The details thereof are as described in the section "Recombinase recognition site (RRS)" above. When the SSRs are provided to the fusion non-human animal cell in the step above, the provided SSRs recognize the pairing of the RRSs (the first RRS and the third RRS) for the first intrachromosomal exchange, which are present in the recipient chromosome) and the pairing of the RRSs (the second RRS and the fourth RRS) for the second intrachromosomal exchange, which are present in the donor chromosome, and the SSRs induce recombination (exchange or replacement) between two chromosomes. Through this step, recombinant chromosomes can be produced. In one embodiment, the produced recombinant chromosome that is formed by exchanging (replacing) a variable region of a non-human animal immunoglobulin locus, located between the first RRS and second RRS of the recipient chromosome, with a variable region of a human immunoglobulin locus, located between the third RRS and fourth RRS of the donor chromosome. The recombinant chromosome is a non-human animal chromosome having a variable region of a human immunoglobulin locus and a constant region of a non-human animal immunoglobulin locus. In another embodiment, the produced recombinant chromosome that is formed by exchanging (replacing) variable and constant regions of a non-human animal immunoglobulin locus, located between the first RRS and second RRS of the recipient chromosome, with variable and constant regions of a human immunoglobulin locus, located between the third RRS and fourth RRS of the donor chromosome. The recombinant chromosome is a non-human animal chromosome having the variable and constant regions of the human immunoglobulin locus. That is, the produced recombinant chromosome is a non-human animal chromosome having a humanized immunoglobulin locus. The recombinant non-human animal cell produced through the step may contain a recombinant chromosome (non-human animal chromosome having a humanized immunoglobulin locus) produced through recombination between the recipient chromosome and the donor chromosome. In this case, the recombinant non-human animal cell can be obtained through a negative selection caused by bonding of the first selection element and the second selection element in the recombinant chromosome (see FIG. 9).

For example, the non-human animal chromosome having a humanized immunoglobulin locus may be a non-human animal chromosome including a variable region of a human immunoglobulin heavy chain gene and a constant region of a non-human animal immunoglobulin heavy chain gene. Alternatively, the non-human animal chromosome having a humanized immunoglobulin locus may be a non-human animal chromosome including a variable region of a human immunoglobulin kappa gene and a constant region of a non-human animal immunoglobulin kappa gene. Alternatively, the non-human animal chromosome having a humanized immunoglobulin locus may be a non-human animal chromosome having a variable region of a human immunoglobulin lambda gene and a constant region of a non-human animal immunoglobulin lambda gene. Alternatively, the non-human animal chromosome having a humanized immunoglobulin locus may be a non-human animal chromosome having a variable region of a human immunoglobulin lambda gene and a constant region of a non-human animal immunoglobulin kappa gene. Alternatively, the non-human animal chromosome having a humanized immunoglobulin locus may be a non-human animal chromosome having a variable region of a human immunoglobulin kappa gene and a constant region of a non-human animal immunoglobulin lambda gene. The examples are provided only for illustrative purposes, and various combinations between immunoglobulin genes (heavy chain, kappa, lambda) are possible, and thus various non-human animal chromosomes having a humanized immunoglobulin locus can be manufactured.

For example, the non-human animal chromosome having a humanized immunoglobulin locus may be a non-human animal chromosome including a variable region and a constant region of a human immunoglobulin kappa gene. Alternatively, the non-human animal chromosome having a humanized immunoglobulin locus may be a non-human animal chromosome including a variable region and a constant region of a human immunoglobulin lambda gene. The examples are provided only for illustrative purposes, and various non-human animal chromosomes having a humanized immunoglobulin locus can be prepared.

The embodiments described above are provided only for illustrative purposes, and the embodiments can be modified or altered according to known techniques.

### An aspect disclosed herein relates to a method for producing a transgenic non-human animal having a genome having a humanized immunoglobulin locus.

More specifically, the production method includes a method for producing a recombinant cell containing a recombinant chromosome having a humanized immunoglobulin locus, using cell fusion and interchromosomal exchange. The production method is a method of producing a transgenic non-human animal, using the transgenic cell described above. The transgenic non-human animal prepared by the production method is a non-human animal capable of producing humanized antibodies or human antibodies.

The production method includes the following:
i) preparing a vector for insertion of RRS;
ii) preparing a non-human animal cell having a recipient chromosome including an immunoglobulin locus with an RRS inserted;
iii) preparing a human cell having a donor chromosome including an immunoglobulin locus with an RRS inserted;
iv) producing a microcell, using the human cell;
v) producing a fusion non-human animal cell by fusing at least one microcell produced in the iv) and the non-human animal cell;
vi) producing a recombinant non-human animal cell containing a recombinant chromosome having a humanized immunoglobulin locus by treating the fusion non-human animal cell with a recombinase; and
vii) producing an offspring using the recombinant non-human animal cell.

In this case, the step i) may be omitted when using a known material (for example, a vector having a known configuration, a vector available on the market, etc.).

In this case, the steps ii) and iii) may be performed sequentially, in reverse order, or simultaneously.

The steps i) through vi) are as described above. Hereinafter, the step vii) is described below.

### vii) Production of an offspring using a recombinant non-human animal cell

The step is a step of producing a transgenic non-human animal using a recombinant non-human animal cell containing a recombinant chromosome. In this case, the presence or absence of a problem with the development of recombinant chromosomes into animals can be determined by Example 1 in which a normal non-human animal is produced using a recombinant non-human animal cell containing a recombinant chromosome produced through recombination between two chromosomes. In Example 1, a mouse containing the TK gene of a mouse genome was normally generated. Through this, it was confirmed that the recombinant chromosome generated through recombination between two chromosomes did not affect developmentally, and an offspring can be generated normally by using the recombinant non-human animal cell containing the recombinant chromosome.

More specifically, the step is a step of producing a transgenic non-human animal using a recombinant non-human animal cell containing the recombinant chromosome having a humanized immunoglobulin locus, which is produced as described above. To this end, this step will be performed using known techniques. For example, known techniques may be, but are not limited to, somatic cell nuclear transfer (SCNT), generation from an embryo, and blastocyst injection.

### vii-1) Somatic cell nuclear transfer (SCNT)

In one embodiment, the transgenic non-human animal can be produced via somatic cell nuclear transfer (SCNT) using recombinant non-human animal cells containing a recombinant chromosome having a humanized immunoglobulin locus. In this case, the recombinant non-human animal cell may be the recombinant non-human animal cell described in the section "v-1) Production of a non-human animal cell containing a recombinant chromosome by treating a non-human animal cell containing a donor chromosome with one RRS inserted and a recipient cell with one RRS inserted, with a recombinase", the section "v-2) Production (1) of a recombinant non-human animal cell containing a recombinant chromosome by treating a non-human animal cell containing a donor chromosome with two or more RRSs inserted and a recipient chromosome with one RRS inserted, with a recombinase", or the section "v-3) Production (2) of a recombinant non-human animal cell containing a recombinant chromosome by treating a non-human animal cell containing a donor chromosome with two or more RRSs inserted and a recipient chromosome with one RRS inserted, with a recombinase". The somatic cell nuclear transfer is a method of obtaining a donor nucleus from the recombinant non-human animal cell, implanting the donor nucleus into a denuclearized egg to produce a replicate egg, and implanting the replicate egg into the womb of a surrogate mother to produce an offspring. The SCNT may be a known technique.

### vii-2) Generation from embryo

In another embodiment, the transgenic non-human animal can be produced using a recombinant non-human animal cell containing a recombinant chromosome having a humanized immunoglobulin locus. In this case, the recombinant non-human animal cell may be the recombinant non-human animal cell described in the section "v-1) Production of a non-human animal cell containing a recombinant chromosome by treating a non-human animal cell containing a donor chromosome with one RRS inserted and a recipient cell with one RRS inserted, with a recombinase", the section "v-2) Production (1) of a recombinant non-human animal cell containing a recombinant chromosome by treating a non-human animal cell containing a donor chromosome with two or more RRSs inserted and a recipient chromosome with one RRS inserted, with a recombinase", or the section "v-3) Production (2) of a recombinant non-human animal cell containing a recombinant chromosome by treating a non-human animal cell containing a donor chromosome with two or more RRSs inserted and a recipient chromosome with one RRS inserted, with a recombinase". The recombinant non-human animal cell may be a recombinant non-human animal embryo. The transgenic non-human animal is one obtained from the embryo by implanting the recombinant non-human animal embryo into the womb of a surrogate mother, and the animal can be obtained using a known method.

### vii-3) Blastocyst injection

In a further embodiment, the transgenic non-human animal can be produced by blastocyst injection, using a recombinant non-human animal cell containing a recombinant chromosome having a humanized immunoglobulin locus. In this case, the recombinant non-human animal cell may be the recombinant non-human animal cell described in the section "v-1) Production of a non-human animal cell containing a recombinant chromosome by treating a non-human animal cell containing a donor chromosome with one RRS inserted and a recipient cell with one RRS inserted, with a recombinase", the section "v-2) Production (1) of a recombinant non-human animal cell containing a recombinant chromosome by treating a non-human animal cell containing a donor chromosome with two or more RRSs inserted and a recipient chromosome with one RRS inserted, with a recombinase", or the section "v-3) Production (2) of a recombinant non-human animal cell containing a recombinant chromosome by treating a non-human animal cell containing a donor chromosome with two or more RRSs inserted and a recipient chromosome with one RRS inserted, with a recombinase". The recombinant non-human animal cell may be a recombinant non-human animal embryonic stem (ES) cell. The transgenic non-human animal is obtained by implanting the recombinant non-human animal embryonic stem cell into the blastula to produce a chimeric blastocyst and then implanting the chimeric blastocyst into the womb of a surrogate mother to obtain an offspring. That is, the transgenic non-human animal is obtained using a known method.

The embodiments described above are provided only for illustrative purposes, and the embodiments can be modified or altered according to known techniques.

### An aspect disclosed herein relates to a transgenic cell having a genome having a humanized immunoglobulin locus.

More specifically, the embodiment relates to a recombinant non-human animal cell produced by the method of producing a transgenic cell having a genome having a humanized immunoglobulin locus. The transgenic cell having a genome having a humanized immunoglobulin locus is a recombinant non-human animal cell containing a recombinant chromosome having a humanized immunoglobulin locus. The recombinant non-human animal cell and the production method thereof are the same as described in the section "Method of producing a transgenic cell having a genome having a humanized immunoglobulin gene locus".

### Another aspect disclosed herein relates to a transgenic non-human animal cell having a genome having a humanized immunoglobulin locus.

More specifically, the embodiment relates to a transgenic non-human animal produced by the method of producing a transgenic non-human animal having a genome having a humanized immunoglobulin locus. The transgenic non-human animal cell having a genome having a humanized immunoglobulin locus is a recombinant non-human animal produced with the use of a recombinant non-human animal cell containing a recombinant chromosome having a humanized immunoglobulin locus. The transgenic non-human animal and the production method thereof are the same as described in the section "Method of producing a transgenic non-human animal having a genome having a humanized immunoglobulin gene locus". The transgenic non-human animal may vary depending on the non-human animal cell used for the recipient cell. For example, the non-human animal may be a rodent such as a mouse, a rat, etc.; an ungulate such as a rabbit, a goat, a cow, a pig, a camel, etc.; a primate other than humans, such as a chimpanzee and a monkey; or a bird such as a chicken, quail, etc.

### A further aspect disclosed herein relates to an antibody production method using a transgenic non-human animal having a genome having a humanized immunoglobulin locus.

More specifically, the aspect relates to an antibody production method using a transgenic non-human animal produced by the method of producing a transgenic non-human animal having a genome having a humanized immunoglobulin locus. The transgenic non-human animal having a genome having a humanized immunoglobulin locus is a transgenic non-human animal produced with the use of a recombinant non-human animal cell containing a recombinant chromosome having a humanized immunoglobulin locus. The transgenic non-human animal and the production method thereof are the same as described in the section "Method of producing a transgenic non-human animal having a genome having a humanized immunoglobulin gene locus". The antibody production method may include a step of injecting an antigen into the transgenic non-human animal. Through the antigen injection, the transgenic non-human animal can produce an antibody specific for the antigen. In this case, the antigen-specific antibody can be produced by recombining (rearranging) the variable regions of the humanized immunoglobulin loci.

A transgenic mouse used to produce antibodies, which is disclosed in the related art (US 8,502,018; US 9,371,553; US 9,528,136; US 9,376,699; US 9,447,177; US 9,505,827 and US 9,434,782) has a genome having a humanized immunoglobulin locus and has shown that it is possible to produce antibodies using such a transgenic mouse. The transgenic mouse has the same genetic configuration as a transgenic non-human animal having a genome having a humanized immunoglobulin locus as disclosed herein. Thus, since the related art has shown that it is possible to produce antibodies using such a transgenic mouse, it is apparent to those skilled in the art that an antibody can be produced using a transgenic non-human animal having a genome having a humanized immunoglobulin locus, the transgenic non-human animal having the same genetic configuration as disclosed herein.

Hereinafter, the present invention will be described in more detail with reference to examples.

These examples are presented only for illustrative purposes, and it will be apparent to those ordinarily skilled in the art that the scope of the present invention is not to be construed as being limited by these examples.

### Example 1. Production of a recombinant chromosome using cell fusion and interchromosomal exchange and production of a transgenic non-human animal using the recombinant chromosome (FIGS. 10 to 24)

This example is a specific experimental example for demonstrating a transgenic introduction method using cell fusion and interchromosomal exchange. The example relates to a cell containing a recombinant chromosome on a specific position, which is produced by recombination of chromosomes and to a transgenic mouse production method. The following description relates to a cell having an antibiotic resistant gene and two RRSs inserted at the end of a variable region of an immunoglobulin heavy chain locus and to an example of a transgenic mouse production method using the cell. The method is an exemplary method using a recombinant chromosome but is not limited the example. The intended recombinant chromosome can be produced by diversely modifying the examples described below, or can be produced by adding various methods other than the examples described below.

### 1. Designing of vector

Vectors were designed for insertion of loxp and an antibiotic resistant gene into the end of each variable region, to exchange (or replace) the antibiotic resistant gene located at the end of the variable region of a mouse immunoglobulin heavy chain locus with an antibiotic resistant gene located at the end of the variable region of a human immunoglobulin heavy chain locus. The antibiotic resistant gene inserted into the end of the variable region of the mouse immunoglobulin heavy chain locus and the antibiotic resistant gene inserted into the end of the variable region of the human immunoglobulin heavy chain locus are different antibiotic resistant genes from each other. In addition, the loxp that is inserted along with the antibiotic resistant gene is used for recombination between two chromosomes (a mouse chromosome having a mouse immunoglobulin heavy chain locus with the antibiotic resistant gene inserted and a human chromosome having a human immunoglobulin heavy chain locus with the antibiotic resistant gene inserted).

A first vector was designed to insert the antibiotic resistant gene and loxp into the end of the variable region of the mouse immunoglobulin heavy chain locus, and a second vector was designed to insert the antibiotic resistant gene and loxp into the end of the variable region of the human immunoglobulin heavy chain locus.

The first vector consists of a first homologous arm (M002) used for insertion into the end of the variable region of the mouse immunoglobulin heavy chain locus, a CMV promoter, Loxm2/71, a hygromycin resistant gene (HygroR), lox66, a second homologous arm (M001) used for insertion into the end of the variable region of the mouse immunoglobulin heavy chain locus, a TK gene used for negative selection, AmpR to be used for bacterial positive selection, and a replication origin.

The second vector consists of a first homologous arm (H002) used for insertion into the end of the variable region of the human immunoglobulin heavy chain locus, lox71, inverted puroΔTK, loxm2/66, NeoR, a second homologous arm (H001) used for insertion into the end of the variable region of the human immunoglobulin heavy chain locus, a TK gene used for negative selection, AmpR to be used for bacterial positive selection, and a replication origin.

**[Table 5] Primers and their DNA sequences used for vector preparation**

| SEQ ID NO: | Primer | DNA sequence (5'→3') |
|---|---|---|
| 12 | Neo^{R}-EcoRI-forward | gaattcGGCTGTGGAATGTGTGTCAGTTAGGGTG |
| 13 | Neo^{R}-loxp-bridge | CTTATCATGTCTGTATACCGTCGcgccaccataacttcgtatag catacattatacgaagttatcggtcgacgtcgg |
| 14 | Neo^{R}-SalI-reverse | CCGACGTCGACCGATAACTT |
| 15 | M001-SalI-forward | ACGCgtcgacAGGATTTGGACCTGAGCATACT |
| 16 | M001-XhoI-reverse | CCGctcgagGAGGCCAAGAGAGGCTAAAGCC |
| 17 | M002-BamHI-forward | CGCggatccCATTCTCCCATCTCCAATTTAT |
| 18 | M002-EcoRI-reverse | GgaattcTTTTGTAACCCCTAGACAGATG |
| 19 | CMV-HindIII-forward | aagcttCCGCCATGTTGACATTG |
| 20 | CMV-reverse | CGGCCGCCCTATAGTG (5'-phosphorylated) |
| 21 | GFP-Neo-forward | AGATGGAGAGCGACGAGAGCGGCCT (5'- phosphorylated) |
| 22 | H001-SalI-forward | ACGCgtcgacTGCGTGAGATCTTTTCTTGGGG |
| 23 | H001-XhoI-reverse | CCGctcgagTCCACACACCCAAGTCATTCGA |
| 24 | H002-BamHI-forward | CGCggatccCTGAAGCCAACCAAGTTTAGGA |
| 25 | H002-HindIII-reverse | CCCaagcttCACATGGTGAACCCAAACACTC |
| 26 | CMV-XhoI-forward | ATCctcgagGACATTGATTATTGACTAG |
| 27 | CMV-KpnI-reverse | ATTggtaccCTCGGCCGCCCTATAG |
| 28 | Hygroloxm2/71-KpnI-forward | ATAggtaccTACCGTTCGTATATGGTTTCTTATACGAAGTTATG AATTCCACCATGAAAAAGCCTGAACTCAC |
| 29 | Hygro-lox66-SalI-reverse | ATCgtcgacTACCGTTCGTATAATGTATGCTATACGAAGTTATG GATCCTAAGATACATTGATG |
| 30 | PuroΔTK-lox71-XhoI-forward | ATTctcgagATAACTTCGTATAATGTATGCTATACGAACGGTAA TCGATCCCCAGCATGCCTGCTATTGTCTTC |
| 31 | PuroΔTK-loxm2/66-HindIII-reverse | CTCaagcttATAACTTCGTATATGGTTTCTTATACGAACGGTAC TTAAGCACCATGGGGACCGAGTACAAGCCCAC |
| 32 | Neo-HindIII-forward | CGCaagcttGTGTGTCAGTTAGGGTGTG |
| 33 | Neo-SalI-reverse | ATCgtcgacTAAGATACATTGATGAGTTTG |

### 2. Production of a cell containing a chromosome into which an antibiotic resistant gene and two or more RRSs are inserted

### 2-1. Production of a mouse cell containing a mouse chromosome into which an antibiotic resistant gene and two or more loxP are inserted

The J1 mouse embryonic stem cell (J1 mESC) used in the example was donated by Macrogen (Seoul, Korea). For proliferation and sustentation of the J1 mESC a dish coated with 0.1% gelatin was used, and a basic culture medium 2i was used. The basic culture medium was obtained by adding MEK inhibitor PD0325901 (1 pM) and GSK3 inhibitor CHIR99021 (3 pM) (both from Sigma Aldrich, St. Louis, MO, USA), and 1,000 U/ml LIF (Milliphore, Billerica, MA, USA) to an FBS-free N2B27 medium. The cells were cultured in an incubator that was maintained at 5% CO₂, 37°C, and a 95% wet state. Transient transfection was performed using Nepa21 (NEPAGENE Co., Ltd., Chiba, Japan) electroporator. The number of cells was 1×10⁶, and 100µl of an FBS-free, antibiotic-free opti-MEM medium was added. In addition, 10 µg of the first vector that was single cut with NotI was added to be transfected under conditions of 125 V, 5 ms, and 2 pulses. After the addition of 300 µl of the 2i medium and mixing of the cells, the cells were placed in a 100-mm dish, and then incubated for 24 to 48 hours in an incubator maintaining a 95% wet state, 5% CO₂, and 37°C.

To confirm the insertion of the first vector into the mESC, cells were selected through the expression of the Hygromycin resistant gene present in the first vector. As a drug, hygromycin B (Fujifilm Wako Pure Chemical Corporation, Osaka, Japan) was used. The cell selection concentration of hygromycin was measured by using a cell counting kit-8 (CCK-8; Dogindo, Kumamoto, Japan). The first vector was transfected into the mESC, and after 48 hours, it was treated with 32 ug/ml of the hygromycin. The cell selection process was performed for 4 to 6 weeks, and clones with antibiotic resistant genes and loxp were obtained and cultured.

As a result, by confirming the expression of the antibiotic resistant gene inserted into the first vector, all the cells in the control group were killed during the selection period, but the colony was identified from the group with the first vector during the selection period. The proliferation and sustentation of cells were also maintained by continuous addition of antibiotics, indicating that the expression of antibiotic resistant genes inserted by the first vector continuously occurred.

### 2-2. Production of a human cell containing a human chromosome into which an antibiotic resistant gene and two or more loxP are inserted

The used human normal foreskin fibroblast cell lines (BJ) were purchased from ATCC (Manassas, VA, USA). As the basic culture medium for proliferation and sustentation of the cells, Dulbecco's Modified Eagle's Medium (DMEM; Corning, Mannasas, VA, USA) containing 10% fetal bovine serum (FBS; Corning, Mannasas, VA, USA), 1% penicillin-streptomycin (Corning, Mannasas, VA, USA), and the cells were incubated in an incubator under conditions of a 95% wet state, 5% CO₂, and 37°C. Transient transfection was performed using Nepa21 (NEPAGENE Co., Ltd., Chiba, Japan) electroporator. The cell concentration was 1×10⁶ cells, and 100µl of an FBS-free, antibiotic-free opti-MEM medium was added. In addition, 10 µg of the second vector that was single cut with NotI was added to be transfected under conditions of 150V, 7.5ms, and 2 pulses. After adding 300 µl of the culture medium with 10% FBS added and well mixing the cells, the cells were placed in a 100-mm dish, and then incubated for 24 to 48 hours in an incubator that was maintained under conditions of a 95% wet state, 5% CO₂, and 37°C.

To confirm the insertion of the second vector into BJ, which was a human cell line, cells were selected using an antibiotic resistant gene present in the second vector. The cells of the BJ cell line were selected through expression of a Neomycin resistant gene present in the second vector. As a drug, G418 (Life technologies, NY, USA) was used. The cell selection concentration of G418 was measured with a cell counting kit-8 (CCK-8; Dogindo, Kumamoto, Japan). The second vector was transfected into the BJ cells, and after 48 hours, the cells were treated with 300 µg/ml of G418. The cell selection process was performed for 4 to 6 weeks, and clones with antibiotic resistant genes and loxp were obtained and cultured.

As a result, by confirming the expression of the antibiotic resistant gene inserted in the first vector, all the cells in the control group were killed during the selection period, but the colony was identified from the group with the second vector during the selection period. The proliferation and sustentation of the cells were maintained by continuous addition of antibiotics, indicating that the expression of antibiotic resistant genes inserted by second first vector continuously occurred.

### 3. Production of a microcell containing a human chromosome into which an antibiotic resistant gene and two or more loxP are inserted

Micronuclei were formed using colcemid (Life Technologies, Grand Island, NY, USA). The human normal foreskin fibroblast cell line (BJ) screed with G418 was placed in a 100-mm dish at a concentration of 1×10⁶, and then the next day, the medium was replaced with DMEM containing 20% FBS, and the cells were treated with 0.1 µg/ml of colcemid and incubated for 48 hours in an incubator under conditions of a 95% wet state, 5% CO₂, and 37°C. Human cells having undergone micronucleation were isolated using tryLE (Life technologies, Grand island, NY, USA), washed with Serum-free DMEM, and centrifuged at 1000 rpm for 5 minutes (Labogen Co., Ltd., Korea). The centrifuged cells were suspended on preheated serum-free DMEM:percoll ((Sigma Aldrich, St. Louis, MO, USA) (1:1 (v:v)) and were treated with cytochalsin B (Sigma Aldrich, St. Louis, MO, USA) to have a final concentration of 10 ug/ml. The centrifugation (LABOGEN) was performed at 16000 g, at 34°C to 37°C for 1 to 1.5 hours to obtain whole cells and microcells. The whole cells and the microcells were transferred to a 50-ml tube, serum-free DMEM was added thereto, and the resulting mixture was centrifuged at 500g for 10 minutes. The supernatant was carefully removed, pellets attached to the surface of the tube were placed in 10 ml of the serum-free DMEM, and microcells with a size of 8 um or less were separated using an 8-µm filter (GE healthcare, Chicago, IL, USA). The supernatant containing the microcells was centrifuged at 400 g for 10 minutes. The centrifuged microcells were separated using a 5-µm filter in the same manner as the filtration using the 8-µm filter. The finally separated microcells were counted using a Nicon eclipse TS100 optical microscope (Nicon Instruments, Melville, NY, USA) .

As a result, after the human normal foreskin fibroblast cell line (BJ) was treated with the colcemid, it was confirmed that micronuclei were present, using an optical microscope. The observation result was the same as the state shown in an existing paper. In addition, the micronuclei thus formed were enucleated using the cytochalasin B, and it was confirmed through optical microscopy that the microcells were sorted by size during the microcell size separation process. Through the process, it was confirmed that the microcells were generated and separated from targeting human cells, i.e., human normal foreskin fibroblast cell line (BJ).

### 4. Production of a fusion cell from a microcell and a mouse cell containing a mouse chromosome with an antibiotic resistant chromosome and loxp that are inserted

Separated human microcells were prepared, and mESCs which contained a mouse chromosome with loxp inserted and which were to be used as recipient cells were prepared. The mESCs were treated with TryLE and centrifuged. The centrifuged mESCs were washed with 1XDPBS (Welgene, Korea) and the number of cells was calculated using a hemacytometer. The fusion of the human microcells and the mESCs was performed by a suspension method, using the HVJ-E protein (Cosmo Bio Co., Ltd., Tokyo, Japan). The ratio of the human microcells to the mESCs was set to be 1:4. The prepared human microcells and the prepared mESCs were washed with 500 ul of a cold 1X cell fusion buffer. The human microcells and mESCs in the buffer were centrifuged at 4°C at 300 g for 5 minutes. 25 µl of the 1X cell fusion buffer was added per 2×10⁵ of mESCs, and the same volume of the 1X cell fusion buffer was added to the human microcells. The mESCs and the human microcells were mixed, 5 to 10 µl of the HVJ-E protein was added, and the mixture was left on ice for 5 minutes. The mixture was left in a 37°C water bath for 15 minutes. The mixture was tapped at intervals of 5 minutes. After the cell fusion of the human microcells and the mESCs was completed, the remaining HVJ-E protein was removed by centrifugation at 300 g for 5 minutes. The fusion cells were placed in a dish containing the culture medium of the mESCs and incubated for 48 hours in an incubator in which a 95% wet state, 5% CO₂, and 37°C were maintained.

After the cell fusion of the human microcells and the mESCs was performed, the fusion cells were put into the culture medium and observed under a microscope. As a result, it was confirmed that the mESCs and the microcells were fused. Since the method using the HVJ-E protein is a suspension method (it is performed to conduct experiments with single cells because the cell proliferation shape of the mESCs is a colony type), the real-time fusion process could not be observed but could be indirectly identified.

### 5. Production of a recombinant mouse cell containing a recombinant chromosome through chromosomal exchange

A recombinant mouse cell (i.e., recombinant mESC) containing a recombinant chromosome was produced by treating the fusion cell produced in the previously described experiment (4. Production of a fusion cell using a microcell and a mouse cell containing a mouse chromosome with an antibiotic resistant chromosome and loxp), with a recombinase. The recombinant chromosome is a recombinant mouse chromosome produced by exchanging (replacing) a hygromycin resistant gene located at the end of a variable region of a mouse immunoglobulin heavy chain locus with a puroΔTK gene located at the end of a variable region of a human immunoglobulin heavy chain locus.

The concentration of the fusion cells was 1×10⁶ cells, and 100 µl of an FBS-free, antibiotic-free opti-MEM medium was added. To express a Cre recombinase, 10 µg of the pCMV-Cre vector (System Biosciences, LLC, Palo Alto, CA, USA) was added and transfected at 125V, 5 ms, and 2 pulses. After the addition of 300 µl of the 2i medium, the cells were well mixed. The mixture was placed in a 100-mm dish, and then incubated for 48 hours in an incubator that was maintained under conditions of a 95% wet state, 5% CO₂, and 37°C.

After 48 hours, the fusion cells were dispensed to a 6-well plate, each well containing 5×10⁴ cells, followed by treatment with 0.6 µg/ml of puromycin. The concentration of puromycin was determined using a cell counting kit-8 (CCK-8; Dogindo, Kumamoto, Japan). The cells were incubated for a week and treated with a fresh medium and puromycin at intervals of 2 to 3 days. The cells were fixed and stained with Crystal violet (Sigma Aldrich, St. Louis, MO, USA), and the mESC colonies having a size of about 70 um were counted. The graphs were drawn using GraphPad PRISM (version 5.01), and statistical significance among the three groups was calculated using One-way ANOVA.

As a result, the process of selecting cells containing recombinant chromosomes was described in the above experiment (2-2. Production of a human cell containing a human chromosome with an antibiotic resistant gene and two loxP), but the cells were screened by normal expression of the puroΔTK gene inverted by the Cre recombinase used to treat the fusion cells. That is, the chromosomes of the human cell line BJ (chromosomes containing the inverted puroΔTK gene inserted by the second vector) migrated into the mESCs through the human microcells. By the Cre recombinase and the paring of two loxPs (lox71 and loxm2/66) located on the chromosome of the BJ (chromosome containing the inverted puroΔTK gene inserted by the second vector) and two loxPs (Loxm2/71 and lox66) located on the chromosome of the mESC (chromosome containing the hygromycin resistant gene inserted by the first vector) (pairing of lox71 and lox66 and pairing of loxm2/66 and Loxm2/71), a recombinant chromosome produced by exchanging (replacing) the hygromycin resistant gene with the puroΔTK gene was generated. The resulting recombinant chromosome include the re-inverted puroΔTK gene. Each group was treated with the recombinant chromosome, and the cell survival was observed. As a result, it was confirmed that the proliferation of the recombinant mESCs occurred in the group in which the Cre recombinase was expressed. This showed that a recombinant chromosome can be generated by exchange between a region (for example, antibiotic resistant gene) of a mouse chromosome and a region (for example, antibiotic resistant gene) of a human chromosome, and the recombinant chromosome can be normally expressed.

### 6. Production of a transgenic non-human animal using a recombinant mouse cell containing a recombinant chromosome

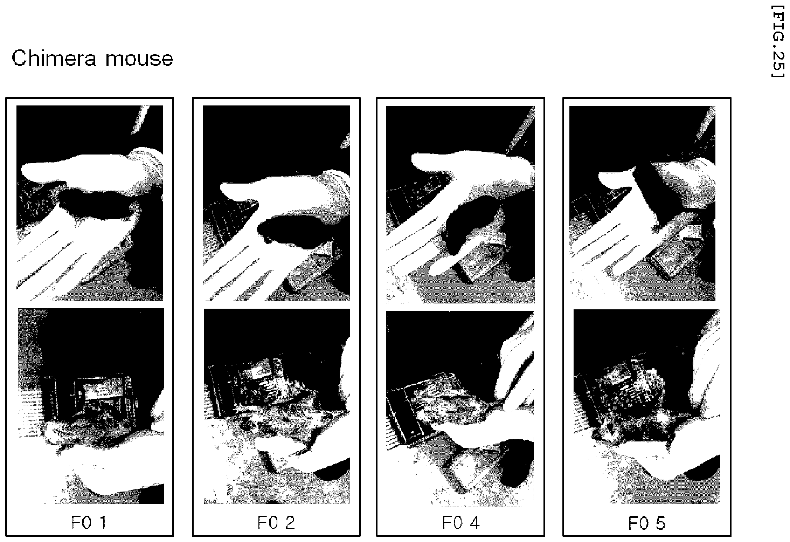

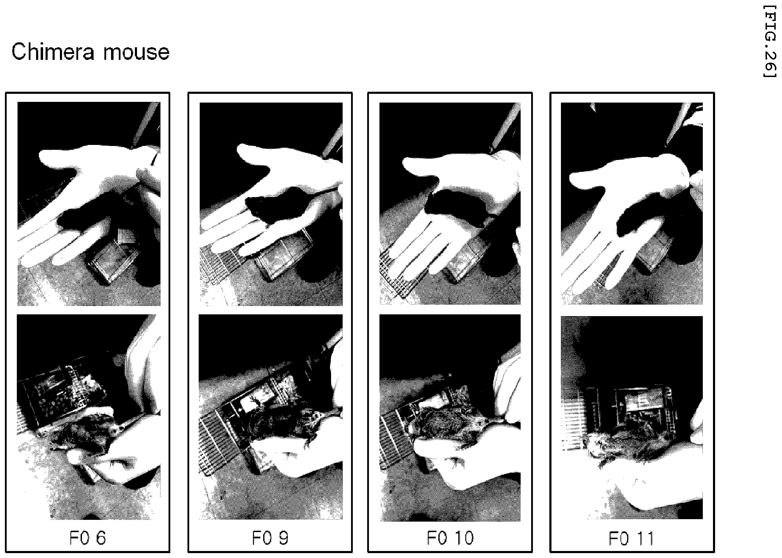

To prepare a transgenic non-human animal using a recombinant mouse cell containing a recombinant mouse chromosome, the recombinant mESC containing the recombinant chromosome obtained through the experiment (5. Production of a recombinant mouse cell containing a recombinant chromosome by chromosome exchange) was implanted into the blastula through the blastocyst injection, and thus a chimeric blastocyst was produced. The fabricated chimeric blastocyst was implanted into the womb of a surrogate mother, to obtain a mouse offspring (FIGS. 25 and 26). The mouse offspring produced was a chimeric transgenic mouse. A heterozygous transgenic mouse or a homozygous transgenic mouse can be produced by mating of the chimeric transgenic mice.

The produced transgenic mouse is a mouse containing the puroΔTK gene on the mouse genome. In this case, the transgenic mice can be produced by various methods as well as the blastocyst injection.

### Example 2. Method of producing a transgenic mouse having a genome having a humanized immunoglobulin locus through interchromosomal exchange

This example relates to a method of producing a transgenic mouse having a genome having a humanized immunoglobulin locus, and relates to a transgenic mouse in which a mouse immunoglobulin locus has a variable region of a human immunoglobulin gene instead of a variable region of a mouse immunoglobulin gene. The following description is about an example of a method of producing a transgenic mouse having a genome having a humanized immunoglobulin locus that is generated by exchanging (or replacing) a variable region of a mouse immunoglobulin heavy chain locus with a variable region of a human immunoglobulin gene. The intended transgenic mouse can be produced by diversely modifying the examples described below, or can be produced by using various methods other than the methods described in the examples.

### 1. Designing of vector

To exchange (or replace) the variable region of the mouse immunoglobulin gene with the variable region of the human immunoglobulin gene, vectors are designed for insertion of loxp into at both ends of each variable region. In this case, the inserted loxp is used for recombination of two chromosomes (the mouse chromosome on which the mouse immunoglobulin gene is located and the human chromosome on which the human immunoglobulin gene is located).

Two vectors (first and second vectors) were designed to insert loxp into each of both ends of the variable region of the mouse immunoglobulin gene.

The first vector (mTV2) includes a first homologous arm used for insertion into the 5 'end of a variable region of a mouse immunoglobulin heavy chain locus, a piggyBac terminal repeat (PB-TR), a promoter, a loxm2/71 (first RRS), a blasticidin resistant gene, a promoter, an FRT, and a second homologous arm used for insertion into the 5' end of a variable region of a mouse immunoglobulin heavy chain locus.

The second vector (mTV1) includes a third homologous arm used for insertion into the 3 'end of a variable region of a mouse immunoglobulin heavy chain locus, an inverted hygromycin resistant gene, a FRT, a lox66 (second RRS), a promoter, a neomycin resistant gene, a piggyBac terminal repeat (PB-TR), and a fourth homologous arm used for insertion into the 3' end of a variable region of a mouse immunoglobulin heavy chain locus.

In this case, the inverted selection element (antibiotic resistant gene) of the second vector was designed as one means to confirm that the first and second vectors were inserted into the same mouse immunoglobulin heavy chain locus. Since a mouse is a diploid organism, the mouse has pair of alleles, and this design allows identifying whether two vectors have been inserted into one allele.

In addition, two vectors (third and fourth vectors) were designed to insert loxp into each of both ends of a variable region of a human immunoglobulin gene.

The third vector (hTV2) includes a fifth homologous arm used for insertion into the 5' end of a variable region of a human immunoglobulin heavy chain locus, a promoter, a blasticidin resistant gene, lox71 (third RRS), a promoter, a FRT, a piggyBac terminal repeat (PB-TR), and a sixth homologous arm used for insertion into the 5' end of a variable region of a human immunoglobulin heavy chain locus.

The fourth vector (hTV1) includes a seventh homologous arm used for insertion into the 3' end of a variable region of a human immunoglobulin heavy chain locus, a piggyBac terminal repeat (PB-TR), an inverted hygromycin resistant gene, a FRT, an inverted puroΔTK gene, loxm2/66 (fourth RRS), a promoter, a neomycin resistant gene, and an eighth homologous arm used for insertion into the 3' end of a variable region of a human immunoglobulin heavy chain locus.

In this case, the inverted selection element (antibiotic resistant gene) of the fourth vector was designed as one means to confirm that the third and fourth vectors were inserted into the same mouse immunoglobulin heavy chain locus. Since a human is a diploid organism, the human has pair of alleles, and this design allows identifying whether two vectors have been inserted into one allele.

In this case, the vector design may vary depending on the insertion position and the loxp type, and the design may be changed to include various elements for the selection process.

### 2. Production of a cell containing a chromosome with Loxp inserted (FIGS. 27 to 37)

Mouse and human cells with loxp inserted were prepared using the vectors. In this case, when there are multiple vectors, the vectors may be introduced in sequence, in random, or simultaneously. The selection process of the cells into which the vector is introduced is diversely modifiable depending on the elements inserted into the vector.

### 2-1. Production of a mouse cell containing a mouse chromosome with loxp inserted (FIGS. 27 to 34)

For the mouse embryonic stem cell (mESC), a basic culture medium 2i was used. The basic culture medium was a culture medium (2i medium) prepared by adding MEK inhibitor PD0325901 (1 µM) and GSK3 inhibitor CHIR99021 (3 µM) (both from Sigma Aldrich, St. Louis, MO, USA), and 1,000 U/ml of LIF (Milliphore, Billerica, MA, USA) to a FBS-free N2B27 medium. The mESCs were incubated for proliferation and sustentation in an incubator maintaining conditions of a 95% wet state, 5% CO₂, and 37°C. Transient transfection was performed using Lipofectamine 3000 or Nepa21 (NEPAGENE Co., Ltd., Chiba, Japan) electroporator. For the transient transfection, 1×10⁶ cells were placed in an FBS-free, antibiotic-free medium. Next, 10 µg of the second vector was added thereto, the Lipofectamin 3000 reagent was added and mixed, and the resulting mixture was left at room temperature for 5 minutes before the transfection. Alternatively, 10 µg of the second vector was added, and the transfection was performed under conditions of 125 V, 5 ms, and 2 pulses. The transfected mESCs were incubated for 24 to 48 hours in an incubator maintaining conditions of a 95% wet state, 5% CO₂, and 37°C.

To determine whether the second vector was inserted into the 3' end of a variable region of a mouse immunoglobulin heavy chain locus, the transfected mESCs were treated with G418 (Life technologies, NY, USA), and the insertion of the second vector was confirmed by checking the viability of the cells. After treatment with G418, surviving mESCs were obtained, and PCR was used to determine whether the second vector was inserted (FIGS. 27 to 29). Samples identified through PCR were each sequenced, and a sample #80 was determined to have the second vector was obtained. The first vector was transfected into the obtained mESC (sample #80) in the same way as the second vector. The transfected mESCs were incubated for 24 to 48 hours in an incubator maintaining conditions of a 95% wet state, 5% CO₂, and 37°C. To determine whether the first vector was inserted into the 5' end of a variable region of a mouse immunoglobulin heavy chain locus, the transfected mESCs were treated with blasticidin, and the insertion of the second vector was confirmed by checking the viability of the mESCs. After the blasticidin treatment, surviving mESCs (#70 and #72 in FIG. 30) were obtained, and PCR and sequencing were performed to determine whether the first vector was inserted (FIG. 30).

To determine whether the first and second vectors were inserted into the same chromosome (chromosome having a mouse immunoglobulin heavy chain locus), the mESCs treated with G418 were treated a flippase (FLP), which is a recombinase. When the first and second vectors were inserted in the same chromosome, recombination is induced by FRTs present in the two vectors and treated FLPs, so that the variable region of the mouse immunoglobulin heavy chain locus was inverted. To confirm this, the cells for which FRT-FLP recombination was induced were treated with hygromycin, and whether the first and second vectors were inserted was determined by checking whether the mESC were survived. The surviving mESCs were obtained after Zeocin^{®} treatment. The obtained mESCs are mESCs in which loxm2/71 (first RRS) and lox66 (second RRS) are into the respective ends of the variable region of the mouse immunoglobulin heavy chain locus. Since a somatic cell has two alleles, this selection process is to exclude a case where only one of the first and second vectors is inserted into the two alleles.

### 2-2. Production of a human cell containing a human chromosome with loxp inserted (FIGS. 31 to 32)

The used human fibroblast was a BJ cell line (ATCC^{®} CRL-2522^{™} is white). To culture the BJ cells, Eagle's Minimum Essential Medium (EMEM) (ATCC^{®}30-2003^{™} medium) containing 10% fetal bovine serum (FBS; Corning, Mannasas, VA, USA) and 1% penicillin-streptomycin (Corning, Mannasas, VA, USA) was used. The BJ cells were incubated in an incubator maintaining conditions of a 95% wet state, 5% CO₂, and 37°C so that the BJ cells were proliferated and sustained. Transient transfection was performed using Lipofectamine 3000 (Invitrogen, Carlsbad, CA, USA) or Nepa21 (NEPAGENE Co., Ltd., Chiba, Japan) electroporator. For the transient transfection, 1×10⁶ cells were placed in an FBS-free, antibiotic-free medium. Next, 10 µg of the third vector was added thereto, the Lipofectamin 3000 reagent was added and mixed, and the resulting mixture was left at room temperature for 5 minutes before the transfection. Alternatively, 10 µg of the third vector was added, and the transfection was performed under conditions of 150 V, 7.5 ms, and 3 pulses. The transfected human fibroblast cells were incubated for 24 to 48 hours in an incubator maintaining conditions of a 95% wet state, 5% CO₂, and 37°C.

To determine whether the third vector was inserted into the 5' end of a variable region of a human immunoglobulin heavy chain locus, the transfected human fibroblast cells were treated with blasticidin, and the insertion of the second vector was confirmed by checking the viability of the third vector. After the blasticidin treatment, surviving fibroblast cells were obtained, and the insertion of the third vector was confirmed by PCR and sequencing (FIGS. 31 to 32). The fourth vector was transfected into the obtained human fibroblast (sample #15 in FIGS. 31 and 32) in the same manner as the third vector. The transfected cells were incubated for 24 to 48 hours in an incubator maintaining conditions of a 95% wet state, 5% CO₂, and 37°C. To determine whether the fourth vector was inserted into the 3' end of a variable region of a human immunoglobulin heavy chain locus, the transfected human fibroblast cells were treated with G418 (Life technologies, NY, USA), and the insertion of the fourth vector was confirmed by checking the viability of the human fibroblast cells. Surviving human fibroblast cells were obtained after the G418 treatment.

To determine whether the third and fourth vectors were inserted into the same chromosome (chromosome having a human immunoglobulin heavy chain locus), the human fibroblast cells treated with G418 mESCs were treated a flippase (FLP), which is a recombinase. When the third and fourth vectors were inserted in the same chromosome, recombination was induced by FRTs present in the two vectors and treated FLPs, so that the variable region of the human immunoglobulin heavy chain locus was inverted. To confirm this, the human fibroblast cells for which FRT-FLP recombination was induced were treated with hygromycin, and the insertion of the third and fourth vectors into the same chromosome was confirmed by checking whether the human fibroblast cells were survived. The surviving human fibroblast cells were obtained after Zeocin^{®} treatment. The obtained human fibroblast cells are human fibroblast cells containing a chromosome in which lox71 (third RRS) and loxm2 (fourth RRS) are into the respective ends of the variable region of the human immunoglobulin heavy chain locus. Since a somatic cell has two alleles, this selection process was to exclude a case where only one of the third and fourth vectors was inserted into the two alleles.

### 3. Production of a microcell using a human fibroblast containing a human chromosome with loxp inserted

Micronuclei were formed using colcemid (Life Technologies, Grand Island, NY, USA). The selected human fibroblast cells (cells containing chromosomes in which lox71 (third RRS) and loxm2/66 (fourth RRS) were inserted into the respective ends of a variable region of a human immunoglobulin heavy chain locus) were cultured in a density of 1×10⁶ cells. On the next day, the culture medium was replaced with EMEM containing 20% FBS, and the cells were treated with 0.1 pg/ml of colcemid and incubated for 48 hours in an incubator maintained in the conditions of a 95% wet state, 5% CO₂, and 37°C. The micronucleated human fibroblast cells were detached using tryLE (Life technologies, Grand island, NY, USA), washed with serum-free EMEM, and centrifuged at 1000 rpm for 5 minutes (LABOGENE Co., Ltd., Korea). The centrifuged cells were suspended on preheated serum-free EMEM:percoll ((Sigma Aldrich, St. Louis, MO, USA) (1:1 (v:v)) and were treated with cytochalsin B (Sigma Aldrich, St. Louis, MO, USA) to have a final concentration of 10 ug/ml. The centrifugation (LABOGEN) was performed at 16000 g, at 34°C to 37°C for 1 to 1.5 hours to obtain whole cells and microcells. The whole cells and the microcells were transferred to a 50-ml tube, serum-free EMEM was added thereto, and the resulting mixture was centrifuged at 500 g for 10 minutes. The supernatant was carefully removed, pellets attached to the surface of the tube were placed in 10 ml of the serum-free EMEM, and microcells with a size of 8 µm or less were separated using an 8-µm filter (GE healthcare, Chicago, IL, USA). The supernatant containing the microcells was centrifuged at 400 g for 10 minutes. The centrifuged microcells were separated using a 5-µm filter in the same manner as the filtration using the 8-µm filter. The finally separated microcells were counted using a Nicon eclipse TS100 optical microscope (Nicon Instruments, Melville, NY, USA). In this way, human microcells were obtained from the human fibroblast.

### 4. Production of a fusion cell using a microcell and a mouse cell containing a mouse chromosome with loxp inserted

The separated human microcells and the mESCs to be used as recipient cells were prepared. The mESC included a chromosome in which loxps ((loxm2/71 (first RRS) and lox66 (second RRS)) were inserted into the respective ends of a variable region of a mouse immunoglobulin heavy chain locus. The mESCs were treated with TryLE and centrifuged. The centrifuged mESCs were washed with 1XDPBS and the number of cells was calculated using a hemacytometer. The fusion of the human microcells and the mESCs was performed by a suspension method, using the HVJ-E protein (Cosmo Bio Co., Ltd., Tokyo, Japan). The ratio of the human microcells and the mESCs was 1:4. The prepared human microcells and the prepared mESCs were washed with 500 ul of a cold 1x cell fusion buffer. The human microcells and mESCs contained in the buffer were centrifuged at 4°C at 300 g for 5 minutes. 25 µl of the 1x cell fusion buffer was added per 2×10⁵ cells of mESCs, and the same volume of the 1x cell fusion buffer was added to the human microcells. The mESCs and the human microcells were mixed, 5 to 10 µl of the HVJ-E protein was added, and the mixture was left on ice for 5 minutes. The mixture was left in a 37°C water bath for 15 minutes. The mixture was tapped at intervals of 5 minutes. After the cell fusion of the human microcells and the mESCs was completed, the remaining HVJ-E protein was removed by centrifugation at 300 g for 5 minutes. The fusion cells were incubated in an incubator containing the culture medium of the mESCs and maintaining the conditions of a 95% wet state, 5% CO₂, and 37°C, for 48 hours. The produced fusion cell was a fused mESC containing a human chromosome and a mouse chromosome, in which the human chromosome was a chromosome including a variable region of a human immunoglobulin heavy chain locus into which lox71 (third RRS) and loxm2/66 (fourth RRS) were inserted, and the mouse chromosome was a chromosome including a variable region of a mouse immunoglobulin heavy chain locus into which loxm2/71 (first RRS) and lox66 (second RRS) were inserted.

### 5. Production of a cell containing a humanized immunoglobulin gene by chromosomal exchange

The produced fusion cell was treated with a recombinase to induce exchange (or replacement) between a variable region of a mouse immunoglobulin heavy chain locus and a variable region of a human immunoglobulin heavy chain locus, thereby producing a recombinant cell, i.e., recombinant mESC, having a humanized immunoglobulin heavy chain locus (humanized immunoglobulin heavy chain locus). In this case, the humanized immunoglobulin heavy chain locus (humanized immunoglobulin heavy chain locus) is a locus having a variable region derived from a human immunoglobulin heavy chain gene and a constant region of a mouse immunoglobulin heavy chain gene.

100 µl of an FBS-free, antibiotic-free opti-MEM medium was added to 1×10⁶ fusion cells (fusion mESCs) . 10 µg of the pCMV-Cre vector (System Biosciences, LLC, Palo Alto, CA, USA) was added thereto, and transfection was performed under conditions of 125 V, 5 ms, and 2 pulses. After the addition of 300 µl of the 2i medium and mixing of the fusion cells, the mixture was transferred to a 100-mm dish, and the cells were incubated in an incubator maintaining a 95% wet state, 5% CO₂, and 37°C.

To confirm whether a chromosome having a humanized immunoglobulin locus was generated in the fusion cell treated with the Cre recombinase, the fusion cell treated with the Cre recombinase was treated with an antibiotic ((Puromycin, G418 and hygromycin). In the case of a Cre recombinase-treated fusion cell, a recombination of a human chromosome and a mouse chromosome was induced, in which the human chromosome was a chromosome including a variable region of a human immunoglobulin heavy chain locus into which lox71 (third RRS) and loxm2/66 (fourth RRS) were inserted, and the mouse chromosome was a chromosome including a variable region of a mouse immunoglobulin heavy chain locus into which loxm2/71 (first RRS) and lox66 (second RRS) were inserted. The first RRS in the mouse chromosome and the fourth RRS in the human chromosome are paired with each other, and the second RRS in the mouse chromosome and the third RRS in the human chromosome are paired with each other. The pairing of the RRSs is recognized by the Cre recombinase, and thus the recombination is induced.

As a result, a first recombinant chromosome in which the variable region of the mouse immunoglobulin heavy chain locus of the mouse chromosome has been replaced with the variable region of the human immunoglobulin heavy chain gene (a mouse chromosome having a humanized immunoglobulin locus) and a second recombinant chromosome in which the variable region of the human immunoglobulin heavy chain locus of the human chromosome has been replaced with the variable region of the mouse immunoglobulin heavy chain gene are produced. In the case of the first recombinant chromosome, the portions other than the variable region of the human immunoglobulin heavy chain gene has a mouse gene (for example, the constant region of the mouse immunoglobulin heavy chain locus is a mouse gene). In the case of the second recombinant chromosome, the portions other than the variable region of the mouse immunoglobulin heavy chain gene have a human gene (for example, the constant region of the human immunoglobulin heavy chain locus is a human gene) .

Surviving cells (mESCs) were obtained after the treatment with antibiotics (Puromycin, G418, and hygromycin). The obtained cell is an mESC containing a first recombinant chromosome and a second recombinant chromosome.

The obtained mESC containing the first recombinant chromosome and the second recombinant chromosome is treated with a piggyBac transposase to remove the RRSs, the PuroΔTK gene, the neomycin resistant gene (NeoR), the hygromycin resistant gene (hygromycin resistant gene), and the FRT included in the first and second recombinant chromosomes. In this case, cells containing the RRSs, the puroΔTK gene, the neomycin resistant gene (NeoR), the hygromycin resistant gene, and the FRT-free recombinant chromosome are treated with Fialuridine (FIAU) and selected.

The recombinant chromosomes may be recombined in many ways depending on the locations, directions, and pairing of the RRSs. For the production of a recombinant chromosome of interest, the design of the vector may be changed to enable production.

### 6. Production of a transgenic mouse using a recombinant mESC with a humanized immunoglobulin locus

A transgenic mouse is produced using a recombinant mESCs having a humanized immunoglobulin locus (chimeric immunoglobulin locus).

The recombinant mESCs having a humanized immunoglobulin locus, obtained in the manner described above, is implanted into the blastula by blastocyst injection to produce a chimeric blastocyst. The fabricated chimeric blastocyst is implanted into the womb of a surrogate mother, to obtain a mouse offspring (FIGS. 25 and 26). The produced mouse offspring is a chimeric transgenic mouse, and a heterozygous transgenic mouse or a homozygous transgenic mouse can be produced by mating the chimeric transgenic mice. In the produced transgenic mouse, the variable region of an immunoglobulin heavy chain locus on the genome is humanized (i.e., exchanged (or replaced) with the variable region of a human immunoglobulin locus). In this case, the transgenic mice can be produced by various methods, aside from the blastocyst injection.

### Example 3. Method of producing a transgenic mouse having a genome having a humanized immunoglobulin locus through interchromosomal exchange

This example relates to a method of producing a transgenic mouse having a genome having a humanized immunoglobulin locus, and relates to a transgenic mouse in which a mouse immunoglobulin locus has a variable region of a human immunoglobulin gene instead of a variable region of a mouse immunoglobulin gene. Although the method of the present example is overall similar to that of Example 2, Example 2 and Example 3 differ in the used vector and the selection method. The following description is only about a method for producing a transgenic mouse, but the present disclosure is not limited thereto. The intended transgenic mouse can be produced by diversely modifying the examples described below, or can be produced by using various methods other than the methods described in the examples.

### 1. Designing of vector

To exchange (or replace) the variable region of the mouse immunoglobulin gene with the variable region of the human immunoglobulin gene, vectors are designed for insertion of loxps into the respective ends of each variable region. In this case, the inserted loxps are used for recombination of two chromosomes (the mouse chromosome on which the mouse immunoglobulin gene is located and the human chromosome on which the human immunoglobulin gene is located).

Two vectors (first and second vectors) were designed to insert loxps into the respective ends of the variable region of the mouse immunoglobulin gene.

The first vector includes a first homologous arm used for insertion into the 5' end of the variable region of the mouse immunoglobulin heavy chain locus, loxm2/71 (first RRS), EF1α promoter, a hygromycin resistant gene) and a second homologous arm used for insertion into the 5' end of the variable region of the mouse immunoglobulin heavy chain locus.

The second vector includes a third homologous arm used for insertion into the 3' end of the variable region of the mouse immunoglobulin heavy chain locus, a CAG promoter, a puroΔTK gene, lox66 (second RRS), and a fourth homologous arm used for insertion into the 3' end of the variable region of the mouse immunoglobulin heavy chain locus.

In this case, each of the first and second vectors includes a selection element (for example, puroΔTK gene) used for a positive selection and a negative selection.

In addition, two vectors (third and fourth vectors) were designed to insert loxps into the respective ends of a variable region of a human immunoglobulin gene.

The third vector includes a fifth homologous arm used for insertion into the 5' end of the variable region of a human immunoglobulin heavy chain locus, a EF1α promoter, a puroΔTK gene, loxm2/66 (second RRS), and a sixth homologous arm used for insertion into the 5' end of the variable region of the human immunoglobulin heavy chain locus.

The fourth vector includes a seventh homologous arm used for insertion into the 3' end of the variable region of a human immunoglobulin heavy chain locus, lox71 (fourth RRS), CAG promoter, a neomycin resistant gene, and an eighth homologous arm for insertion into the 3' end of the variable region of the human immunoglobulin heavy chain locus.

In this case, the third vector or the fourth vector includes a selection element not included in the first vector or the second vector. This design was intended to confirm that a human chromosome (including the third RRS and the fourth RRS) migrated to a mouse cell (containing a mouse chromosome into which the first RRS and the second RRS are inserted) using the microcell and the cell fusion.

In this case, the vector design may vary depending on the insertion position and the loxp type, and the design change can be made to include various elements for the selection process.

### 2. Production of a cell containing a chromosome with loxp inserted

Mouse and human cells with loxp inserted were prepared using the vectors. In this case, when there are multiple vectors, the vectors may be introduced in sequence, in random, or simultaneously. The selection process of the cells into which the vector is introduced is diversely modifiable depending on the elements inserted into the vector.

### 2-1. Production of a mouse cell containing a chromosome with loxp inserted

For the mouse embryonic stem cell (mESC), a basic culture medium 2i was used. The basic culture medium was a culture medium (2i medium) prepared by adding MEK inhibitor PD0325901 (1 µM) and GSK3 inhibitor CHIR99021 (3 µM) (both from Sigma Aldrich, St. Louis, MO, USA), and 1,000 U/ml of LIF (Milliphore, Billerica, MA, USA) to a FBS-free N2B27 medium. The mESCs were incubated for proliferation and sustentation in an incubator maintained under conditions of a 95% wet state, 5% CO₂, 37°C. Transient transfection was performed using Lipofectamine 3000 or Nepa21 (NEPAGENE Co., Ltd., Chiba, Japan) electroporator. For the transient transfection, 1×10⁶ cells were placed in an FBS-free, antibiotic-free medium. Each of the first and second vectors was added thereto in an amount of 5 µg, the Lipofectamin 3000 reagent was added thereto and mixed, and the resulting mixture was left at room temperature for 5 minutes, followed by transfection. Alternatively, each of the first and second vectors was added thereto in an amount of 5 µg, and the transfection was performed under conditions of 125 V, 5 ms, and 1 pulse. The transfected mESCs were incubated for 24 to 48 hours in an incubator maintaining the conditions of a 95% wet state, 5% CO₂, and 37°C.

To confirm whether the first vector and the second vector were inserted respectively into the 5' end and 3' end of the variable region of the mouse heavy chain locus, the transfected mESCs were treated with hygromycin and puromycin. Whether the first and second vectors were inserted was determined according to survival or death of the cells. After the cells were treated with an antibiotic, the surviving mESCs were collected, and PCR was performed to determine whether the first and second vectors were inserted (FIG. 33).

Sample #2 illustrated in FIG. 38 was targeted by the first and second vectors, and it was confirmed that the first and second vectors were inserted into the respective ends of a variable region of ta mouse endogenous immunoglobulin heavy chain gene. In this case, the PCR conditions and target band size are as follows:
The target band size of a portion of the 5' end of the variable region of the mouse endogenous immunoglobulin heavy chain gene and a portion of the loxm2/71 (first RRS) and EF1α promoter included in the first vector is 2.2 kb, in which mIgHP-left-F2 and EF1α-R3 are used as primers, and the PCR is performed with 1 cycle of an initial denaturation (94°C, 5 minutes), 35 cycles of denaturation (94°C, 1 minute) -annealing (64°C, 1 minute) -extension (72°C, 3 minutes), and 1 cycle of a final extension (72°C, 10 minute);
the target band size of a portion of the hygromycin resistant gene included in the first vector and a portion (denoted by mHP right) of the 5' end of the variable region of the mouse endogenous immunoglobulin heavy chain gene is 3.7 kb, in which HygR-Rl and mTV-targeting Right F2 are used as primers, and the PCR is performed with 1 cycle of an initial denaturation (94°C, 5 minute), 35 cycles of denaturation (94°C, 1 minute)-annealing (62°C, 2 minutes)-extension (72°C, 4 minutes), and 1 cycle of a final extension (72°C, 10 minutes);
the target band size of a portion of the 3' end of the variable region of the mouse endogenous immunoglobulin heavy chain gene and a portion (denoted by mHM left) of a CAG promoter included in the second vector is 2.0 kb, in which mIgHM-F1 and CAG-R1 are used as primers, and the PCR is performed with 1 cycle of an initial denaturation (94°C, 5 minutes), 35 cycles of denaturation (94°C, 1 minute) -annealing (63°C, 1 minute)-extension (72°C, 2 minutes), and 1 cycle of a final extension (72°C, 10 minutes); and
the target band size of a portion of the puroΔTK gene included in the second vector and a portion (denoted by mHM right) of the 3' end of the variable region of the mouse endogenous immunoglobulin heavy chain gene is 1.6 kb, in which TK-F1 and mlgHM-right-R1 are used as primers, and the PCR is performed with 1 cycle of an initial denaturation (94°C, 5 minute), 35 cycles of denaturation (94°C, 1 minute)-annealing (63°C, 1 minute)-extension (72°C, 2 minutes), and 1 cycle of a final extension (72°C, 10 minutes).

The used primers are summarized in Table 6.

**[Table 6]**

| **No.** | **Name** | **Sequence (5'_{→}3')** | **SEQ ID NO:** |
|---|---|---|---|
| 1 | mIgHP-left-F2 | ttggtcctcctccttctaagc | 34 |
| 2 | EF1α-R3 | gccataacccgtaaagaggc | 35 |
| 3 | HygR-Rl | CCGATGGCTGTGTAGAAGTAC | 36 |
| 4 | mTV2-targeting Right F2 | ATT TCA CAC TGC ATA TTT CAC CCA | 37 |
| 5 | mIgHM-left-F1 | ACTCTGATGTCTAGGACCAGG | 38 |
| 6 | CAG-R1 | ACGCGGAACTCCATATATGGG | 39 |
| 7 | TK-F1 | tcc tgg att acg acc aat cgc | 40 |
| 8 | mIgHM-right-R1 | cttcacgtcactgactgactg | 41 |

In addition, sequencing was performed to determine whether the target band was satisfied, using the detected PCR product. As a result, it was confirmed that the first and second vectors were precisely and respectively inserted into the targeted 5' end and 3' end of the variable region of the mouse endogenous immunoglobulin heavy chain gene.

The cells thus obtained were used as recipient cells as described below.

### 2-2. Production of a human cell containing a human chromosome with loxp inserted

The used human fibroblast was a BJ cell line (ATCC^{®} CRL-2522^{™} is white). To culture the BJ cells, Eagle's Minimum Essential Medium (EMEM) (ATCC^{®}30-2003^{™} medium) containing 10% fetal bovine serum (FBS; Corning, Mannasas, VA, USA) and 1% penicillin-streptomycin (Corning, Mannasas, VA, USA) was used. The BJ cells were incubated in an incubator maintaining conditions of a 95% wet state, 5% CO₂, and 37°C so that the BJ cells were proliferated and sustained. Transient transfection was performed using Lipofectamine 3000 (Invitrogen, Carlsbad, CA, USA) or Nepa21 (NEPAGENE Co., Ltd., Chiba, Japan) electroporator. For the transient transfection, 1×10⁶ cells were placed in an FBS-free, antibiotic-free medium. Each of the third and fourth vectors was added thereto in an amount of 5 µg, the Lipofectamin 3000 reagent was added thereto and mixed, and the resulting mixture was left at room temperature for 5 minutes, followed by transfection. Alternatively, each of the third and fourth vectors was added thereto in an amount of 5 µg, and the transfection was performed under conditions of 150 V, 7.5 ms, and 2 pulses. The transfected human fibroblast cells were incubated for 24 to 48 hours in an incubator maintaining the conditions of a 95% wet state, 5% CO₂, and 37°C.

To confirm whether the third vector and the fourth vector were inserted respectively into the 5' end and 3' end of the variable region of a human immunoglobulin heavy chain locus, the transfected human fibroblast cells were treated with hygromycin and G418. Whether the third and fourth vectors were inserted was determined according to survival or death of the cells. After the cells were treated with an antibiotic, the surviving human fibroblast cells were collected, and PCR was performed to determine whether the third and fourth vectors were inserted (FIG. 34).

Sample #4 illustrated in FIG. 34 was targeted by the third and fourth vectors, and it was confirmed that the third and fourth vectors were inserted into the respective ends of a variable region of a human endogenous immunoglobulin heavy chain gene. In this case, the PCR conditions and target band size are as follows:
the target band size of a portion of the 5' end of the variable region of the human endogenous immunoglobulin heavy chain gene and a portion (denoted by hHP left) of a EF1α promoter included in the third vector is 3.1 kb, in which hTV2-left-F5 and EF1α-R3 are used as primers, and the PCR is performed with 1 cycle of an initial denaturation (94°C, 5 minutes), 35 cycles of denaturation (94°C, 0.5 minute) - annealing (64°C, 0.5 minute)-extension (72°C, 3 minutes), and 1 cycle of a final extension (72°C, 10 minutes);
the target band size of a portion of the hygromycin resistant gene included in the third vector and a portion (denoted by hHP right) of the 5' end of the variable region of the mouse endogenous immunoglobulin heavy chain gene and loxm2/66 (third RRS) is 1.6 kb, in which HygR-Rl and hTV2-right-R1 are used as primers, and the PCR is performed with 1 cycle of an initial denaturation (94°C, 5 minutes), 35 cycles of denaturation (94°C, 0.5 minute)-annealing (64°C, 0.5 minutes)-extension (72°C, 2 minutes), and 1 cycle of a final extension (72°C, 10 minutes);
the target band size of a portion of the 3' end of the variable region of the human endogenous immunoglobulin heavy chain gene and a portion (denoted by hHM left) of a CAG promoter and lox71 (fourth RRS) included in the fourth vector is 2.0 kb, in which hIgHM-left-F1 and CAG-R1 are used as primers, and the PCR is performed with 1 cycle of an initial denaturation (94°C, 5 minutes), 35 cycles of denaturation (94°C, 1 minute) -annealing (64°C, 1 minute)-extension (72°C, 2 minutes), and 1 cycle of a final extension (72°C, 10 minutes); and
the target band size of a portion of the hygromycin resistant gene included in the fourth vector and a portion (denoted by hHM right) of the 3' end of the variable region of the human endogenous immunoglobulin heavy chain gene is 2.7 kb, in which Neo-F2 and hIgHM-right-R1 are used as primers, and the PCR is performed with 1 cycle of an initial denaturation (94°C, 5 minute), 35 cycles of denaturation (94°C, 1 minute)-annealing (64°C, 1 minute)-extension (72°C, 3 minutes), and 1 cycle of a final extension (72°C, 10 minutes).

The used primers are summarized in Table 7.

**[Table 7]**

| **No.** | **Name** | **Sequence (5'_{→}3')** | **SEQ ID NO:** |
|---|---|---|---|
| 1 | hTV2-left-F5 | CTGCAGACATCACCCTCATTC | 42 |
| 2 | EF1α-R3 | gccataacccgtaaagaggc | 35 |
| 3 | HygR-Rl | CCGATGGCTGTGTAGAAGTAC | 36 |
| 4 | hTV2-right-R4 | GTA AGA AGG GTG GAG ACA CAG | 43 |
| 5 | hIgHM-left-F1 | tgtggctacaagtgcttggag | 44 |
| 6 | CAG-R1 | ACGCGGAACTCCATATATGGG | 39 |
| 7 | Neo-F2 | TGTCATCTCACCTTGCTCCTG | 45 |
| 8 | hIgHM-right-R1 | gtgcgatgatgacctacagtc | 46 |

Sequencing was performed to determine whether the target band was satisfied, using the detected PCR product. As a result, it was confirmed that the third and fourth vectors were precisely and respectively inserted into the targeted 5' end and 3' end of the variable region of the human endogenous immunoglobulin heavy chain gene.

The cells thus obtained were used as donor cells as described below.

### 3. Production of a microcell using a human fibroblast containing a human chromosome with loxp inserted

Micronuclei were formed using colcemid (Life Technologies, Grand Island, NY, USA). The selected human fibroblast cells (cells containing chromosomes in which loxm2/66 (third RRS) and lox71 (fourth RRS) were inserted into the respective ends of a variable region of a human immunoglobulin heavy chain locus) were cultured in a density of 1×10⁶ cells. On the next day, the culture medium was replaced with EMEM containing 20% FBS, and the cells were treated with 0.1 pg/ml of colcemid and incubated for 48 hours in an incubator maintained in the conditions of a 95% wet state, 5% CO₂, and 37°C. The micronucleated human fibroblast cells were detached using tryLE (Life technologies, Grand island, NY, USA), washed with serum-free EMEM, and centrifuged at 1000 rpm for 5 minutes (LABOGENE Co., Ltd., Korea). The centrifuged cells were suspended on preheated serum-free EMEM:percoll ((Sigma Aldrich, St. Louis, MO, USA) (1:1 (v:v)) and were treated with cytochalsin B (Sigma Aldrich, St. Louis, MO, USA) to have a final concentration of 10 ug/ml. The centrifugation (LABOGEN) was performed at 16000 g, at 34°C to 37°C for 1 to 1.5 hours to obtain whole cells and microcells. The whole cells and the microcells were transferred to a 50-ml tube, serum-free EMEM was added thereto, and the resulting mixture was centrifuged at 500 g for 10 minutes. The supernatant was carefully removed, pellets attached to the surface of the tube were placed in 10 ml of the serum-free EMEM, and microcells with a size of 8 µm or less were separated using an 8-µm filter (GE healthcare, Chicago, IL, USA). The supernatant containing the microcells was centrifuged at 400 g for 10 minutes. The centrifuged microcells were separated using a 5-µm filter in the same manner as the filtration using the 8-µm filter. The finally separated microcells were counted using a Nicon eclipse TS100 optical microscope (Nicon Instruments, Melville, NY, USA). In this way, human microcells were obtained from the human fibroblast.

### 4. Production of a fusion cell using a microcell and a mouse cell containing a mouse chromosome with loxp inserted

Separated human microcells were prepared, and mESCs which were to be used as recipient cells and into which loxp was inserted were prepared, in which the mESCs were cells containing a chromosome in which loxm2/71 (first RRS) and lox66 (second RRS) were inserted into the respective ends of a variable region of a mouse immunoglobulin heavy chain locus. The mESCs were treated with TryLE and centrifuged. The centrifuged mESCs were washed with 1XDPBS and the number of cells was calculated using a hemacytometer. The fusion of the human microcells and the mESCs was performed by a suspension method, using the HVJ-E protein (Cosmo Bio Co., Ltd., Tokyo, Japan) . The ratio of the human microcells and the mESCs was 1:4. The prepared human microcells and the prepared mESCs were washed with 500 ul of a cold 1x cell fusion buffer. The human microcells and mESCs contained in the buffer were centrifuged at 4°C at 300 g for 5 minutes. 25 µl of the 1x cell fusion buffer was added per 2×10⁵ cells of mESCs, and the same volume of the 1x cell fusion buffer was added to the human microcells. The mESCs and the human microcells were mixed, 5 to 10 µl of the HVJ-E protein was added, and the mixture was left on ice for 5 minutes. The mixture was left in a 37°C water bath for 15 minutes. The mixture was tapped at intervals of 5 minutes. After the cell fusion of the human microcells and the mESCs was completed, the remaining HVJ-E protein was removed by centrifugation at 300 g for 5 minutes. To select fusion cells, the fusion cells were treated with G418 (Life Technologies, NY, USA) at a concentration of 200 ug/ml for one week. The surviving fusion cells after being treated with G418 were fusion mESCs containing a human chromosome and a mouse chromosome, in which the human chromosome was a chromosome including a variable region of a human immunoglobulin heavy chain locus into which loxm2/66 (third RRS) and lox71 (fourth RRS) were inserted, and the mouse chromosome was a chromosome including a variable region of a mouse immunoglobulin heavy chain locus into which loxm2/71 (first RRS) and lox66 (second RRS) were inserted. In this case, the selection of the fusion mESCs through the G418 treatment can be omitted.

After cell fusion was performed on J1 mES cells, G418 treatment was performed at a concentration of 200 ug/ml for 3 to 7 days, and colony was collected and cultured. gDNA was collected by PCIA extraction from 1.0 × 10⁵ cells among the cultured cells, and genotyping was performed by PCR.

### 5. Production of a cell containing a humanized immunoglobulin gene by chromosomal exchange

The produced fusion cells were treated with a recombinase so that a variable region of a mouse immunoglobulin heavy chain locus is exchanged (or replaced) with a variable region of a human immunoglobulin heavy chain region. Thus, recombinant cells having a humanized immunoglobulin heavy chain locus were produced. That is, recombinant mESCs were produced. In this case, the humanized immunoglobulin heavy chain locus is a locus having a variable region derived from a human immunoglobulin heavy chain gene and a constant region of a mouse immunoglobulin heavy chain gene.

100 µl of an FBS-free, antibiotic-free opti-MEM medium was added to 1×10⁶ fusion cells (fusion mESCs) . 10 µg of the pCMV-Cre vector (System Biosciences, LLC, Palo Alto, CA, USA) was added thereto, and transfection was performed under conditions of 125 V, 5 ms, and 2 pulses. After the addition of 300 µl of the 2i medium and mixing of the fusion cells, the mixture was transferred to a 100-mm dish, and the cells were incubated in an incubator maintaining a 95% wet state, 5% CO₂, and 37°C.

In the case of a Cre recombinase-treated fusion cell, recombination of a human chromosome and a mouse chromosome was induced, in which the human chromosome was a chromosome including a variable region of a human immunoglobulin heavy chain locus into which loxm2/66 (third RRS) and lox71/66 (fourth RRS) were inserted, and the mouse chromosome was a chromosome including a variable region of a mouse immunoglobulin heavy chain locus into which loxm2/71 (first RRS) and lox66 (second RRS) were inserted. The first RRS in the mouse chromosome and the third RRS in the human chromosome are paired with each other, and the second RRS in the mouse chromosome and the fourth RRS in the human chromosome are paired with each other. The pairing of the RRSs is recognized by the Cre recombinase, and thus the recombination is induced.

As a result, a first recombinant chromosome in which the variable region of the mouse immunoglobulin heavy chain locus of the mouse chromosome has been replaced with the variable region of the human immunoglobulin heavy chain gene (a mouse chromosome having a humanized immunoglobulin locus) and a second recombinant chromosome in which the variable region of the human immunoglobulin heavy chain locus of the human chromosome has been replaced with the variable region of the mouse immunoglobulin heavy chain gene are produced. In the case of the first recombinant chromosome, the portions other than the variable region of the human immunoglobulin heavy chain gene has a mouse gene (for example, the constant region of the mouse immunoglobulin heavy chain locus is a mouse gene). In the case of the second recombinant chromosome, the portions other than the variable region of the mouse immunoglobulin heavy chain gene have a human gene (for example, the constant region of the human immunoglobulin heavy chain locus is a human gene).

To confirm that a first recombinant chromosome having a humanized immunoglobulin heavy chain locus is generated in the fusion cell treated with the Cre recombinase, the fusion cells treated with the Cre recombinase are cultured for a predetermined period of time, the cultured cells are then treated with fialuridine(1-(2-deoxy-2-fluoro-1-D-arabinofuranosyl)-5-iodouracil) (FIAU), and surviving fusion cells are selected. The surviving cells (mESCs) were collected after the FIAU treatment. The obtained cells were mESCs containing the first recombinant chromosome. When the puroΔTK gene that was inserted into the mouse chromosome was recombined by the Cre recombinase and replaced with a second recombinant chromosome, the human chromosome (second recombinant chromosome) not originally included in the mESC was degraded after a predetermined culture time was elapsed, and the second recombinant chromosome containing the puroΔTK gene was extinguished. Therefore, the mESCs containing the first recombinant chromosome can be obtained.

The recombinant chromosomes may be recombined in many ways depending on the locations, directions, and pairing of the RRSs. For the production of a recombinant chromosome of interest, the design of the vector may be changed to enable production.

### 6. Production of a transgenic mouse containing a recombinant mESC with a humanized immunoglobulin locus

A transgenic mouse is produced with the use of the selected recombinant mESC, which is a recombinant mESC having the first recombinant chromosome (i.e., chromosome having a humanized immunoglobulin heavy chain locus).

The obtained recombinant mESC having a humanized immunoglobulin heavy chain locus is implanted into the blastula by blastocyst injection, thereby producing a chimeric blastocyst. The fabricated chimeric blastocyst is implanted into the womb of a surrogate mother, thereby producing a mouse offspring (FIGS. 25 and 26). The produced mouse offspring is a chimeric transgenic mouse, and a heterozygous transgenic mouse or a homozygous transgenic mouse can be produced by mating the chimeric transgenic mice. In the produced transgenic mouse, the variable region of an immunoglobulin heavy chain locus on the genome is humanized (i.e., exchanged (or replaced) with the variable region of a human immunoglobulin locus) . In this case, the transgenic mice can be produced by various methods, aside from the blastocyst injection.

### Example 4. Method of producing a transgenic mouse having a genome having a humanized immunoglobulin locus through interchromosomal exchange

The present example relates to a method of producing a transgenic mouse having a genome having a humanized immunoglobulin locus, and relates to a transgenic mouse in which a mouse immunoglobulin locus has a variable region and a constant region of a human immunoglobulin gene instead of a variable region and a constant of a mouse immunoglobulin gene. The overall method of the present example is similar to the method of Example 3 but is different in that the targeted immunoglobulin gene locus is an immunoglobulin kappa locus. The following description is only about a method of producing a transgenic mouse, but the present disclosure is not limited thereto. The intended transgenic mouse can be produced by diversely modifying the examples described below, or can be produced by using various methods other than the methods described in the examples.

### 1. Designing of vector

To exchange (or replace) a variable region and a constant region of a mouse immunoglobulin gene with a variable region and a constant region of a human immunoglobulin gene, vectors are designed for insertion of loxps into the respective ends of each of the variable regions. In this case, the inserted loxps are used for recombination of two chromosomes (the mouse chromosome on which the mouse immunoglobulin gene is located and the human chromosome on which the human immunoglobulin gene is located).

Two vectors (first and second vectors) were designed to insert loxps into the respective ends of a mouse immunoglobulin gene having a variable region and a constant region.

The first vector includes a first homologous arm used for insertion into the 5' end of a variable region of a mouse immunoglobulin kappa locus, loxm2/71 (first RRS), an EF1α promoter, a hygromycin resistant gene, and a second homologous arm used for insertion into the 5' end of the variable region of the mouse immunoglobulin kappa locus.

The second vector includes a third homologous arm used for insertion into the 3' end of the variable region of the mouse immunoglobulin kappa locus, a CAG promoter, a puroΔTK gene, lox66 (second RRS), and a fourth homologous arm used for insertion into the 3' end of the variable region of the mouse immunoglobulin kappa locus.

In this case, each of the first and second vectors includes a selection element (for example, puroΔTK gene) used for a positive selection and a negative selection.

In addition, two vectors (third and fourth vectors) were designed to insert loxps into the respective ends of a human immunoglobulin gene having a variable region and a constant region.

The third vector includes a fifth homologous arm used for insertion into the 5' end of a variable region of a human immunoglobulin kappa locus, an EF1α promoter, a hygromycin resistant gene, loxm2/66 (second RRS), and a sixth homologous arm used for insertion into the 5' end of the variable region of the human immunoglobulin kappa locus.

The fourth vector includes a seventh homologous arm used for insertion into the 3' end of a variable region of a human immunoglobulin kappa locus, lox71 (fourth RRS), a CAG promoter, a neomycin resistant gene, and an eighth homologous arm for insertion into the 3' end of the variable region of the human immunoglobulin kappa gene.

In this case, the third vector or the fourth vector includes a selection element not included in the first vector or the second vector. This design was intended to confirm that a human chromosome (including the third RRS and the fourth RRS) migrated to a mouse cell (containing a mouse chromosome into which the first RRS and the second RRS are inserted) using the microcell and the cell fusion.

In this case, the vector design may vary depending on the insertion position and the loxp type, and the design change can be made to include various elements for the selection process.

### 2. Production of a cell containing a chromosome with loxp inserted

Mouse and human cells with loxp inserted were prepared using the vectors. In this case, when there are multiple vectors, the vectors may be introduced in sequence, in random, or simultaneously. The selection process of the cells into which the vector is introduced is diversely modifiable depending on the elements inserted into the vector.

### 2-1. Production of a mouse cell containing a chromosome with loxp inserted

The mouse embryonic stem cell (mESC) used a basic culture medium 2i. The basic culture medium was a culture medium (2i medium) prepared by adding MEK inhibitor PD0325901 (1 µM) and GSK3 inhibitor CHIR99021 (3 µM) (both from Sigma Aldrich, St. Louis, MO, USA), and 1,000 U/ml of LIF (Milliphore, Billerica, MA, USA) to a FBS-free N2B27 medium. The mESCs were incubated for proliferation and sustentation in an incubator maintained in the conditions of a 95% wet state, 5% CO₂, 37°C. Transient transfection was performed using Lipofectamine 3000 or Nepa21 (NEPAGENE Co., Ltd., Chiba, Japan) electroporator. For the transient transfection, 1×10⁶ cells were placed in an FBS-free, antibiotic-free medium. Each of the first and second vectors was added thereto in an amount of 5 µg, the Lipofectamin 3000 reagent was added thereto and mixed, and the resulting mixture was left at room temperature for 5 minutes, followed by transfection. Alternatively, each of the first and second vectors was added thereto in an amount of 5 µg, and the transfection was performed under conditions of 125 V, 5 ms, and 1 pulse. The transfected mESCs were incubated for 24 to 48 hours in an incubator maintaining the conditions of a 95% wet state, 5% CO₂, and 37°C.

To confirm whether the first vector and the second vector were inserted respectively into the 5' end and 3' end of the variable region of the mouse heavy chain locus, the transfected mESCs were treated with hygromycin and puromycin. Whether the first and second vectors were inserted was determined according to survival or death of the cells. After the cells were treated with an antibiotic, the surviving mESCs were collected, and PCR was performed to determine whether the first and second vectors were inserted (FIG. 35).

Sample #2, sample #6, and sample #8 illustrated in FIG. 35 were targeted, and it was confirmed that both of the first and second vectors were inserted into the respective ends (a variable region and a constant region) of a mouse endogenous immunoglobulin kappa gene. In this case, the PCR conditions and target band size are as follows:

The target band size of a portion of the 5' end of the variable region of the mouse endogenous immunoglobulin kappa gene and a portion (denoted by mKP left) of the loxm2/71 (first RRS) and the EF1α promoter included in the first vector are 1.65 kb, in which mlgKP-left-F1 and EF1α-R3 are used as primers, and the PCR is performed with 1 cycle of an initial denaturation (94°C, 5 minutes), 35 cycles of denaturation (94°C, 1 minute)-annealing (63°C, 1 minute)-extension (72°C, 2 minutes), and 1 cycle of a final extension (72°C, 10 minutes);
the target band size of a portion of a hygromycin resistant gene included in the first vector and a portion (denoted by mKP right) of the 5' end of the variable region of the mouse endogenous immunoglobulin kappa gene are 1.29 kb, in which HygR-R1 and mlgKP-right-R1 are used as primers, and the PCR is performed with 1 cycle of an initial denaturation (94°C, 5 minutes), 35 cycles of denaturation (94°C, 1 minute)-annealing (63°C, 1 minute)-extension (72°C, 2 minutes), and 1 cycle of a final extension (72°C, 10 minutes);
the target band size of a portion of the 3' end of the variable region of the mouse endogenous immunoglobulin kappa gene and a portion (denoted by mKC left) of a CAG promoter included in the second vector are 1.39 kb, in which mlgKC-left-F1 and CAG-R1 are used as primers, and the PCR is performed with 1 cycle of an initial denaturation (94°C, 5 minutes), 35 cycles of denaturation (94°C, 1 minute) -annealing (63°C, 1 minute)-extension (72°C, 2 minutes), and 1 cycle of a final extension (72°C, 10 minutes); and
the target band size of a portion of the puroΔTK gene included in the second vector and a portion (denoted by mKC right) of the 3' end of the variable region of the mouse endogenous immunoglobulin kappa gene are 3.05 kb, in which PuroR-F3 and mlgKC-right-R1 are used as primers, and the PCR is performed with 1 cycle of an initial denaturation (94°C, 5 minutes), 35 cycles of denaturation (94°C, 1 minute)-annealing (63°C, 1 minute)-extension (72°C, 3 minutes), and 1 cycle of a final extension (72°C, 10 minutes).

The used primers are summarized in Table 8.

**[Table 8]**

| **No.** | **Name** | **Sequence (5'_{→}3')** | **SEQ ID NO:** |
|---|---|---|---|
| 1 | mIgKP-left-F1 | ttccacagctgttgcctatcc | 47 |
| 2 | EF1α-R3 | gccataacccgtaaagaggc | 35 |
| 3 | HygR-R1 | CCGATGGCTGTGTAGAAGTAC | 36 |
| 4 | mIgKP-right-R1 | caaggttcctgactaccactg | 48 |
| 5 | mIgKC-left-F1 | gaagtgaagtctgccagttcc | 49 |
| 6 | CAG-R1 | ACGCGGAACTCCATATATGGG | 39 |
| 7 | PuroR-F3 | tgcaagaactcttcctcacgc | 50 |
| 8 | mIgKC-right-R1 | tgcactgccttcagagtgtag | 51 |

In addition, sequencing was performed to determine whether the target band was satisfied, using the detected PCR product. As a result, it was confirmed that the first and second vectors were precisely and respectively inserted into the 5' end and 3' end (of a variable region and a constant region) of the mouse endogenous immunoglobulin kappa gene.

The cells thus obtained were used as recipient cells as described below.

### 2-2. Production of a human cell containing a human chromosome with loxp inserted

The used human fibroblast was a BJ cell line (ATCC^{®} CRL-2522^{™} is white). To culture the BJ cells, Eagle's Minimum Essential Medium (EMEM) (ATCC^{®}30-2003^{™} medium) containing 10% fetal bovine serum (FBS; Corning, Mannasas, VA, USA) and 1% penicillin-streptomycin (Corning, Mannasas, VA, USA) was used. The BJ cells were incubated in an incubator maintaining conditions of a 95% wet state, 5% CO₂, and 37°C so that the BJ cells were proliferated and sustained. Transient transfection was performed using Lipofectamine 3000 (Invitrogen, Carlsbad, CA, USA) or Nepa21 (NEPAGENE Co., Ltd., Chiba, Japan) electroporator. For the transient transfection, 1×10⁶ cells were placed in an FBS-free, antibiotic-free medium. Each of the third and fourth vectors was added thereto in an amount of 5 µg, the Lipofectamin 3000 reagent was added thereto and mixed, and the resulting mixture was left at room temperature for 5 minutes, followed by transfection. Alternatively, each of the third and fourth vectors was added thereto in an amount of 5 µg, and the transfection was performed under conditions of 150 V, 7.5 ms, and 3 pulses. The transfected human fibroblast cells were incubated for 24 to 48 hours in an incubator maintaining the conditions of a 95% wet state, 5% CO₂, and 37°C.

To confirm whether the third vector and the fourth vector were inserted respectively into the 5' end and 3' end of the variable region of a human immunoglobulin heavy chain locus, the transfected human fibroblast cells were treated with hygromycin and G418. Whether the third and fourth vectors were inserted was determined according to survival or death of the cells. After the cells were treated with an antibiotic, the surviving fibroblast cells were collected, and PCR was performed to determine whether the third and fourth vectors were inserted (FIG. 36).

Sample #16 in FIG. 36 was targeted by the third and fourth vectors, and it was confirmed that the third and fourth vectors were inserted into the respective ends (of a variable region and a constant region) of a human endogenous immunoglobulin kappa gene. In this case, the PCR conditions and target band size are as follows:
the target band size of a portion of the 5' end of the variable region of the human endogenous immunoglobulin kappa gene and a portion (denoted by KP left) of an EF1α promoter included in the third vector are 1.99 kb, in which hIgKP-left-F2 and EF1α-R3 are used as primers, and the PCR is performed with 1 cycle of an initial denaturation (94°C, 5 minutes), 35 cycles of denaturation (94°C, 1 minute)-annealing (63°C, 2 minutes)-extension (72°C, 2 minutes), and 1 cycle of a final extension (72°C, 10 minutes);
the target band size of a portion of the hygromycin resistant gene included in the third vector and a portion (denoted by KP right) of the 5' end of a variable region of a human endogenous immunoglobulin kappa gene and loxm2/66 (third RRS) are 4.2 kb, in which HygR-R1 and hIgKP-right-R3 are used as primers, and the PCR is performed with 1 cycle of an initial denaturation (94°C, 5 minutes), 35 cycles of denaturation (94°C, 1 minute)-annealing (65°C, 2 minutes)-extension (72°C, 4 minutes), and 1 cycle of a final extension (72°C, 10 minutes);
the target band size of a portion of the 3' end of the variable region of the human endogenous immunoglobulin kappa gene and a portion (denoted by KC left) of a CAG promoter and lox71 (fourth RRS) included in the fourth vector are 1.4 kb, in which hIgKC-left-F2 and CAG-R1 are used as primers, and the PCR is performed with 1 cycle of an initial denaturation (94°C, 5 minutes), 35 cycles of denaturation (94°C, 1 minute)-annealing (65°C, 2 minutes)-extension (72°C, 2 minutes), and 1 cycle of a final extension (72°C, 10 minutes); and
the target band size of a portion of a neomycin resistant gene included in the fourth vector and a portion (denoted by KC right) of the 3' end of the variable region of the human endogenous immunoglobulin kappa gene are 1.8 kb, in which Neo-F2 and hIgKC-right-R2 are used as primers, and the PCR is performed with 1 cycle of an initial denaturation (94°C, 5 minutes), 35 cycles of denaturation (94°C, 1 minute)-annealing (65°C, 2 minutes)-extension (72°C, 2 minutes), and 1 cycle of a final extension (72°C, 10 minutes).

The used primers are summarized in Table 9.

**[Table 9]**

| **No.** | **Name** | **Sequence (5'_{→}3')** | **SEQ ID NO:** |
|---|---|---|---|
| 1 | hIgKP-left-F2 | ACTCGTGTGAACGCGTCATAC | 52 |
| 2 | EF1α-R3 | gccataacccgtaaagaggc | 35 |
| 3 | HygR-R1 | CCGATGGCTGTGTAGAAGTAC | 36 |
| 4 | hIgKC-right-R2 | cctaacaacaaccacaaccac | 53 |
| 5 | hIgKC-left-F2 | tctggaactgcctctgttgtg | 54 |
| 6 | CAG-R1 | ACGCGGAACTCCATATATGGG | 39 |
| 7 | Neo-F2 | TGTCATCTCACCTTGCTCCTG | 45 |
| 8 | hIgKC-right-R2 | catagctggattatgcctgcc | 55 |

Sequencing was performed to determine whether the target band was satisfied, using the detected PCR product. As a result, it was confirmed that the third and fourth vectors were precisely and respectively inserted into the 5' end and 3' end (of a variable region and a constant region) of the human endogenous immunoglobulin kappa gene.

The cells thus obtained were used as donor cells as described below.

### 3. Production of a microcell using a human fibroblast containing a human chromosome with loxp inserted

Micronuclei were formed using colcemid (Life Technologies, Grand Island, NY, USA). The selected human fibroblast cells (cells containing chromosomes in which loxm2/66 (third RRS) and lox71 (fourth RRS) were inserted into the respective ends (of a variable region and a constant region) of a human immunoglobulin kappa gene) were cultured to have a density of 1×10⁶ cells. On the next day, the culture medium was replaced with EMEM containing 20% FBS, and the cells were treated with 0.1 pg/ml of colcemid and incubated for 48 hours in an incubator maintained in the conditions of a 95% wet state, 5% CO₂, and 37°C. The micronucleated human fibroblast cells were detached using tryLE (Life technologies, Grand island, NY, USA), washed with serum-free EMEM, and centrifuged at 1000 rpm for 5 minutes (LABOGENE Co., Ltd., Korea). The centrifuged cells were suspended on preheated serum-free EMEM:percoll ((Sigma Aldrich, St. Louis, MO, USA) (1:1 (v:v)) and were treated with cytochalsin B (Sigma Aldrich, St. Louis, MO, USA) to have a final concentration of 10 ug/ml. The centrifugation (LABOGEN) was performed at 16000 g, at 34°C to 37°C for 1 to 1.5 hours to obtain whole cells and microcells. The whole cells and the microcells were transferred to a 50-ml tube, serum-free EMEM was added thereto, and the resulting mixture was centrifuged at 500g for 10 minutes. The supernatant was carefully removed, pellets attached to the surface of the tube were placed in 10 ml of the serum-free EMEM, and microcells with a size of 8 um or less were separated using an 8-µm filter (GE healthcare, Chicago, IL, USA). The supernatant containing the microcells was centrifuged at 400 g for 10 minutes. The centrifuged microcells were separated using a 5-µm filter in the same manner as the filtration using the 8-µm filter. The finally separated microcells were counted using a Nicon eclipse TS100 optical microscope (Nicon Instruments, Melville, NY, USA). Human microcells were obtained from human fibroblast cells, which are donor cells (FIG. 37).

### 4. Production of a fusion cell using a microcell and a mouse cell containing a mouse chromosome with loxp inserted

Separated human microcells were prepared, and mESCs which were to be used as recipient cells and into which loxp was inserted were prepared, in which the mESCs were cells containing a chromosome in which loxm2/71 (first RRS) and lox66 (second RRS) were inserted into the respective ends (of a variable region and a constant region) of a mouse immunoglobulin kappa gene. The mESCs were treated with TryLE and centrifuged. The centrifuged mESCs were washed with 1XDPBS and the number of cells was calculated using a hemacytometer. The fusion of the human microcells and the mESCs was performed by a suspension method, using the HVJ-E protein (Cosmo Bio Co., Ltd., Tokyo, Japan). The ratio of the human microcells and the mESCs was 1:4. The prepared human microcells and the prepared mESCs were washed with 500 µl of a cold 1x cell fusion buffer. The human microcells and mESCs contained in the buffer were centrifuged at 4°C at 300 g for 5 minutes. 25 µl of the 1x cell fusion buffer was added per 2 × 10⁵ cells of the mESCs, and the same volume of the 1x cell fusion buffer was added to the human microcells. The mESCs and the human microcells were mixed, 5 to 10 µl of the HVJ-E protein was added, and the mixture was left on ice for 5 minutes. The mixture was left in a 37°C water bath for 15 minutes. The mixture was tapped at intervals of 5 minutes. After the cell fusion of the human microcells and the mESCs was completed, the remaining HVJ-E protein was removed by centrifugation at 300 g for 5 minutes. To select fusion cells, the fusion cells were treated with G418 (Life Technologies, NY, USA) at a concentration of 200 ug/ml for one week. The surviving fusion cells after being treated with G418 were fusion mESCs containing a human chromosome and a mouse chromosome, in which the human chromosome was a chromosome containing a human immunoglobulin kappa gene into which loxm2/66 (third RRS) and lox71 (fourth RRS) were inserted, and the mouse chromosome was a chromosome containing a mouse immunoglobulin kappa gene into which loxm2/71 (first RRS) and lox66 (second RRS) were inserted (FIG 38). In this case, the selection of the fusion mESCs through the G418 treatment can be omitted.

After cell fusion was performed on J1 mES cells, the G418 treatment was performed at a concentration of 200 ug/ml for 3 to 7 days, and colony was collected and cultured. gDNA was collected by PCIA extraction from 1.0 × 10⁵ cells among the cultured cells, and genotyping was performed by PCR.

As a result, it was found that sample #3 and sample #4 in FIG. 39 had a human chromosome (chromosome containing a human immunoglobulin kappa gene into which loxm2/66 (third RRS) and lox71 (fourth RRS) were inserted). In this case, the PCR conditions and target band size are as follows:
the target band size of a portion of the 5' end of a variable region of a human endogenous immunoglobulin kappa gene and a portion (denoted by hKP left) of an EF1α promoter is 1.99 kb, in which hIgKP-left-F2 and EF1α-R3 are used as primers, and the PCR is performed with 1 cycle of an initial denaturation (94°C, 5 minutes), 35 cycles of denaturation (94°C, 1 minute)-annealing (63°C, 2 minutes)-extension (72°C, 2 minutes), and 1 cycle of a final extension (72°C, 10 minutes);
the target band size of a portion of a hygromycin resistant gene and a portion (denoted by hKP right) of the 5' end of a variable region of a human endogenous immunoglobulin kappa gene and loxm2/66 (third RRS) is 1.6 kb, in which HygR-R1 and hIgKP-right-R5 are used as primers, and the PCR is performed with 1 cycle of an initial denaturation (94°C, 5 minutes), 35 cycles of denaturation (94°C, 1 minute)-annealing (63°C, 2 minutes)-extension (72°C, 2 minutes), and 1 cycle of a final extension (72°C, 10 minutes);
the target band size of a portion of the 3' end of a variable region of a human endogenous immunoglobulin kappa gene and a portion (denoted by hKC left) of a CAG promoter and lox71 (fourth RRS) included is 1.4 kb, in which hIgKC-left-F2 and CAG-R1 are used as primers, and the PCR is performed with 1 cycle of an initial denaturation (94°C, 5 minutes), 35 cycles of denaturation (94°C, 1 minute)-annealing (65°C, 2 minutes)-extension (72°C, 2 minutes), and 1 cycle of a final extension (72°C, 10 minutes); and
the target band size of a portion of a neomycin resistant gene included and a portion (denoted by hKC right) of the 3' end of a variable region of a human endogenous immunoglobulin kappa gene is 1.8 kb, in which Neo-F2 and hIgKC-right-R2 are used as primers, and the PCR is performed with 1 cycle of an initial denaturation (94°C, 5 minutes), 35 cycles of denaturation (94°C, 1 minute)-annealing (65°C, 2 minutes)-extension (72°C, 2 minutes), and 1 cycle of a final extension (72°C, 10 minutes).

The used primers are summarized in Table 10.

**[Table 10]**

| **No.** | **Name** | **Sequence (5'_{→}3')** | **SEQ ID NO:** |
|---|---|---|---|
| 1 | hIgKP-left-F2 | ACTCGTGTGAACGCGTCATAC | 52 |
| 2 | EF1α-R3 | gccataacccgtaaagaggc | 35 |
| 3 | HygR-R1 | CCGATGGCTGTGTAGAAGTAC | 36 |
| 4 | hIgKP-right-R5 | acagacagctaatgtggtggc | 56 |
| 5 | hIgKC-left-F2 | tctggaactgcctctgttgtg | 54 |
| 6 | CAG-R1 | ACGCGGAACTCCATATATGGG | 39 |
| 7 | Neo-F2 | TGTCATCTCACCTTGCTCCTG | 45 |
| 8 | hIgKC-right-R2 | catagctggattatgcctgcc | 55 |

### 5. Production of a cell containing a humanized immunoglobulin gene by chromosomal exchange

The produced fusion cells were treated with a recombinase so that a variable region and a constant region of a mouse immunoglobulin kappa locus is exchanged (or replaced) with a variable region and a constant region of a human immunoglobulin kappa locus. Thus, recombinant cells having a humanized immunoglobulin kappa locus were produced. That is, recombinant mESCs were produced. In this case, the humanized immunoglobulin kappa locus is a locus having a variable region and a constant region derived from a human immunoglobulin kappa gene.

100 µl of an FBS-free, an antibiotic-free opti-MEM medium was added to 1×10⁶ fusion cells (fusion mESCs). 10 µg of the pCMV-Cre vector (System Biosciences, LLC, Palo Alto, CA, USA) was added thereto, and transfection was performed under the conditions of 125 V, 5 ms, and 2 pulses. After the addition of 300 µl of the 2i medium and mixing of the fusion cells, the mixture was transferred to a 100-mm dish, and the cells were incubated in an incubator maintained in the conditions of a 95% wet state, 5% CO₂, and 37°C.

In the case of a Cre recombinase-treated fusion cell, recombination of a human chromosome and a mouse chromosome was induced by the Cre recombinase, in which the human chromosome was a chromosome including a human immunoglobulin kappa gene into which loxm2/66 (third RRS) and lox71/66 (fourth RRS) were inserted, and the mouse chromosome was a chromosome including a mouse immunoglobulin kappa gene into which loxm2/71 (first RRS) and lox66 (second RRS) were inserted. The first RRS in the mouse chromosome and the third RRS in the human chromosome are paired with each other, and the second RRS in the mouse chromosome and the fourth RRS in the human chromosome are paired with each other. The pairing of the RRSs is recognized by the Cre recombinase, and thus the recombination is induced.

As a result, a first recombinant chromosome (a mouse chromosome including a humanized immunoglobulin kappa locus) in which a variable region and a constant region of a mouse immunoglobulin kappa locus was replaced with a variable region and a constant region of a human immunoglobulin heavy chain gene and a second recombinant chromosome in which a variable region and a constant region of a human immunoglobulin kappa locus was replaced with a variable region and a constant region of a mouse immunoglobulin kappa locus are produced. The first recombinant chromosome has a variable region and a constant region of a human immunoglobulin kappa gene at a mouse immunoglobulin kappa locus. The second recombinant chromosome has a variable region and a constant region of a mouse immunoglobulin heavy chain gene at a human immunoglobulin kappa locus.

To confirm that a first recombinant chromosome having a humanized immunoglobulin kappa locus is generated in the fusion cell treated with the Cre recombinase, the fusion cells treated with the Cre recombinase were cultured for a predetermined period of time, the cultured cells were then treated with fialuridine(1-(2-deoxy-2-fluoro-1-D-arabinofuranosyl)-5-iodouracil)(FIAU), and surviving fusion cells were selected. The surviving cells (mESCs) were collected after the FIAU treatment. The obtained cells were mESCs containing the first recombinant chromosome. When the puroΔTK gene that was inserted into a mouse chromosome was recombined by the Cre recombinase and replaced with the second recombinant chromosome, the human chromosome (second recombinant chromosome) not originally included in the mESC was degraded after a predetermined time of culturing, and the second recombinant chromosome containing the puroΔTK gene was extinguished. Therefore, the mESCs containing the first recombinant chromosome can be obtained.

The recombinant chromosomes may be recombined in many ways depending on the locations, directions, and pairing of the RRSs. For the production of a recombinant chromosome of interest, the design of the vector may be changed to enable production.

### 6. Production of a transgenic mouse using a recombinant mESC with a humanized immunoglobulin locus

A transgenic mouse is produced with the use of the selected recombinant mESC, which is a recombinant mESC having the first recombinant chromosome (i.e., chromosome having a humanized immunoglobulin kappa locus).

The obtained recombinant mESC having a humanized immunoglobulin kappa locus is implanted into the blastula by blastocyst injection, thereby producing a chimeric blastocyst. The fabricated chimeric blastocyst is implanted into the womb of a surrogate mother, thereby producing a mouse offspring (FIGS. 25 and 26). The produced mouse offspring is a chimeric transgenic mouse, and a heterozygous transgenic mouse or a homozygous transgenic mouse can be produced by mating the chimeric transgenic mice. In the produced transgenic mouse, the variable region and constant region of the immunoglobulin kappa locus on the genome was humanized (i.e., exchanged (or replaced) with the variable region and constant region of a human immunoglobulin locus). In this case, the transgenic mice can be produced by various methods as well as the blastocyst injection.

### Sequence Listing Free Text

Sequence listing includes site-specific recombinases, recombinase recognition sites (RRSs), and primer sequences used in the examples.

## Claims

1. A method for producing transgenic non-human animal cell having genome comprising humanized immunoglobulin gene locus, wherein the humanized immunoglobulin locus comprises variable region of a human immunoglobulin gene and constant region of an endogenous non-human animal immunoglobulin locus, the method comprising:
a) preparing a recipient cell having a recipient chromosome and a donor cell having a donor chromosome,
wherein the recipient chromosome is an engineered non-human-animal chromosome having two or more RRS and non-human animal immunoglobulin locus; the two or more RRS are a first RRS and a second RRS; wherein the first RRS and the second RRS are not paired; the first RRS is located on the 5' end of the variable region of the non-human animal immunoglobulin locus, the second RRS is located on the 3' end of the variable region of the non-human animal immunoglobulin locus; the recipient cell is a non-human-animal cell;
wherein the donor chromosome is an engineered human chromosome having two or more RRS and human immunoglobulin locus; the two or more RRS are a third RRS and a fourth RRS; wherein the third RRS and the fourth RRS are not paired; the third RRS is located on the 5' end of the variable region of the human immunoglobulin locus, the fourth RRS is located on the 3' end of the variable region of the human immunoglobulin locus; the donor cell is a human cell;
wherein the first RRS and the third RRS are RRS for a first interchromosomal exchange, and the second RRS and fourth RRS are RRS for a second interchromosomal exchange;
b) producing a plurality of microcells using the donor cell,
at least one microcell among the plurality of microcells includes the donor chromosome;
c) producing a fusion non-human animal cell by contacting at least one microcell with the recipient cell;
the fusion non-human animal cell comprises the recipient chromosome and the donor chromosome; and
d) producing a recombinant non-human-animal cell having a recombinant chromosome by treating the fused non-human-animal cell with a recombinase,
the recombinase recognizes the RRS for a first interchromosomal exchange and the RRS for a second interchromosomal exchange to induce recombination between the recipient chromosome and the donor chromosome; whereby variable region of the non-human animal immunoglobulin locus existing between the first and second RRS of the recipient chromosome is exchanged for the variable region of the human immunoglobulin locus between the third and fourth RRS of the donor chromosome;
As a result, a recombinant chromosome comprising variable region of a human immunoglobulin locus and constant region of an endogenous non-human animal immunoglobulin locus is generated.

2. The method of claim 1,
wherein the non-human animal immunoglobulin locus is non-human animal immunoglobulin heavy locus, the human immunoglobulin locus is human immunoglobulin heavy locus.

3. The method of claim 2,
wherein the recombinant chromosome comprises a human immunoglobulin locus comprising variable region of a human immunoglobulin heavy locus and the constant region of an endogenous non-human animal immunoglobulin heavy locus.

4. The method of claim 1,
wherein the non-human animal immunoglobulin locus is an non-human-animal immunoglobulin kappa locus, the human immunoglobulin locus is a human immunoglobulin kappa locus.

5. The method of claim 4,
wherein the recombinant chromosome comprises a human immunoglobulin locus comprising variable region of a human immunoglobulin kappa locus and the constant region of an endogenous non-human animal immunoglobulin kappa locus.

6. The method of claim 1,
wherein the non-human animal immunoglobulin locus is an non-human-animal immunoglobulin lambda locus, the human immunoglobulin locus is a human immunoglobulin lambda locus.

7. The method of claim 6,
wherein the recombinant chromosome comprises a human immunoglobulin locus comprising variable region of a human immunoglobulin lambda locus and the constant region of an endogenous non-human animal immunoglobulin lambda locus.

8. The method of claim 1,
wherein the non-human animal immunoglobulin locus is an non-human-animal immunoglobulin kappa locus, the human immunoglobulin locus is a human immunoglobulin lambda locus.

9. The method of claim 8,
wherein the recombinant chromosome comprises a human immunoglobulin locus comprising variable region of a human immunoglobulin lambda locus and the constant region of an endogenous non-human animal immunoglobulin kappa locus.

10. The method of claim 1,
wherein in the a), the recipient cell is prepared by providing a vector for a recipient chromosome to a non-human-animal cell,
the donor cell is prepared by providing a vector for a donor chromosome to a human cell.

11. The method of claim 10,
wherein the vector for a recipient chromosome includes a first vector and a second vector,
the first vector includes a first RRS, and the second vector includes a second RRS.

12. The method of claim 10,
wherein the vector for a donor chromosome includes a third vector and a fourth vector,
the third vector includes a third RRS, and the fourth vector includes a fourth RRS.

13. The method of claim 1,
wherein the first RRS and the third RRS are each one selected from Loxp, FRT, attP, attB, ITR, and variants thereof,
the first RRS and the third RRS are paired,
wherein the second RRS and the fourth RRS are each one selected from Loxp, FRT, attP, attB, ITR, and variants thereof,
the second RRS and the fourth RRS are paired.

14. The method of claim 13,
wherein the recombinase includes a first recombinase capable of recognizing RRS for a first interchromosomal exchange and a second recombinase capable of recognizing RRS for a second interchromosomal exchange,
the first recombinase is Cre recombinase, flippase (FLP), integrase or transposase,
the second recombinase is Cre recombinase, flippase (FLP), integrase or transposase.

15. The method of claim 14,
wherein the first recombinase and the second recombinase are the same recombinase.

16. The method of claim 1,
wherein the non-human animal cell is mouse cell,
the non-human animal immunoglobulin locus is mouse immunoglobulin locus.

17. A method for producing transgenic non-human animal having genome comprising humanized immunoglobulin gene locus, wherein the humanized immunoglobulin locus comprises variable region of a human immunoglobulin gene and constant region of an endogenous non-human animal immunoglobulin locus, the method comprising:
a) preparing a recipient cell having a recipient chromosome and a donor cell having a donor chromosome,
wherein the recipient chromosome is an engineered non-human-animal chromosome having two or more RRS and non-human animal immunoglobulin locus; the two or more RRS are a first RRS and a second RRS; wherein the first RRS and the second RRS are not paired; the first RRS is located on the 5' end of the variable region of the non-human animal immunoglobulin locus, the second RRS is located on the 3' end of the variable region of the non-human animal immunoglobulin locus; the recipient cell is a non-human-animal cell;
wherein the donor chromosome is an engineered human chromosome having two or more RRS and human immunoglobulin locus; the two or more RRS are a third RRS and a fourth RRS; wherein the third RRS and the fourth RRS are not paired; the third RRS is located on the 5' end of the variable region of the human immunoglobulin locus, the fourth RRS is located on the 3' end of the variable region of the human immunoglobulin locus; the donor cell is a human cell;
wherein the first RRS and the third RRS are RRS for a first interchromosomal exchange, and the second RRS and fourth RRS are RRS for a second interchromosomal exchange;
b) producing a plurality of microcells using the donor cell,
at least one microcell among the plurality of microcells include the donor chromosome;
c) producing a fusion non-human animal cell by contacting at least one microcell with the recipient cell;
the fusion non-human animal cell comprises the recipient chromosome and the donor chromosome;
d) producing a recombinant non-human-animal cell having a recombinant chromosome by treating the fused non-human-animal cell with a recombinase,
the recombinase recognizes the RRS for a first interchromosomal exchange and the RRS for a second interchromosomal exchange to induce recombination between the recipient chromosome and the donor chromosome; whereby variable region of the non-human animal immunoglobulin locus existing between the first and second RRS of the recipient chromosome is exchanged for the variable region of the human immunoglobulin locus between the third and fourth RRS of the donor chromosome;
As a result, a recombinant chromosome comprising variable region of a human immunoglobulin locus and constant region of an endogenous non-human animal immunoglobulin locus is generated, and
e) producing offspring using the recombinant non-human-animal cell.

18. The method of claim 17,
wherein the non-human animal cell is a somatic cell,
the recombinant non-human animal cell is a somatic cell,
wherein in the e), offspring is produced through somatic cell nuclear transfer (SCNT) using recombinant somatic cells.

19. The method of claim 17,
wherein the non-human animal cell is a non-human animal embryo,
the recombinant non-human animal cell is a non-human animal embryo,
wherein in the e), offspring is produced by implantation of a recombinant non-human animal embryo into the uterus of a surrogate mother.

20. The method of claim 17,
wherein the non-human animal cell is a non-human animal embryonic stem cell(ES cell),
the recombinant non-human animal cell is a recombinant non-human animal embryonic stem cell,
wherein in the e), recombinant non-human animal embryonic stem cell is transplanted into blastocysts to produce a chimeric blastocyst, offspring is produced by implantation of a chimeric blastocyst into the uterus of a surrogate mother.

21. The method of claim 17,
wherein the non-human animal cell is a mouse cell,
wherein the transgenic non-human animal is a mouse.
